# EUROPEAN PATENT APPLICATION

(11) **EP 1 710 582 A2**
(43) Date of publication of application: **11.10.2006**
(21) Application number: 06076277.0
(22) Date of filing: 06.08.2001
(51) Int. Cl.: G01N 33/53, C12Q 1/68, C12N 15/12, C07K 14/47, C12N 15/11, C12N 5/10, C07K 16/18, A61K 38/17, A61K 48/00, G01N 33/50

(54) **Suppressor genes**

(30) Priority: 04.08.2000 GB 0019018; 08.12.2000 GB 0029996; 26.05.2001 GB 0112890
(62) Divisional of application: 01954168.9
(71) Applicant: LUDWIG INSTITUTE FOR CANCER RESEARCH, 8024 Zürich (CH)
(72) Inventor: Xin, Lu Ludwing Inst. of Cancer, London W1W 7BS (GB)
(74) Representative: Docherty, Robert Charles

(57) **Abstract**

The invention relates to the identification of a new member of a family of tumour suppressor genes (apoptosis stimulating proteins, ASP's) which encode polypeptides capable of modulating the activity of p53 and polypeptides capable of modulating the activity of said tumour suppressor polypeptide.

## Description

The invention relates to members of a family of tumour suppressor genes, (Apoptosis Stimulating Proteins (ASP)), which encode polypeptides capable of modulating the activity of p53 and also polypeptides capable of modulating the activity of said ASP polypeptides.

Tumour suppressor genes encode proteins which function to inhibit cell growth or division and are therefore important with respect to maintaining proliferation, growth and differentiation of normal cells. Mutations in tumour suppressor genes result in abnormal cell-cycle progression whereby the normal cell-cycle check points which arrest the cell-cycle, when, for example, DNA is damaged, are ignored and damaged cells divide uncontrollably. The products of tumour suppressor genes function in all parts of the cell (eg cell surface, cytoplasm, nucleus) to prevent the passage of damaged cells through the cell- cycle (ie G1, S, G2, M and cytokinesis).

A number of tumour suppressor genes have been isolated and sequenced. These include, by example only, the Retinoblastoma gene (Rb), mutations in which are linked to cancers such as bone (osteocarcoma), bladder, small cell lung and breast cancer, as well as retinoblastoma, and the Wilms Tumour ― 1 gene (WT-1), mutations in which are linked to nephroblastoma and neurofibromatosis.

The tumour suppressor gene family, MAD (Mothers against *dpp* (decapentapelgic gene) and MADR (MAD related genes) have been identified in a number of species. These genes encode proteins involved in signal transduction pathways required for serine/threonine receptor signalling. MADR1 is essential for signalling of *dpp* pathway. MADR2 is another MADR and mutations in this gene have been linked with colorectal cancer (6% of sporadic colorectal cancers). The sequence of the MADR2 gene, also known as Smad2, is disclosed in WO98/07849.

Arguably the tumour suppressor gene which has been the subject of the most intense research is p53. p53 encodes a protein which functions as a transcription factor and is a key regulator of the cell division cycle. It was discovered in 1978 (Lane and Crawford, 1979) as a protein shown to bind with affinity to the SV40 large T antigen. The p53 gene encodes a 393 amino acid polypeptide with a molecular weight of 53kDa.

Genes regulated by the transcriptional activity of p53 contain a p53 recognition sequence in their 5' regions. These genes are activated when the cellular levels of p53 are elevated due to, for example DNA damage. Examples of genes which respond to p53 include, mdm2 (Momand et al 1992), Bax (Miyashita and Reed, 1995) and PIG-3 (Polyak et al, 1997). Bax and PIG-3 are involved in one of the most important functions of p53, the induction of apoptosis. Apoptosis, or programmed cell death, is a natural process which removes damaged cells. It is of importance with respect to many cellular processes, including the removal of pre-cancerous cells, cell/tissue development and homeostasis.

As mentioned above, one of the most important tumour suppression functions of p53 is its ability to induce apoptosis. The ability to up-regulate the expression of some of the pro-apoptotic genes such as Bax provided some evidence of how p53 induces apoptosis. However, by comparing the Bax expression in p53(-/-) and p53(+/+) transgenic mice and wild-type it is clear that only in a limited number of tissues was the expression of Bax regulated by p53 in response to DNA damage. Thus it remains unclear why the expression of p53 could only induce the expression of Bax in a cell type specific manner. It was shown recently that mutation in p53 can change *promoter specificity.* Two of the tumour-derived mutant p53 genes were shown to be defective in transactivation of the Bax promoter but competent to transactivate other promoters of p53 target genes such as mdm2 and p21wafl. These observations suggested that to be able to transactivate genes like Bax is very important for the tumour suppression function of p53.

It is known that p53 can induce apoptosis through transcriptional dependent and independent pathways. In addition, p53 induced-apoptosis can be blocked by the oncogene bcl-2. However, bcl-2 does not inhibit the transactivation function of p53. So far, very little is known about the molecular mechanisms of how bcl-2 inhibits p53- induced apoptosis.

53BP2 is a p53 binding protein initially discovered by Iwabuchi *et al* (1994). 53BP2 was isolated in a yeast 2-hybrid screen and was found to consist of 528 amino acids from the C-terminus of the protein. It contains a proline rich sequence, four ankryin repeats and an SH3 domain. Subsequently it was identified as a protein which interacted with Bcl-2 (Naumovski and Cleary, 1996). A longer version of this protein was isolated and named as bBP2/53BP2. Based on the *in vitro* translation data, the authors (Naumoviski and Cleary, 1996) predicted that the bBP2/53BP2 protein consisted of 1005 amino acids.

In an attempt to understand how the apoptotic function of p53 can be regulated *in vivo,* we generated antibodies to 53BP2 and showed that in most of the cells tested, the expression level of 53BP2 is low. We also observed that the endogenous bBP2/53BP2 unexpectedly encodes a protein larger than the 1005 amino acids predicted by Naumovski and Cleary. This protein, ASP-2, consists of 1135 amino acids.

For the sake of clarity the following nomenclature will be used:
i) the 528 amino acid polypeptide will be referred to as 53BP2 or ASP-2/53BP2 (607-1135)
ii) the 1005 amino acid polypeptide will be referred to as bBP2/53BP or ASP-2/Bbp2 (130-1135)
iii) the 1135 amino acid polypeptide will be referred to as ASP-2/53BP, or simply ASP-2 (1-1135).

The numbers in brackets indicate the equivalent amino acids of ASP-2.

We have shown that the C-terminal half of bBP2/53BP does not have a significant effect on the activity of p53. However, ASP-2/53BP stimulated the transactivation function of p53. Most interestingly, ASP-2/53BP can specifically enhance the transactivation function of p53 on the promoters derived from pro-apoptosis related genes such as Bax and PIG-3.

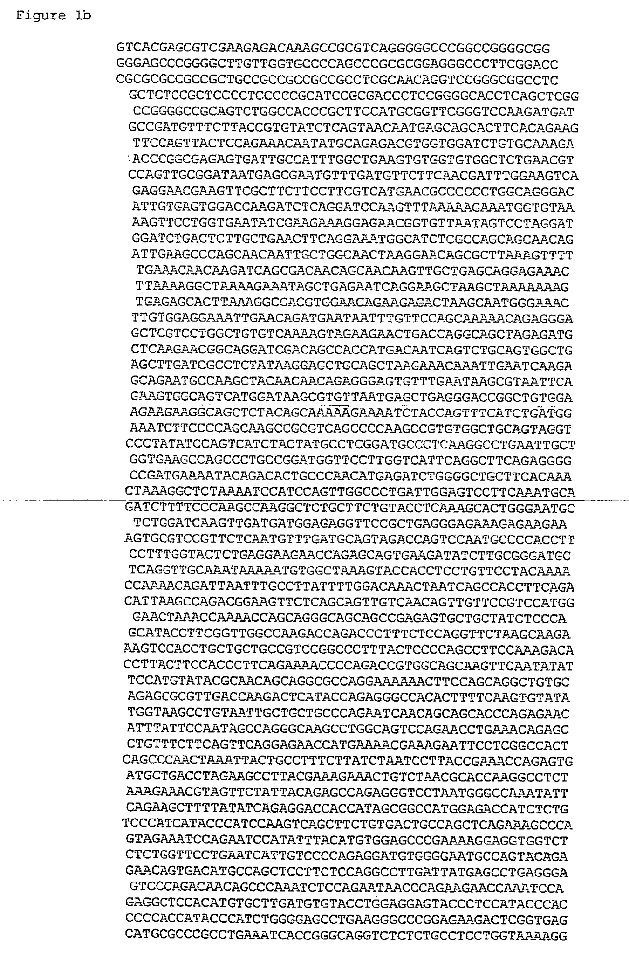

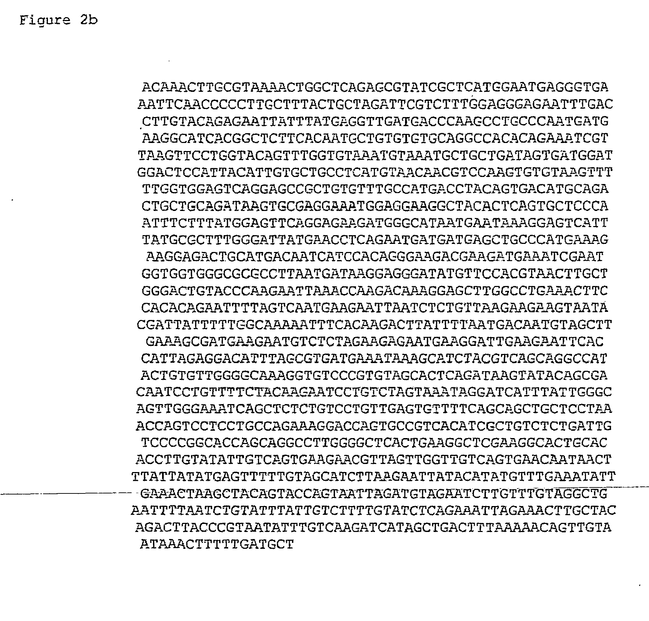

Using the cDNA sequence of ASP-2, we did a BLAST search and identified a clone named as KIAA0771 with significant homology to the nucleic acid sequence encoding bBP2/BP53 suggesting that the newly identified sequence is a member of a family of genes which encode apoptosis stimulating proteins (ASP's). This member of the family is referred to as ASP-1. Using a PCR -RACE, a techinique known in the art, we cloned 100bp of ASP-1 cDNA which is 5'-upstream to KIAA0771. The cloned 100bp sequence was used to carry out a BLAST search, which allowed us to identify another EST clone (EMBO entry AI625004) which overlaps with the 100bp sequence but contains a further 700bp 5'-sequence of ASP-1. We obtained the EST clones AI625004 and KIAA0771 and subcloned both together to generate the full length clone of ASP-1 cDNA as shown in figure 1B.

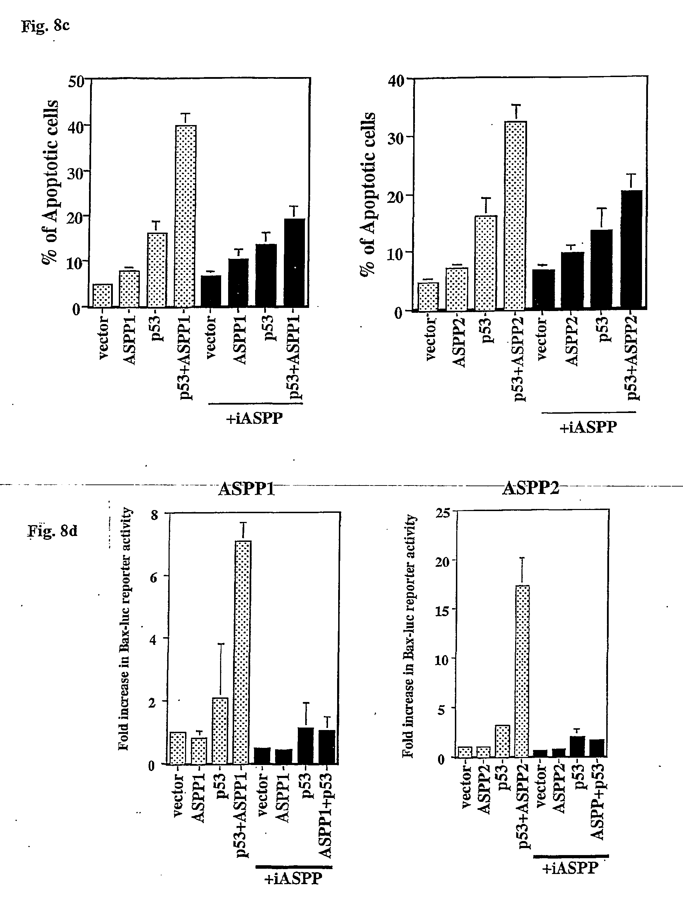

We have named the novel nucleic acid sequence ASP-1, (Apoptosis Stimulating Protein 1), which encodes a polypeptide which has sequence homology to 53BP2/bBP2. The sequence homologies between ASP-1 and ASP-2, at the level of protein sequence, is shown in Figure 8A . The highest homology between ASP-1 and ASP-2 is found in the N- and the C-terminal parts of the protein.

The chromosomal locations of these two genes were also identified. ASP-1 is encoded by a gene located on chromosome 14. The boundaries for 17 exons and introns are illustrated in figure 1C. Most of the exons and introns are within the genomic clone under the the EMBO entry AL049840. The promoter region and the 5'end exons and introns are located within the genomic clone EMBO entry CNS01DTD.

Additionally we have identified a regulator of ASP-2 which inhibits the p53-stimulatory effect of ASP-2. We have called this regulator I-ASP. In tumours expressing ASP-1 and ASP-2, the expression of I-ASP is up-regulated compared to the matched normal controls. This suggests that the tumour suppression function of p53 may be positively and negatively regulated by ASP and I-ASP *in vivo.*

According to a first aspect of the invention there is provided a polypeptide, or part thereof, comprising:
i) at least one ankyrin repeat
ii) an α helical domain;
iii) a SH3 domain; and
characterised in that said polypeptide is capable of stimulating at least the apoptotic function of p53.

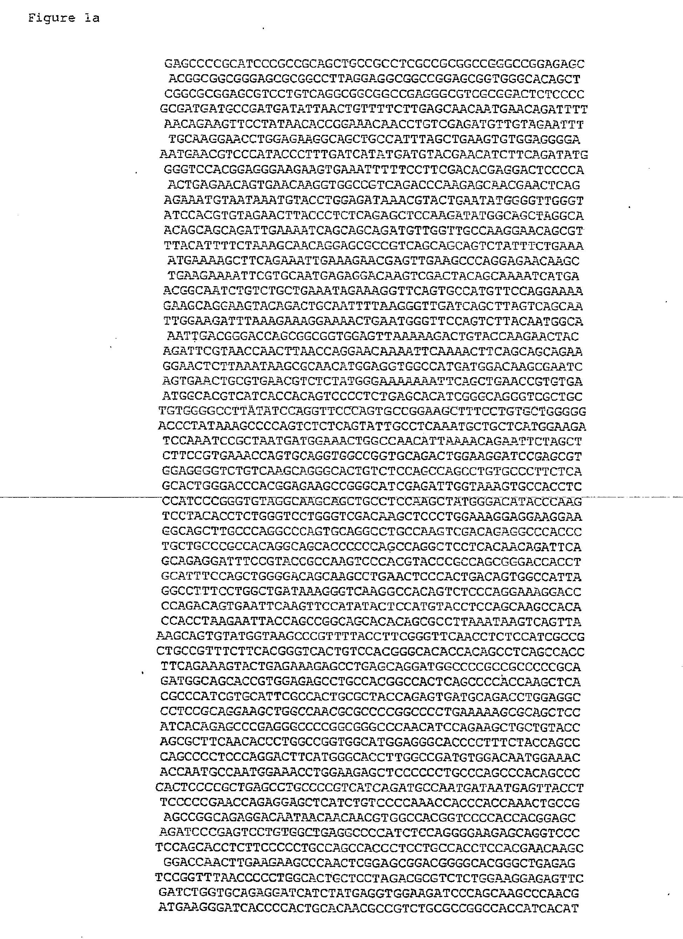

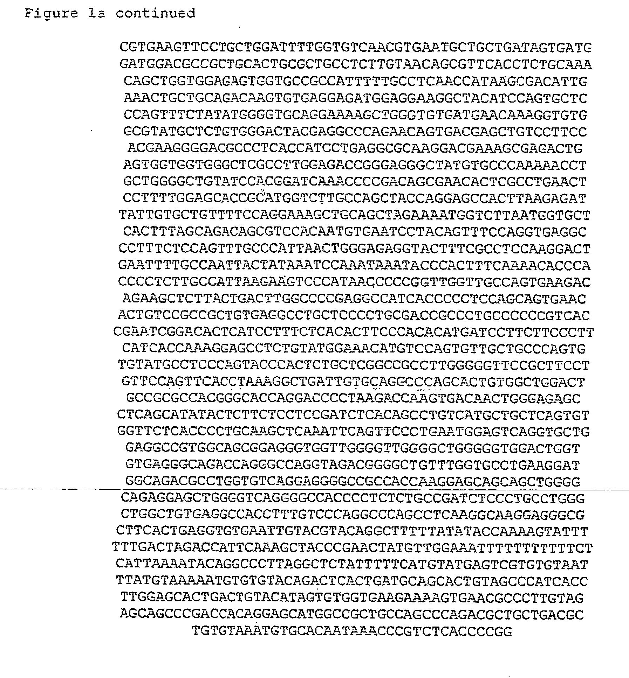

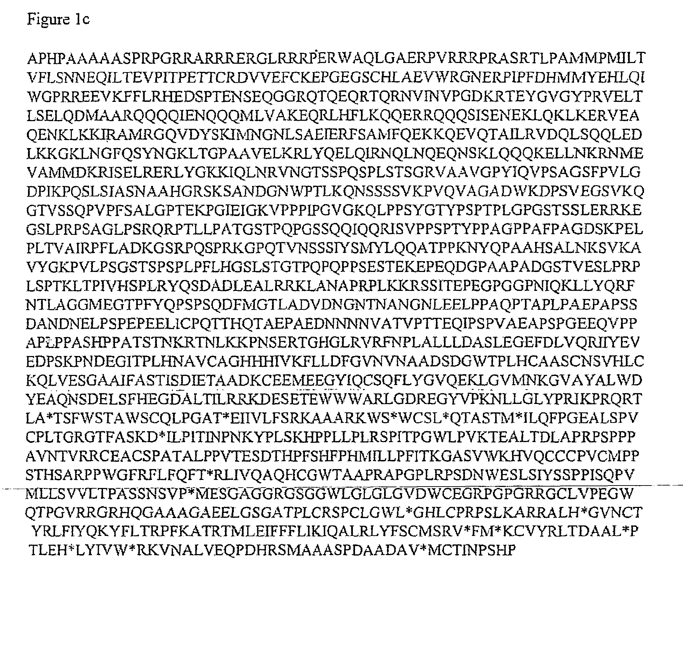

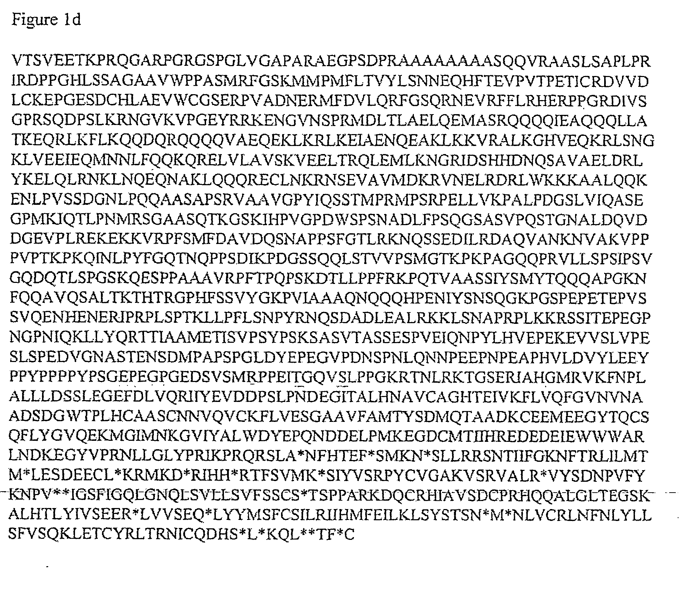

In a further preferred embodiment of the invention said polypeptide is characterised by being capable of binding to an antibody, preferably a monoclonal antibody, to at least one region of the polypeptide of sequence presented in Figure 1 c or 1 d.

In yet a further preferred embodiment of the invention said polypeptide comprises a binding site capable of binding, and thereby associating, with p53. Preferably said association is capable of inducing and/or enhancing apoptosis.

In a preferred embodiment of the invention said polypeptide is of mammalian origin, ideally human.

In a preferred embodiment of the invention said polypeptide is represented by the amino acid sequence of figure 1c or 1d, which is further modified by deletion, addition, substitution of at least one amino acid.

According to a second aspect of the invention there is provided a nucleic acid molecule comprising a DNA sequence selected from :
i) the DNA sequence as represented in Figure 1a or 1b;
ii) DNA sequences which hybridise to the sequence presented in Figure 1a or 1b which encode a tumour suppressor polypeptide according to the invention; and
iii) DNA sequences which are degenerate as a result of the genetic code to the DNA sequences defined in (i) and (ii).

In a preferred embodiment of the invention there is provided an isolated nucleic acid molecule which anneals under stringent hybridisation conditions to the sequence presented in Figure 1a or 1b.

In yet a still further preferred embodiment of the invention said nucleic acid molecule is cDNA.

In yet a still further preferred embodiment of the invention said nucleic acid molecule is genomic DNA.

In a further preferred embodiment of the invention there are provided isolated polypeptides encoded by the nucleic acid molecules according to the invention.

According to a third aspect of the invention there is provided a nucleic acid molecule characterised in that said nucleic acid molecule is part of a vector adapted to facilitate recombinant expression of the polypeptide encoded by said nucleic acid molecule.

In a further preferred embodiment of the invention said vector is an expression vector adapted for eukaryotic gene expression.

According to a fourth aspect of the invention there is provided a method for the production of the polypeptide according to the invention comprising:
i) providing a cell transformed/transfected with a nucleic acid molecule according to the invention;
ii) growing said cell in conditions conducive to the manufacture of said polypeptide; and
iii) purifying/isolating said polypeptide from said cell, or its growth environment

In a preferred embodiment of the invention said said nucleic acid molecule is the vector according to the invention.

In a preferred method of the invention said vector encodes, and thus said recombinant polypeptide is provided with, a secretion signal to facilitate purification of said polypeptide.

In a further preferred embodiment of the invention said vector encodes, and thus recombinant polypeptide is provided with an additional amino acid sequence which facilitates its purification from a cell or cell culture medium. For example a His-tag sequence which allows the binding of the recombinant polypeptide to a nickel column, or the use of biotinylated recombinant polypeptides which are purified on avidin columns, each of which are known in the art.

According to a fifth aspect of the invention there is provided an antibody or binding part thereof, binds to at least a part of the polypeptide of the invention.

In a preferred embodiment of the invention said binding part is selected from the group consisting of: F(ab')₂, Fab, Fv and Fd fragments; antibodies comprising CDR3 regions.

In a preferred embodiment of the invention said antibody is a monoclonal antibody.

In yet a further preferred embodiment of the invention said antibody is humanised.

Alternatively, said antibody is a chimeric antibody produced by recombinant methods to contain the variable region of said antibody with an invariant or constant region of a human antibody.

Chimeric antibodies are recombinant antibodies in which all of the V-regions of a mouse or rat antibody are combined with human antibody C-regions. Humanised antibodies are recombinant hybrid antibodies which fuse the complimentarity determining regions from a rodent antibody V-region with the framework regions from the human antibody V-regions. The C-regions from the human antibody are also used. The complimentarity determining regions (CDRs) are the regions within the N-terminal domain of both the heavy and light chain of the antibody to where the majority of the variation of the V-region is restricted. These regions form loops at the surface of the antibody molecule. These loops provide the binding surface between the antibody and antigen.

The production of antibodies is well known in the art. Several laboratory text books are availalable to the skilled artisan. For example, Antibodies, Lane & Harlow, Cold Spring Harbour Laboratories.

According to a sixth aspect of the invention there is provided host cells which have been transformed/transfected, ideally using the vector according to the invention, so as to include at least part of the nucleic acid molecule according to the invention, so as to permit expression of at least part, or a significant part, such as a functional fragment, of the polypeptide encoded by said nucleic acid sequence.

Ideally said host cells are eukaryotic cells, for example, insect cells such as cells from a species *Spodoptera frugiperda* using the baculovirus expression system. This expression system is favoured in the instance where post-translational modification of the polypeptide is required. If such modification is not required a prokaryotic system may be used.

According to a seventh aspect of the invention there is provided a method for determining the expression of mRNA and/or the polypeptide according to the invention.

According to an eighth aspect of the invention there is provided a pharmaceutical or veterinary composition characterised in that said composition comprises the vector according to the invention.

According to a ninth aspect of the invention there is provided a pharmaceutical or veterinary composition characterised in that said composition comprises the polypeptide according to the invention.

In a preferred embodiment of the invention said vector and/or said polypeptide optionally also includes a diluent, carrier or excipient.

According to a tenth aspect of the invention there is provided a method of treatment comprising:
i) administering to an animal an effective amount of a composition according to the invention; and
ii) monitoring the effect of said therapeutic composition on said animal.

In a preferred method of the invention said treatment is cancer therapy.

In a further preferred embodiment of the invention said animal is human.

In yet a further preferred embodiment of the invention said effect is the induction of apoptosis.

According to a further aspect of the invention there is provided method to screen for agents capable of modulating the activity of the polypeptide according to the invention comprising:
i) providing a cell or cell-line which expresses the polypeptide according to the invention;
ii) exposing the cell to at least one agent to be tested; and
iii) monitoring the effect of the agent(s) on the activity of the polypeptide.

According to yet a further aspect of the invention there is provided a method to screen for agents capable of modulating the activity of the polypeptide according to the invention comprising:
i) providing at least the polypeptide according to the invention;
ii) exposing the polypeptide to at least one agent to be tested; and
iii) monitoring the binding of said agent(s) by said polypeptide.

According to yet a further aspect of the invention there is provided an agent(s) identified by the screening methods according to the invention.

In a preferred embodiment of the invention said agent is an agonist which promotes the activity of the polypeptide according to the invention.

In a further preferred embodiment of the invention said agent is an antagonist which inhibits the activity of the polypeptide according to the invention. Preferably said agent is a polypeptide.

According to a further aspect of the invention there is provided an antisense nucleic acid molecule wherein said molecule comprises the antisense sequence of the sense sequence according to the invention. Preferably said antisense nucleic acid molecule comprises the antisense sequence represented in Figure 1b, or part thereof. Preferably said antisense nucleic acid molecule is the antisense sequence of the sense sequence comprising nucleotides -253 to 839 of the ASP-2 sequence.

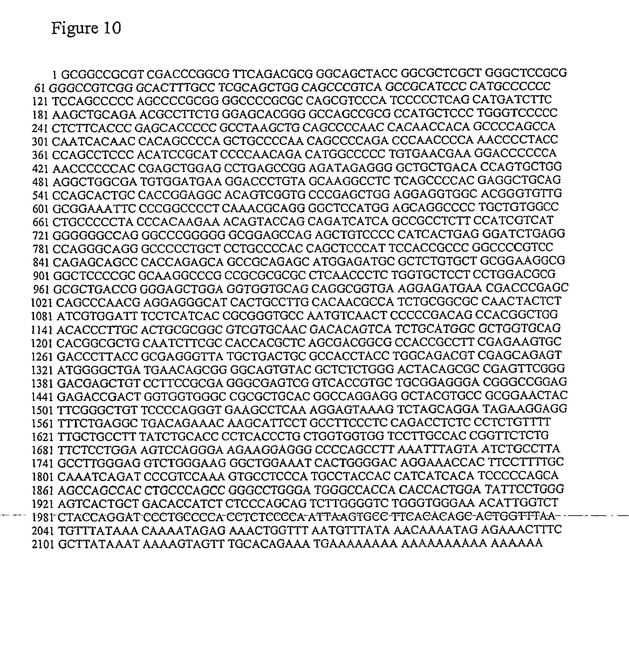

According to a further aspect of the invention there is provided an isolated nucleic acid molecule selected from the group comprising:
i) the DNA sequence as represented in Figure 10;
ii) DNA sequences which hybridise to the sequence presented in Figure 10 which encode an inhibitor of the tumour suppressor polypeptide according to the invention; and
iii) DNA sequences which are degenerate as a result of the genetic code to the DNA sequences defined in (i) and (ii).

In a preferred embodiment of the invention there is provided an isolated nucleic acid molecule which anneals under stringent hybridisation conditions to the sequence presented in Figure 10.

According to a further aspect of the invention there is provided a polypeptide, or part thereof, comprising:
i) at least one ankyrin repeat;
ii) a SH3 domain; and
characterised in that said polypeptide is capable of inhibiting the p53-stimulatory activity of the polypeptide represented in Figure 1d.

In a preferred embodiment of the invention said polypeptide also comprises a proline - rich region.

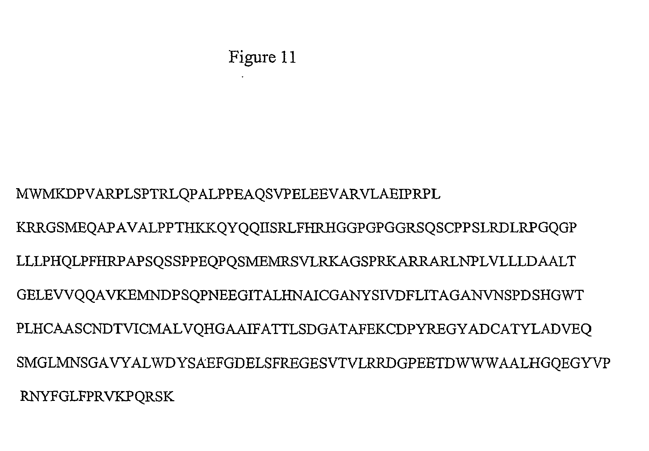

According to a yet further aspect of the invention there is provided a polypeptide, as represented by the amino acid sequence of Figure 11, which is further modified by deletion, addition, substitution of at least one amino acid. Preferably said polypeptide is of human origin.

Aspects and embodiments applicable to ASP-1 or ASP-2 are equally applicable to I-ASP. For example, the creation of expression vectors including I-ASP DNA; cell-lines transformed or transfected with nucleic acid molecules encoding I-ASP; monoclonal antibodies capable of binding to polypeptides encoded by nucleic acid molecules encoding I-ASP, or homologues thereof; pharmaceutical compositions comprising nucleic acid molecules encoding I-ASP; pharmaceutical compositions comprising I-ASP polypeptides, or homologues thereof; methods of treatment employing nucleic acid molecules encoding I-ASP or I-ASP polypeptides; methods to detect the nucleic acid encoding I-ASP, or homologues thereof; methods to detect the I-ASP polypeptides, or homologues thereof.

According to a further aspect of the invention there is provided an antisense nucleic acid molecule wherein said molecule comprises the antisense sequence of the sense sequence according to the invention. Preferably said antisense nucleic acid molecule comprises the antisense sequence of the sense sequence represented in Figure 10, or part thereof. More preferably still said antisense nucleic acid molecule is the antisense sequence of the sense sequence comprising nucleotides -37-536 of I-ASP.

According to a further aspect of the invention there is provided a pharmaceutical composition comprising an antisense molecule according to the invention.

In a preferred embodiment of the invention said antisense nucleic acid is combined with at least one chemotherapeutic agent. Preferably said agent is an anti-cancer agent selected from the group consisting of: cisplatin; carboplatin; cyclosphosphamide; melphalan; carmusline; methotrexate; 5-fluorouracil; cytarabine; mercaptopurine; daunorubicin; doxorubicin; epirubicin; vinblastine; vincristine; dactinomycin; mitomycin C; taxol; L-asparaginase; G-CSF; an enediyne such as chalicheamicin or esperamicin; chlorambucil; ARA-C; vindesine; bleomycin; and etoposide. Other agents that can be combined with the foregoing include agents that acts on the tumor neovasculature or immunomodulators. Preferably the agent that acts on the tumor neovasculature is selected from the group consisting of combrestatin A4, angiostatin and endostatin. Preferably the immunomodulator is selected from the group consisting of α-interferon, γ-interferon, and tumor necrosis factor alpha (TNFα).

In a preferred embodiment of the invention said agent is cisplatin.

According to a yet further aspect of the invention there is provided a method for the preparation of monoclonal antibodies which bind amino acids 1-130 of the sequence presented in figure 1d comprising the steps of:
(a) immunising an immunocompetent mammal with an immunogen wherein said immunogen comprises a polypeptide having the amino acid sequence as represented by amino acids 1-130 of figure 1d ;
(b) fusing lymphocytes of the immunised immunocompetent mammal with myeloma cells to form hybridoma cells;
(c) screening monoclonal antibodies produced by the hybridoma cells of step (b);
(d) culturing the hybridoma cells producing monoclonal activity to proliferate and/or to secrete said monoclonal antibody; and
(e) recovering the monoclonal antibody from the culture supernatant.

In a preferred method of the invention said immunocompetent mammal is a mouse. In an alternative preferred method A said immunocompetent mammal is a rat.

In a further preferred method of the invention said mammal is transgenic for human immunoglobulin genes or chromosomal nucleic acids containing human immunoglobulin genes.

The invention thus involves in one aspect isolated ASP-1, ASP-2 and/or I-ASP polypeptides, genes encoding those polypeptides, functional modifications and variants of the foregoing, useful fragments of the foregoing, as well as therapeutics relating thereto. The expression of these genes affects apoptosis by binding to p53 and related polypeptides.

As used herein with respect to nucleic acids, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art.

Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art. An isolated nucleic acid as used herein is not a naturally occurring chromosome.

As used herein with respect to polypeptides, "isolated" means separated from its native environment and present in sufficient quantity to permit its identification or use. Isolated, when referring to a protein or polypeptide, means, for example: (i) selectively produced by expression cloning or (ii) purified as by chromatography or electrophoresis. Isolated proteins or polypeptides may be, but need not be, substantially pure. The term "substantially pure" means that the proteins or polypeptides are essentially free of other substances with which they may be found in nature or *in vivo* systems to an extent practical and appropriate for their intended use. Substantially pure polypeptides may be produced by techniques well known in the art. Because an isolated protein may be admixed with a pharmaceutically acceptable carrier in a pharmaceutical preparation, the protein may comprise only a small percentage by weight of the preparation. The protein is nonetheless isolated in that it has been separated from the substances with which it may be associated in living systems, i.e. isolated from other proteins.

One aspect of the invention relates to those nucleic acid sequences which code for ASP-1, ASP-2 and/or I-ASP polypeptides and which hybridize to the nucleic acid molecules disclosed herein, preferably consisting of the coding region of the molecules depicted in Figures 1a, 1b or 10, under stringent conditions.

Thus, an aspect of the invention is those nucleic acid sequences which code for ASP-1, ASP-2 and/or I-ASP polypeptides and which hybridize to a nucleic acid molecule as provided herein, under stringent conditions. The term "stringent conditions" as used herein refers to parameters with which the art is familiar. Nucleic acid hybridization parameters may be found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. More specifically, stringent conditions, as used herein, refers, for example, to hybridization at 65°C in hybridization buffer (3.5 x SSC, 0.02% Ficoll, 0.02% polyvinyl pyrrolidone, 0.02% Bovine Serum Albumin, 2.5mM NaH₂PO₄(pH7), 0.5% SDS, 2mM EDTA). SSC is 0.15M sodium chloride/0.015M sodium citrate, pH7; SDS is sodium dodecyl sulphate; and EDTA is ethylenediaminetetracetic acid. After hybridization, the membrane upon which the DNA is transferred is washed at 2 x SSC at room temperature and then at 0.1 - 0.5 X SSC/0.1 x SDS at temperatures up to 68°C.

There are other conditions, reagents, and so forth which can be used, which result in a similar degree of stringency. The skilled artisan will be familiar with such conditions, and thus they are not given here. It will be understood, however, that the skilled artisan will be able to manipulate the conditions in a manner to permit the clear identification of homologs and alleles of ASP-1, ASP-2 or I-ASP nucleic acids of the invention. The skilled artisan also is familiar with the methodology for screening cells and libraries for expression of such molecules which then are routinely isolated, followed by isolation of the pertinent nucleic acid molecule and sequencing.

In general homologs and alleles typically will share at least 90% nucleotide identity and/or at least 95% amino acid identity to the disclosed nucleotide and amino acid sequences respectively, in some instances will share at least 95% nucleotide identity and/or at least 97% amino acid identity and in still other instances will share at least 98% nucleotide identity and/or at least 99% amino acid identity. The homology can be calculated using various, publicly available software tools developed by NCBI (Bethesda, Maryland) that can be obtained through the Internet (ftp:/ncbi.nlm.nih.gov/pub/).

Exemplary tools include the BLAST system available at http://www.ncbi.nlm.nih.gov, preferably using default settings. Pairwise and ClustalW alignments (BLOSUM30 matrix setting) as well as Kyle-Doolittle hydropathic analysis can be obtained using the MacVector sequence analysis software (Oxford Molecular Group). Watson-Crick complements of the foregoing nucleic acids also are embraced by the invention.

In screening for nucleic acids encoding ASP-1 ASP-2 and/or I-ASP proteins with sequence homology to the nucleic acids described herein, a Southern blot may be performed using the foregoing conditions, together with a detectable probe (e.g., radioactive, chemiluminescent). After washing the membrane to which the DNA is finally transferred, the probe signal can be detected, such as by placing the membrane against X-ray film or phosphorimager plates to detect the radioactive signal, or by processing the membrane to detect chemiluminescent signal.

The invention also includes degenerate nucleic acids which include alternative codons to those present in the native materials. For example, serine residues are encoded by the codons TCA, AGT, TCC, TCG, TCT and AGC. Each of the six codons is equivalent for the purposes of encoding a serine residue. Thus, it will be apparent to one of ordinary skill in the art that any of the serine-encoding nucleotide triplets may be employed to direct the protein synthesis apparatus, *in vitro* or *in vivo,* to incorporate a serine residue into an elongating polypeptide. Similarly, nucleotide sequence triplets which encode other amino acid residues include, but are not limited to: CCA, CCC, CCG and CCT (proline codons); CGA, CGC, CGG, CGT, AGA and AGG (arginine codons); ACA, ACC, ACG and ACT (threonine codons); AAC and AAT (asparagine codons); and ATA, ATC and ATT (isoleucine codons). Other amino acid residues may be encoded similarly by multiple nucleotide sequences. Thus, the invention embraces degenerate nucleic acids that differ from the biologically isolated nucleic acids in codon sequence due to the degeneracy of the genetic code.

The invention also provides modified nucleic acid molecules or polypeptides which include additions, substitutions and deletions of one or more nucleotides or amino acids. As used herein, "one or more" means 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more up to a number that does not substantially change the function of the molecule for those molecules in which the function is desired to be substantially similar to the original nucleic acid or polypeptide. A substantial change of function would be, for example, a dominant negative protein, or a protein which has lost one or more of its functions.

In preferred embodiments, these modified nucleic acid molecules and/or the polypeptides they encode retain at least one activity or function of the unmodified nucleic acid molecule and/or the polypeptides, such as p53 binding, antigenicity, transcriptional activity, etc. In certain embodiments, the modified nucleic acid molecules encode modified polypeptides, preferably polypeptides having conservative amino acid substitutions as are described elsewhere herein. The modified nucleic acid molecules are structurally related to the unmodified nucleic acid molecules and in preferred embodiments are sufficiently structurally related to the unmodified nucleic acid molecules so that the modified and unmodified nucleic acid molecules hybridize under highly stringent conditions known to one of skill in the art.

For example, modified nucleic acid molecules which encode polypeptides having single amino acid changes can be prepared. Each of these nucleic acid molecules can have one, two or three nucleotide substitutions exclusive of nucleotide changes corresponding to the degeneracy of the genetic code as described herein. Likewise, modified nucleic acid molecules which encode polypeptides having two amino acid changes can be prepared which have, e.g., 2-6 nucleotide changes. Numerous modified nucleic acid molecules like these will be readily envisioned by one of skill in the art, including for example, substitutions of nucleotides in codons encoding amino acids 2 and 3, 2 and 4, 2 and 5, 2 and 6, and so on. In the foregoing example, each combination of two amino acids is included in the set of modified nucleic acid molecules, as well as all nucleotide substitutions which code for the amino acid substitutions. Additional nucleic acid molecules that encode polypeptides having additional substitutions (i.e., 3 or more), additions or deletions (e.g., by introduction of a stop codon or a splice site(s)) also can be prepared and are embraced by the invention as readily envisioned by one of ordinary skill in the art. Any of the foregoing nucleic acids or polypeptides can be tested by routine experimentation for retention of structural relation or activity to the nucleic acids and/or polypeptides disclosed herein.

The invention also provides isolated fragments of ASP-1, ASP-2 and I-ASP or complements thereof of sufficient length to represent a sequence unique within the human genome, and identifying a nucleic acid encoding ASP-1, ASP-2 and I-ASP polypeptides. These fragments can be considered unique in that a unique fragment is one that is a 'signature' for the larger nucleic acid. A unique fragment, for example, is long enough to assure that its precise sequence is not found in molecules outside of the ASP-1, ASP-2 and I-ASP nucleic acids defined above, i.e., that it specifically identifies the ASP-1, ASP-2 and I-ASP sequences. A unique fragment includes a sequence of contiguous nucleotides which is not identical to any sequence present in publicly available databases (e.g., GenBank) as of the filing date of this application, although certain fragments may contain as a portion of the fragment some previously known sequence deposited in GenBank. Likewise, complements of publicly known sequences and fragments of the publicly known sequences and complements thereof can be a portion of, but not all of the unique fragments of ASP-1, ASP-2 and I-ASP. Thus a unique fragment excludes, by definition, sequences consisting solely of EST and/or gene sequences deposited in publicly available databases as of the earliest filing date of the sequences contained in this application. Thus, a unique fragment must contain a nucleotide sequence other than the exact sequence of those in GenBank or fragments thereof. The difference may be an addition, deletion or substitution with respect to the GenBank sequence or it may be a sequence wholly separate from the GenBank sequence.

Fragments of ASP-1, ASP-2 and I-ASP nucleic acid molecules, including unique fragments, can be used as probes in hybridization blot assays (e.g., Southern, Northern) to identify such nucleic acids, in nuclease protection assays to measure transcription, or can be used in amplification assays such as those employing PCR. As known to those skilled in the art, large probes such as 200 250, 300 or more nucleotides are preferred for certain uses such as Southern and Northern blots, while smaller fragments will be preferred for uses such as PCR. Fragments also can be used to produce fusion proteins for generating antibodies or determining binding of the polypeptide fragments, or for generating immunoassay components. Likewise, fragments can be employed to produce nonfused fragments of the ASP-1, ASP-2 and/or I-ASP polypeptides such as N-terminal or C-terminal fragments, or the various protein domains disclosed herein, useful, for example, in the preparation of antibodies, in immunoassays, and as a competitive binding partners of the ASP-1, ASP-2 and I-ASP polypeptides and/or other polypeptides which bind to p53 or rel polypeptides, for example, in therapeutic applications. Fragments further can be used as antisense molecules, as described herein, to inhibit the expression of ASP-1, ASP-2 and/or I-ASP nucleic acids and polypeptides, particularly for therapeutic purposes as described in greater detail herein.

As will be recognized by those skilled in the art, the size of the unique fragment will depend upon its conservancy in the genetic code. Thus, some regions of ASP-1, ASP-2 and I-ASP nucleic acid molecules and their complements will require longer segments to be unique while others will require only short segments, typically between 12 and 32 nucleotides (e.g. 12, 13, 14, 15, 16,17,18, 19, 20, 21, 22, 23, 24, 25, 26, 27, 28, 29, 30, 31 and 32 bases long). This disclosure intends to embrace each and every fragment of each sequence, beginning at the first nucleotide, the second nucleotide and so on, up to 8 nucleotides short of the end, and ending anywhere from nucleotide number 8, 9, 10 and so on for each sequence, up to the very last nucleotide (provided the sequence is unique as described above). Many segments of the ASP-1, ASP-2 and I-ASP nucleic acids, or complements thereof, that are 25 or more nucleotides in length will be unique. Those skilled in the art are well versed in methods for selecting such sequences, typically on the basis of the ability of the unique fragment to selectively distinguish the sequence of interest from non-ASP and I-ASP nucleic acids. A comparison of the sequence of the fragment to those on known databases typically is all that is necessary, although *in vitro* confirmatory hybridization and sequencing analysis may be performed.

A fragment can be a functional fragment. A functional fragment of a nucleic acid molecule of the invention is a fragment which retains some functional property of the larger nucleic acid molecule, such as coding for a functional polypeptide, binding to proteins (e.g., p53), regulating transcription of operably linked nucleic acids, coding for immunologically recognized epitopes and the like. One of ordinary skill in the art can readily determine using the assays described herein and those well known in the art to determine whether a fragment is a functional fragment of a nucleic acid molecule using no more than routine experimentation.

As mentioned above, the invention embraces antisense oligonucleotides that selectively bind to a nucleic acid molecule encoding a ASP-1, ASP-2 and I-ASP polypeptide, to modulate p53 binding, transcriptional activity or apoptosis, for example. This is desirable in virtually any medical condition wherein a modulation of p53 activity is desirable, such as cancer and conditions involving aberrant apoptosis.

As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA. The antisense molecules are designed so as to interfere with transcription or translation of a target gene upon hybridization with the target gene or transcript. Those skilled in the art will recognize that the exact length of the antisense oligonucleotide and its degree of complementarity with its target will depend upon the specific target selected, including the sequence of the target and the particular bases which comprise that sequence. It is preferred that the antisense oligonucleotide be constructed and arranged so as to bind selectively with the target under physiological conditions, i.e., to hybridize substantially more to the target sequence than to any other sequence in the target cell under physiological conditions. Based upon the ASP-1, ASP-2 or I-ASP nucleic acid sequences provided herein, or upon allelic or homologous genomic and/or cDNA sequences, one of skill in the art can easily choose and synthesize any of a number of appropriate antisense molecules for use in accordance with the present invention. For example, a "gene walk" comprising a series of oligonucleotides of 15-30 nucleotides spanning the length of a ASP-1, ASP-2 or I-ASP nucleic acid can be prepared, followed by testing for inhibition of the corresponding ASP-1, ASP-2 or I-ASP expression. Optionally, gaps of 5-10 nucleotides can be left between the oligonucleotides to reduce the number of oligonucleotides synthesized and tested.

In order to be sufficiently selective and potent for inhibition, such antisense oligonucleotides should comprise at least 10 and, more preferably, at least 15 consecutive bases which are complementary to the target, although in certain cases modified oligonucleotides as short as 7 bases in length have been used successfully as antisense oligonucleotides (Wagner et al., Nature Biotechnol. 14:840-844, 1996). Most preferably, the antisense oligonucleotides comprise a complementary sequence of 20-30 bases. Although oligonucleotides may be chosen which are antisense to any region of the gene or mRNA transcripts, in preferred embodiments the antisense oligonucleotides correspond to N-terminal or 5' upstream sites such as translation initiation, transcription initiation or promoter sites. In addition, 3'-untranslated regions may be targeted. Targeting to mRNA splicing sites has also been used in the art but may be less preferred if alternative mRNA splicing occurs. In addition, the antisense is targeted, preferably, to sites in which mRNA secondary structure is not expected (see, e.g., Sainio et al., Cell Mol. Neurobiol. 14(5):439-457, 1994) and at which proteins are not expected to bind. Finally, although the ASP-1, ASP-2 and I-ASP cDNA sequences are disclosed herein, one of ordinary skill in the art may easily derive the genomic DNA corresponding to these cDNAs. Thus, the present invention also provides for antisense oligonucleotides which are complementary to ASP-1, ASP-2 or I-ASP genomic DNA. Similarly, antisense to allelic or homologous cDNAs and genomic DNAs are enabled without undue experimentation.

In one set of embodiments, the antisense oligonucleotides of the invention may be composed of "natural" deoxyribonucleotides, ribonucleotides, or any combination thereof That is, the 5' end of one native nucleotide and the 3' end of another native nucleotide may be covalently linked, as in natural systems, via a phosphodiester internucleoside linkage. These oligonucleotides may be prepared by art recognized methods which may be carried out manually or by an automated synthesizer. They also may be produced recombinantly by vectors.

In preferred embodiments, however, the antisense oligonucleotides of the invention also may include "modified" oligonucleotides. That is, the oligonucleotides may be modified in a number of ways which do not prevent them from hybridizing to their target but which enhance their stability or targeting or which otherwise enhance their therapeutic effectiveness.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, carboxymethyl esters and peptides.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus modified oligonucleotides may include a 2'-O-alkylated ribose group. In addition, modified oligonucleotides may include sugars such as arabinose instead of ribose. The present invention, thus, contemplates pharmaceutical preparations containing modified antisense molecules that are complementary to and hybridizable with, under physiological conditions, nucleic acids encoding ASP-1, ASP-2 and/or I-ASP polypeptides, together with pharmaceutically acceptable carriers.

Antisense oligonucleotides may be administered as part of a pharmaceutical composition. Such a pharmaceutical composition may include the antisense oligonucleotides in combination with any standard physiologically and/or pharmaceutically acceptable carriers which are known in the art. The compositions should be sterile and contain a therapeutically effective amount of the antisense oligonucleotides in a unit of weight or volume suitable for administration to a patient. The characteristics of the carrier will depend on the route of administration. Physiologically and pharmaceutically acceptable carriers include diluents, fillers, salts, buffers, stabilizers, solubilizers, and other materials which are well known in the art.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes. A cloning vector is one which is able to replicate in a host cell, and which typically is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., β-galactosidase, luciferase or alkaline phosphatase), and genes which visibly affect the phenotype of transformed or transfected cells, hosts, colonies or plaques (e.g., various fluorescent proteins such as green fluorescent protein, GFP). Preferred vectors are those capable of autonomous replication and expression of the structural gene products present in the DNA segments to which they are operably joined.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. In particular, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g., Sambrook et al., Molecular Cloning: A Laboratory Manual, Second Edition, Cold Spring Harbor Laboratory Press, 1989. Cells are genetically engineered by the introduction into the cells of heterologous DNA (RNA) encoding a ASP-1, ASP-2 and/or I-ASP polypeptide or fragment or variant thereof. That heterologous DNA (RNA) is placed under operable control of transcriptional elements to permit the expression of the heterologous DNA in the host cell.

Preferred systems for mRNA expression in mammalian cells are those such as pcDNA3.1 and pRc/CMV (available from Invitrogen, Carlsbad, CA) that contain a selectable marker such as a gene that confers G418 resistance (which facilitates the selection of stably transfected cell lines) and the human cytomegalovirus (CMV) enhancer-promoter sequences. Additionally, suitable for expression in primate or canine cell lines is the pCEP4 vector (Invitrogen), which contains an Epstein Barr virus (EBV) origin of replication, facilitating the maintenance of plasmid as a multicopy extrachromosomal element. Another expression vector is the pEF-BOS plasmid containing the promoter of polypeptide Elongation Factor 1α, which stimulates efficiently transcription *in vitro.* The plasmid is described by Mishizuma and Nagata (Nuc. Acids Res. 18:5322, 1990), and its use in transfection experiments is disclosed by, for example, Demoulin (Mol. Cell. Biol. 16:4710-4716, 1996). Still another preferred expression vector is an adenovirus, described by Stratford-Perricaudet, which is defective for E1 and E3 proteins (J. Clin. Invest. 90:626-630, 1992). The use of the adenovirus as an Adeno.P1A recombinant is disclosed by Warnier et al., in intradermal injection in mice for immunization against P1A (Int. J. Cancer, 67:303-310, 1996).

The invention also embraces so-called expression kits, which allow the artisan to prepare a desired expression vector or vectors. Such expression kits include at least separate portions of each of the previously discussed coding sequences. Other components may be added, as desired, as long as the previously mentioned sequences, which are required, are included.

The invention also permits the construction of pcDNA3gene "knock-outs" in cells and in animals, providing materials for studying certain aspects of p53 activity, apoptosis, and cancer.

The invention also provides isolated polypeptides, which include the disclosed ASP-1, ASP-2 and I-ASP polypeptides and fragments thereof. Such polypeptides are useful, for example, alone or as fusion proteins to test and modulate p53 binding, to modulate apoptosis, to generate antibodies, and as a components of an immunoassay.

A fragment of an ASP-1, ASP-2 or I-ASP polypeptide, in general, has the features and characteristics of fragments as discussed above in connection with nucleic acids. As will be recognized by those skilled in the art, the size of a unique fragment will depend upon factors such as whether the fragment constitutes a portion of a conserved protein domain. Thus, some regions of ASP-1, ASP-2 and/or I-ASP polypeptides will require longer segments to be unique while others will require only short segments, typically between 5 and 12 amino acids (e.g. 5, 6, 7, 8, 9, 10, 11 and 12 amino acids long).

Fragments of an ASP-1, ASP-2 and/or I-ASP polypeptide preferably are those fragments which retain a distinct functional capability of the polypeptide. Functional capabilities which can be retained in a unique fragment of a polypeptide include binding of p53 or rel, interaction with antibodies, and enzymatic activity. For example, as exemplified herein, certain fragments of ASP-1, ASP-2 and/or I-ASP polypeptides can be used as a functional equivalent of full length ASP-1, ASP-2 and/or I-ASP polypeptide in the methods of the invention, including e.g., binding p53, modulation of apoptosis, etc. Other ASP-1, ASP-2 and/or I-ASP polypeptide fragments can be selected according to their functional properties. For example, one of ordinary skill in the art can prepare ASP-1, ASP-2 and/or I-ASP fragments recombinantly and test those fragments according to the methods exemplified below, such as binding to a p53 polypeptide. Those skilled in the art also are well versed in methods for selecting unique amino acid sequences, typically on the basis of the ability of the unique fragment to selectively distinguish the sequence of interest from non-family members. A comparison of the sequence of the fragment to those on known data bases typically is all that is necessary.

The invention embraces variants of the ASP-1, ASP-2 and I-ASP polypeptides described above. As used herein, a "variant" of a ASP-1, ASP-2 or I-ASP polypeptide is a polypeptide which contains one or more modifications to the primary amino acid sequence of the polypeptide. Modifications which create a variant can be made to a ASP-1, ASP-2 and/or I-ASP polypeptide 1) to reduce or eliminate an activity of a ASP-1, ASP-2 or I-ASP polypeptide, such as binding to another polypeptide; 2) to enhance a property of a ASP-1, ASP-2 or I-ASP polypeptide, such as protein stability in an expression system or the stability of protein-protein binding; or 3) to provide a novel activity or property to a ASP-1, ASP-2 or I-ASP polypeptide, such as addition of an antigenic epitope or addition of a detectable moiety. Modifications to an ASP-1, ASP-2 or I-ASP polypeptide are typically made to the nucleic acid which encodes the ASP-1, ASP-2 or I-ASP polypeptide, and can include deletions, point mutations, truncations, amino acid substitutions and additions of one or more amino acids or non-amino acid moieties. As used in connection with variants, "one or more" means 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more changes. Alternatively, modifications can be made directly to the polypeptide, such as by cleavage, addition of a linker molecule, addition of a detectable moiety, such as biotin, addition of a fatty acid, and the like. Modifications also embrace fusion proteins comprising all or part of a ASP-1, ASP-2 or I-ASP amino acid sequence.

In general, variants include ASP-1, ASP-2 or I-ASP polypeptides which are modified specifically to alter a feature of the polypeptide unrelated to its physiological activity. For example, cysteine residues can be substituted or deleted to prevent unwanted disulfide linkages. Similarly, certain amino acids can be changed to enhance expression of a ASP-1, ASP-2 or I-ASP polypeptide by eliminating proteolysis by proteases in an expression system (e.g., dibasic amino acid residues in yeast expression systems in which KEX2 protease activity is present).

Mutations of a nucleic acid which encode a ASP-1, ASP-2 or I-ASP polypeptide preferably preserve the amino acid reading frame of the coding sequence, and preferably do not create regions in the nucleic acid which are likely to hybridize to form secondary structures, such a hairpins or loops, which can be deleterious to expression of the variant polypeptide.

Mutations can be made by selecting an amino acid substitution, or by random mutagenesis of a selected site in a nucleic acid which encodes the polypeptide. Variant polypeptides are then expressed and tested for one or more activities to determine which mutation provides a variant polypeptide with the desired properties.

Further mutations can be made to variants (or to non-variant ASP-1, ASP-2 or I-ASP polypeptides) which are silent as to the amino acid sequence of the polypeptide, but which provide preferred codons for translation in a particular host. The preferred codons for translation of a nucleic acid in, e.g., *E. coli,* are well known to those of ordinary skill in the art. Still other mutations can be made to the noncoding sequences of an ASP-1, ASP-2 or I-ASP gene or cDNA clone to enhance expression of the polypeptide. The activity of variants of ASP-1, ASP-2 or I-ASP polypeptides can be tested by cloning the nucleic acid molecule encoding the variant ASP-1, ASP-2 or I-ASP polypeptide into a bacterial or mammalian expression vector, introducing the vector into an appropriate host cell, expressing the variant ASP-1, ASP-2 or I-ASP polypeptide, and testing for a functional capability of the ASP-1, ASP-2 or I-ASP polypeptides as disclosed herein. For example, a variant ASP polypeptide can be tested for p53 binding as disclosed in the Examples. Preparation of other variant polypeptides may favor testing of other activities, as will be known to one of ordinary skill in the art.

The skilled artisan will also realize that conservative amino acid substitutions may be made in ASP-1, ASP-2 or I-ASP polypeptides to provide functionally equivalent variants of the foregoing polypeptides, i.e, the variants retain the functional capabilities of the ASP-1, ASP-2 or I-ASP polypeptides. As used herein, a "conservative amino acid substitution" refers to an amino acid substitution which does not alter the relative charge or size characteristics of the protein in which the amino acid substitution is made. Variants can be prepared according to methods for altering polypeptide sequence known to one of ordinary skill in the art such as are found in references which compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Exemplary functionally equivalent variants of the ASP-1, ASP-2 or I-ASP polypeptides include one or more conservative amino acid substitutions of the amino acid sequences disclosed herein. Conservative substitutions of amino acids include substitutions made amongst amino acids within the following groups: (a) M, I, L, V; (b) F, Y, W; (c) K, R, H; (d) A, G; (e) S, T; (f) Q, N; and (g) E, D.

Conservative amino-acid substitutions in the amino acid sequence of ASP-1, ASP-2 or I-ASP polypeptides to produce functionally equivalent variants of these polypeptides typically are made by alteration of a nucleic acid encoding an ASP-1, ASP-2 or I-ASP polypeptide. Such substitutions can be made by a variety of methods known to one of ordinary skill in the art. For example, amino acid substitutions may be made by PCR-directed mutation, site-directed mutagenesis according to the method of Kunkel (Kunkel, Proc. Nat. Acad. Sci. U.S.A. 82: 488-492, 1985), or by chemical synthesis of a gene encoding an ASP-1, ASP-2 or I-ASP polypeptide. Where amino acid substitutions are made to a small unique fragment of an ASP-1, ASP-2 or I-ASP polypeptide, such as a p53 binding site peptide, the substitutions can be made by directly synthesizing the peptide. The activity of functionally equivalent fragments of ASP-1, ASP-2 or I-ASP polypeptides can be tested by cloning the gene encoding the altered ASP-1, ASP-2 or I-ASP polypeptide into a bacterial or mammalian expression vector, introducing the vector into an appropriate host cell, expressing the altered ASP-1, ASP-2 or I-ASP polypeptide, and testing for a functional capability of the ASP-1, ASP-2 or I-ASP polypeptides as disclosed herein. Peptides which are chemically synthesized can be tested directly for function, e.g., for binding to p53.

The invention as described herein has a number of uses, some of which are described elsewhere herein. First, the invention permits isolation of the complete ASP-1, ASP-2 or I-ASP protein molecules. A variety of methodologies well-known to the skilled practitioner can be utilized to obtain isolated the ASP-1, ASP-2 or I-ASP polypeptide molecules. The polypeptide may be purified from cells which naturally produce the polypeptide by chromatographic means or immunological recognition. Alternatively, an expression vector may be introduced into cells to cause production of the polypeptide. In another method, mRNA transcripts may be microinjected or otherwise introduced into cells to cause production of the encoded polypeptide.

Translation of mRNA in cell-free extracts such as the reticulocyte lysate system also may be used to produce polypeptide. Those skilled in the art also can readily follow known methods for isolating ASP-1, ASP-2 or I-ASP polypeptides. These include, but are not limited to, immunochromatography, HPLC, size-exclusion chromatography, ion-exchange chromatography and immune-affinity chromatography.

The isolation of the ASP-1, ASP-2 and I-ASP nucleic acid molecules also makes it possible for the artisan to diagnose a disorder characterized by expression (or relative lack thereof) of these molecules. These methods involve determining expression of the ASP-1, ASP-2 or I-ASP nuclei acids, and/or polypeptides derived therefrom. In the former situation, such determinations can be carried out via any standard nucleic acid determination assay, including the polymerase chain reaction as exemplified in the examples below, or assaying with labeled hybridization probes.

The invention also makes it possible isolate proteins such as p53 and rel by the binding of such proteins to ASP-1, ASP-2 or I-ASP as disclosed herein. The identification of the ASP-1, ASP-2 and I-ASP binding activity, also permits one of skill in the art to modulate protein binding and downstream functions, such as apoptosis. Additional uses are described herein.

The invention also provides, in certain embodiments, "dominant negative" polypeptides derived from ASP-1, ASP-2 or I-ASP. A dominant negative polypeptide is an inactive variant of a protein, which, by interacting with the cellular machinery, displaces an active protein from its interaction with the cellular machinery or competes with the active protein, thereby reducing the effect of the active protein. For example, a dominant negative receptor which binds a ligand but does not transmit a signal in response to binding of the ligand can reduce the biological effect of expression of the ligand. Likewise, a dominant negative catalytically-inactive kinase which interacts normally with target proteins but does not phosphorylate the target proteins can reduce phosphorylation of the target proteins in response to a cellular signal. Similarly, a dominant negative transcription factor which binds to another transcription factor or to a promoter site in the control region of a gene but does not increase gene transcription can reduce the effect of a normal transcription factor by occupying promoter binding sites without increasing transcription.

The end result of the expression of a dominant negative polypeptide in a cell is a reduction in function of active proteins. One of ordinary skill in the art can assess the potential for a dominant negative variant of a protein, and using standard mutagenesis techniques to create one or more dominant negative variant polypeptides. For example, given the teachings contained herein of ASP-1, ASP-2 and I-ASP polypeptides, one of ordinary skill in the art can modify the sequence of the ASP-1, ASP-2 or I-ASP polypeptides by site-specific mutagenesis, scanning mutagenesis, partial gene deletion or truncation, and the like. See, e.g., U.S. Patent No. 5,580,723 and Sambrook et al., Molecular Cloning: A Laboratory Manual, Second Edition, Cold Spring Harbor Laboratory Press, 1989. The skilled artisan then can test the population of mutagenized polypeptides for diminution in a selected activity (e.g., p53 binding, modulation of apoptosis) and/or for retention of such an activity. Other similar methods for creating and testing dominant negative variants of a protein will be apparent to one of ordinary skill in the art.

The invention also involves agents such as polypeptides which bind to ASP-1, ASP-2 or I-ASP polypeptides and to complexes of ASP-1, ASP-2 or I-ASP polypeptides and binding partners such as p53. Such binding agents can be used, for example, in screening assays to detect the presence or absence of ASP-1, ASP-2 or I-ASP polypeptides and complexes of ASP-1, ASP-2 or I-ASP polypeptides and their binding partners and in purification protocols to isolate ASP-1, ASP-2 or I-ASP polypeptides and complexes of ASP-1, ASP-2 or I-ASP polypeptides and their binding partners. Such agents also can be used to inhibit the native activity of the ASP-1, ASP-2 or I-ASP polypeptides or their binding partners, for example, by binding to such polypeptides, or their binding partners or both.

The invention, therefore, embraces peptide binding agents which, for example, can be antibodies or fragments of antibodies having the ability to selectively bind to ASP-1, ASP-2 or I-ASP polypeptides. Antibodies include polyclonal and monoclonal antibodies, prepared according to conventional methodology.

Significantly, as is well-known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated a Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. See, e.g., U.S. patents 4,816,567, 5,225,539, 5,585,089, 5,693,762 and 5,859,205.

Thus, for example, PCT International Publication Number WO 92/04381 teaches the production and use of humanized murine RSV antibodies in which at least a portion of the murine FR regions have been replaced by FR regions of human origin. Such antibodies, including fragments of intact antibodies with antigen-binding ability, are often referred to as "chimeric" antibodies.

Fully human monoclonal antibodies also can be prepared, for example, by immunization of non-human animals transgenic for human immunoglobulin genes. See, for example, U.S. patents 5,814,318, 5,877,397, 6,091,001, 6,114,598.

Thus, as will be apparent to one of ordinary skill in the art, the present invention also provides for F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

Thus, the invention involves polypeptides of numerous size and type that bind specifically to ASP-1, ASP-2 or I-ASP polypeptides, and complexes of both ASP-1, ASP-2 or I-ASP polypeptides and their binding partners. These polypeptides may be derived also from sources other than antibody technology. For example, such polypeptide binding agents can be provided by degenerate peptide libraries which can be readily prepared in solution, in immobilized form or as phage display libraries. Combinatorial libraries also can be synthesized of peptides containing one or more amino acids. Libraries further can be synthesized of peptoids and non-peptide synthetic moieties.

Phage display can be particularly effective in identifying binding peptides useful according to the invention. Briefly, one prepares a phage library (using e.g. m13, fd, or lambda phage), displaying inserts from 4 to about 80 amino acid residues using conventional procedures. The inserts may represent, for example, a completely degenerate or biased array. One then can select phage-bearing inserts which bind to the ASP-1, ASP-2 or I-ASP polypeptide. This process can be repeated through several cycles of reselection of phage that bind to the ASP-1, ASP-2 or I-ASP polypeptide. Repeated rounds lead to enrichment of phage bearing particular sequences. DNA sequence analysis can be conducted to identify the sequences of the expressed polypeptides. The minimal linear portion of the sequence that binds to the ASP-1, ASP-2 or I-ASP polypeptide can be determined. One can repeat the procedure using a biased library containing inserts containing part or all of the minimal linear portion plus one or more additional degenerate residues upstream or downstream thereof. Yeast two-hybrid screening methods also may be used to identify polypeptides that bind to the ASP-1, ASP-2 or I-ASP polypeptides. Thus, the ASP-1, ASP-2 and I-ASP polypeptides of the invention, or fragments thereof, can be used to screen peptide libraries, including phage display libraries, to identify and select peptide binding partners of the ASP-1, ASP-2 or I-ASP polypeptides of the invention. Such molecules can be used, as described, for screening assays, for purification protocols, for interfering directly with the functioning of ASP-1, ASP-2 or I-ASP and for other purposes that will be apparent to those of ordinary skill in the art.

It will also be recognized that the invention embraces the use of ASP-1, ASP-2 or I-ASP cDNAs sequences in expression vectors, as well as to transfect host cells and cell lines, be these prokaryotic (e.g., *E. coli*), or eukaryotic (e.g., CHO cells, COS cells, yeast expression systems and recombinant baculovirus expression in insect cells). Especially useful are mammalian cells such as human, mouse, hamster, pig, goat, primate, etc. They may be of a wide variety of tissue types, and include primary cells and cell lines. Specific examples include keratinocytes, peripheral blood leukocytes, fibroblasts, bone marrow stem cells and embryonic stem cells. The expression vectors require that the pertinent sequence, i.e., those nucleic acids described above, be operably linked to a promoter.

The invention also includes transgenic non-human animals. As used herein, "transgenic non-human animals" includes non-human animals having one or more exogenous nucleic acid molecules incorporated in germ line cells and/or somatic cells. Thus the transgenic animal include "knockout" animals having a homozygous or heterozygous gene disruption by homologous recombination, animals having episomal or chromosomally incorporated expression vectors, etc. Knockout animals can be prepared by homologous recombination using embryonic stem cells as is well known in the art. The recombination can be facilitated by the cre/lox system or other recombinase systems known to one of ordinary skill in the art. In certain embodiments, the recombinase system itself is expressed conditionally, for example, in certain tissues or cell types, at certain embryonic or post-embryonic developmental stages, inducibly by the addition of a compound which increases or decreases expression, and the like. In general, the conditional expression vectors used in such systems use a variety of promoters which confer the desired gene expression pattern (e.g., temporal or spatial). Conditional promoters also can be operably linked to ASP-1, ASP-2 or I-ASP nucleic acid molecules to increase expression of these nucleic acid molecules in a regulated or conditional manner. *Trans-acting* negative regulators of ASP-1, ASP-2 or I-ASP activity or expression also can be operably linked to a conditional promoter as described above. Such *trans*-acting regulators include antisense nucleic acids molecules, nucleic acid molecules which encode dominant negative molecules, ribozyme molecules specific for ASP-1, ASP-2 or I-ASP nucleic acids, and the like. The transgenic non-human animals are useful in experiments directed toward testing biochemical or physiological effects of diagnostics or therapeutics for conditions characterized by increased or decreased ASP-1, ASP-2 or I-ASP expression. Other uses will be apparent to one of ordinary skill in the art.

The invention also contemplates gene therapy. The procedure for performing *ex vivo* gene therapy is outlined in U.S. Patent 5,399,346 and in exhibits submitted in the file history of that patent, all of which are publicly available documents. In general, it involves introduction *in vitro* of a functional copy of a gene into a cell(s) of a subject which contains a defective copy of the gene, and returning the genetically engineered cell(s) to the subject. The functional copy of the gene is under operable control of regulatory elements which permit expression of the gene in the genetically engineered cell(s). Numerous transfection and transduction techniques as well as appropriate expression vectors are well known to those of ordinary skill in the art; some of which are described in PCT application WO95/00654. *In vivo* gene therapy using vectors such as adenovirus, retroviruses, herpes virus, and targeted liposomes also is contemplated according to the invention.

The invention further provides efficient methods of identifying pharmacological agents or lead compounds for agents active at the level of a ASP-1, ASP-2 or I-ASP modulatable cellular function. In particular, such functions include p53 binding, and apoptosis. Generally, the screening methods involve assaying for compounds which interfere with a ASP-1, ASP-2 or I-ASP activity such as p53 binding, etc, although compounds which enhance ASP-1, ASP-2 or I-ASP activity also can be assayed using the screening methods. Such methods are adaptable to automated, high throughput screening of compounds. The target therapeutic indications for pharmacological agents detected by the screening methods are limited only in that the target cellular function be subject to modulation by alteration of the formation of a complex comprising a ASP-1, ASP-2 or I-ASP polypeptide or fragment thereof and one or more natural ASP-1, ASP-2 or I-ASP intracellular binding targets, such as p53. Target indications include apoptosis.

A wide variety of assays for pharmacological agents are provided, including, labeled *in vitro* protein-protein binding assays, electrophoretic mobility shift assays, immunoassays, cell-based assays such as two- or three-hybrid screens, expression assays, etc. For example, hybrid screens are used to rapidly examine the effect of transfected nucleic acids on the intracellular binding of ASP-1, ASP-2 or I-ASP polypeptides or fragments thereof to specific intracellular targets. The transfected nucleic acids can encode, for example, combinatorial peptide libraries or antisense molecules. Convenient reagents for such assays, e.g., GAL4 fusion proteins, are known in the art. An exemplary cell-based assay involves transfecting a cell with a nucleic acid encoding a ASP polypeptide fused to a GAL4 DNA binding domain and a nucleic acid encoding a p53 domain which interacts with ASP fused to a transcription activation domain such as VP 16. The cell also contains a reporter gene operably linked to a gene expression regulatory region, such as one or more GAL4 binding sites. Activation of reporter gene transcription occurs when the ASP and p53 fusion polypeptides bind such that the GAL4 DNA binding domain and the VP16 transcriptional activation domain are brought into proximity to enable transcription of the reporter gene. Agents which modulate a ASP polypeptide mediated cell function are then detected through a change in the expression of reporter gene. Methods for determining changes in the expression of a reporter gene are known in the art.

ASP-1, ASP-2 or I-ASP fragments used in the methods, when not produced by a transfected nucleic acid are added to an assay mixture as an isolated polypeptide.

ASP-1, ASP-2 or I-ASP polypeptides preferably are produced recombinantly, although such polypeptides may be isolated from biological extracts. Recombinantly produced ASP-1, ASP-2 or I-ASP polypeptides include chimeric proteins comprising a fusion of a ASP-1, ASP-2 or I-ASP protein with another polypeptide, e.g., a polypeptide capable of providing or enhancing protein-protein binding, sequence specific nucleic acid binding (such as GAL4), enhancing stability of the ASP-1, ASP-2 or I-ASP polypeptide under assay conditions, or providing a detectable moiety, such as green fluorescent protein or Flag epitope.

The assay mixture is comprised of a natural intracellular ASP binding target such as p53 or a fragment thereof capable of interacting with ASP. While natural ASP binding targets may be used, it is frequently preferred to use portions (e.g., peptides or nucleic acid fragments) or analogs (i.e., agents which mimic the ASP binding properties of the natural binding target for purposes of the assay) of the ASP binding target so long as the portion or analog provides binding affinity and avidity to the ASP fragment measurable in the assay.

The assay mixture also comprises a candidate pharmacological agent. Typically, a plurality of assay mixtures are run in parallel with different agent concentrations to obtain a different response to the various concentrations. Typically, one of these concentrations serves as a negative control, i.e., at zero concentration of agent or at a concentration of agent below the limits of assay detection. Candidate agents encompass numerous chemical classes, although typically they are organic compounds. Preferably, the candidate pharmacological agents are small organic compounds, i.e., those having a molecular weight of more than 50 yet less than about 2500, preferably less than about 1000 and, more preferably, less than about 500. Candidate agents comprise functional chemical groups necessary for structural interactions with polypeptides and/or nucleic acids, and typically include at least an amine, carbonyl, hydroxyl or carboxyl group, preferably at least two of the functional chemical groups and more preferably at least three of the functional chemical groups. The candidate agents can comprise cyclic carbon or heterocyclic structure and/or aromatic or polyaromatic structures substituted with one or more of the above-identified functional groups. Candidate agents also can be biomolecules such as peptides, saccharides, fatty acids, sterols, isoprenoids, purines, pyrimidines, derivatives or structural analogs of the above, or combinations thereof and the like. Where the agent is a nucleic acid, the agent typically is a DNA or RNA molecule, although modified nucleic acids as defined herein are also contemplated.

Candidate agents are obtained from a wide variety of sources including libraries of synthetic or natural compounds. For example, numerous means are available for random and directed synthesis of a wide variety of organic compounds and biomolecules, including expression of randomized oligonucleotides, synthetic organic combinatorial libraries, phage display libraries of random peptides, and the like. Alternatively, libraries of natural compounds in the form of bacterial, fungal, plant and animal extracts are available or readily produced. Additionally, natural and synthetically produced libraries and compounds can be readily be modified through conventional chemical, physical, and biochemical means. Further, known pharmacological agents may be subjected to directed or random chemical modifications such as acylation, alkylation, esterification, amidification, etc. to produce structural analogs of the agents.

A variety of other reagents also can be included in the mixture. These include reagents such as salts, buffers, neutral proteins (e.g., albumin), detergents, etc. which may be used to facilitate optimal protein-protein and/or protein-nucleic acid binding. Such a reagent may also reduce non-specific or background interactions of the reaction components. Other reagents that improve the efficiency of the assay such as protease, inhibitors, nuclease inhibitors, antimicrobial agents, and the like may also be used.

The mixture of the foregoing assay materials is incubated under conditions whereby, but for the presence of the candidate pharmacological agent, the ASP-1, ASP-2 or I-ASP polypeptide specifically binds the cellular binding target, a portion thereof or analog thereof. The order of addition of components, incubation temperature, time of incubation, and other perimeters of the assay may be readily determined. Such experimentation merely involves optimization of the assay parameters, not the fundamental composition of the assay. Incubation temperatures typically are between 4°C and 40°C. Incubation times preferably are minimized to facilitate rapid, high throughput screening, and typically are between 0.1 and 10 hours.

After incubation, the presence or absence of specific binding between the ASP-1, ASP-2 or I-ASP polypeptide and one or more binding targets is detected by any convenient method available to the user. For cell free binding type assays, a separation step is often used to separate bound from unbound components. The separation step may be accomplished in a variety of ways. Conveniently, at least one of the components is immobilized on a solid substrate, from which the unbound components may be easily separated. The solid substrate can be made of a wide variety of materials and in a wide variety of shapes, e.g., microtiter plate, microbead, dipstick, resin particle, etc. The substrate preferably is chosen to maximum signal to noise ratios, primarily to minimize background binding, as well as for ease of separation and cost.

Separation may be effected for example, by removing a bead or dipstick from a reservoir, emptying or diluting a reservoir such as a microtiter plate well, rinsing a bead, particle, chromatographic column or filter with a wash solution or solvent. The separation step preferably includes multiple rinses or washes. For example, when the solid substrate is a microtiter plate, the wells may be washed several times with a washing solution, which typically includes those components of the incubation mixture that do not participate in specific bindings such as salts, buffer, detergent, non-specific protein, etc. Where the solid substrate is a magnetic bead, the beads may be washed one or more times with a washing solution and isolated using a magnet.

Detection may be effected in any convenient way for cell-based assays such as two- or three-hybrid screens. The transcript resulting from a reporter gene transcription assay of ASP-1, ASP-2 or I-ASP polypeptide interacting with a target molecule typically encodes a directly or indirectly detectable product, e.g., β-galactosidase activity, luciferase activity, and the like. For cell free binding assays, one of the components usually comprises, or is coupled to, a detectable label. A wide variety of labels can be used, such as those that provide direct detection (e.g., radioactivity, luminescence, optical or electron density, etc). or indirect detection (e.g., epitope tag such as the FLAG epitope, enzyme tag such as horseradish peroxidase, etc.). The label may be bound to a ASP-1, ASP-2 or I-ASP binding partner, or incorporated into the structure of the binding partner.

A variety of methods may be used to detect the label, depending on the nature of the label and other assay components. For example, the label may be detected while bound to the solid substrate or subsequent to separation from the solid substrate. Labels may be directly detected through optical or electron density, radioactive emissions, nonradiative energy transfers, etc. or indirectly detected with antibody conjugates, strepavidin-biotin conjugates, etc. Methods for detecting the labels are well known in the art. The invention provides ASP-1, ASP-2 or I-ASP-specific binding agents, methods of identifying and making such agents, and their use in diagnosis, therapy and pharmaceutical development. For example, ASP-1, ASP-2 or I-ASP-specific pharmacological agents are useful in a variety of diagnostic and therapeutic applications, especially where disease or disease prognosis is associated with improper utilization of a pathway involving ASP, e.g., apoptosis, etc. Novel ASP-1, ASP-2 or I-ASP-specific binding agents include ASP-1, ASP-2 or I-ASP-specific antibodies and other natural intracellular binding agents identified with assays such as two hybrid screens, and non-natural intracellular binding agents identified in screens of chemical libraries and the like.

In general, the specificity of ASP-1, ASP-2 or I-ASP binding to a binding agent is shown by binding equilibrium constants. Targets which are capable of selectively binding an ASP-1, ASP-2 or I-ASP polypeptide preferably have binding equilibrium constants of at least about 10⁷ M⁻¹, more preferably at least about 10⁸ M⁻¹, and most preferably at least about 10⁹ M⁻¹. The wide variety of cell based and cell free assays may be used to demonstrate ASP-1, ASP-2 or I-ASP-specific binding. Cell based assays include one, two and three hybrid screens, assays in which ASP-1, ASP-2 or I-ASP-mediated transcription is inhibited or increased, etc. Cell free assays include ASP-1, ASP-2 or I-ASP-protein binding assays, immunoassays, etc. Other assays useful for screening agents which bind ASP-1, ASP-2 or I-ASP polypeptides include fluorescence resonance energy transfer (FRET), and electrophoretic mobility shift analysis (EMSA).

Various techniques may be employed for introducing nucleic acids of the invention into cells, depending on whether the nucleic acids are introduced *in vitro* or *in vivo* in a host. Such techniques include transfection of nucleic acid-CaPO₄ precipitates, transfection of nucleic acids associated with DEAE, transfection with a retrovirus including the nucleic acid of interest, liposome mediated transfection, and the like. For certain uses, it is preferred to target the nucleic acid to particular cells. In such instances, a vehicle used for delivering a nucleic acid of the invention into a cell (e.g., a retrovirus, or other virus; a liposome) can have a targeting molecule attached thereto. For example, a molecule such as an antibody specific for a surface membrane protein on the target cell or a ligand for a receptor on the target cell can be bound to or incorporated within the nucleic acid delivery vehicle. For example, where liposomes are employed to deliver the nucleic acids of the invention, proteins which bind to a surface membrane protein associated with endocytosis may be incorporated into the liposome formulation for targeting and/or to facilitate uptake. Such proteins include capsid proteins or fragments thereof tropic for a particular cell type, antibodies for proteins which undergo internalization in cycling, proteins that target intracellular localization and enhance intracellular half life, and the like. Polymeric delivery systems also have been used successfully to deliver nucleic acids into cells, as is known by those skilled in the art. Such systems even permit oral delivery of nucleic acids.

When administered, the therapeutic compositions of the present invention are administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents, such as chemotherapeutic agents.

The therapeutics of the invention can be administered by any conventional route, including injection or by gradual infusion over time. The administration may, for example, be oral, intravenous, intraperitoneal, intramuscular, intracavity, subcutaneous, or transdermal. When antibodies are used therapeutically, a preferred route of administration is by pulmonary aerosol. Techniques for preparing aerosol delivery systems containing antibodies are well known to those of skill in the art. Generally, such systems should utilize components which will not significantly impair the biological properties of the antibodies, such as the paratope binding capacity (see, for example, Sciarra and Cutie, "Aerosols," in Remington's Pharmaceutical Sciences, 18th edition, 1990, pp 1694-1712; incorporated by reference). Those of skill in the art can readily determine the various parameters and conditions for producing antibody aerosols without resort to undue experimentation. When using antisense preparations of the invention, slow intravenous administration is preferred.

The compositions of the invention are administered in effective amounts. An "effective amount" is that amount of a composition that alone, or together with further doses, produces the desired response. In the case of treating a particular disease, such as cancer, the desired response is inhibiting the progression of the disease. This may involve only slowing the progression of the disease temporarily, although more preferably, it involves halting the progression of the disease permanently. This can be monitored by routine methods or can be monitored according to diagnostic methods of the invention discussed herein.

Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of ASP-1, ASP-2 or I-ASP or nucleic acid encoding ASP-1, ASP-2 or I-ASP for producing the desired response in a unit of weight or volume suitable for administration to a patient. The response can, for example, be measured by determining the signal transduction enhanced or inhibited by the ASP-1, ASP-2 or I-ASP composition via a reporter system as described herein, by measuring downstream effects such as gene expression, or by measuring the physiological effects of the ASP-1, ASP-2 or I-ASP composition, such as regression of a tumor, decrease of disease symptoms, modulation of apoptosis, etc. Likewise, the effects of antisense ASP-1, ASP-2 or I-ASP molecules can be readily determined by measuring expression of the individual genes in cells to which an antisense composition is added. Other assays will be known to one of ordinary skill in the art and can be employed for measuring the level of the response.

The doses of ASP-1, ASP-2 or I-ASP polypeptide or nucleic acid administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

In general, doses of ASP-1, ASP-2 or I-ASP are formulated and administered in doses between 1 ng and 1 mg, and preferably between 10 ng and 100 µg, according to any standard procedure in the art. Where nucleic acids encoding ASP-1, ASP-2 or I-ASP or variants thereof are employed, doses of between 1 ng and 0.1 mg generally will be formulated and administered according to standard procedures. Other protocols for the administration of ASP-1, ASP-2 or I-ASP compositions will be known to one of ordinary skill in the art, in which the dose amount, schedule of injections, sites of injections, mode of administration (e.g., intra-tumoral) and the like vary from the foregoing. Administration of ASP-1, ASP-2 or I-ASP compositions to mammals other than humans, e.g. for testing purposes or veterinary therapeutic purposes, is carried out under substantially the same conditions as described above. A subject, as used herein, is a mammal, preferably a human, and including a non-human primate, cow, horse, pig, sheep, goat, dog, cat or rodent.

When administered, the pharmaceutical preparations of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

ASP-1, ASP-2 or I-ASP compositions may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction which would substantially impair the desired pharmaceutical efficacy.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration conveniently comprise a sterile aqueous or non-aqueous preparation of ASP-1, ASP-2 or I-ASP polypeptides or nucleic acids, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

In another aspect of the invention, ASP-1, ASP-2 or I-ASP polypeptides or nucleic acids are used in the manufacture of a medicament for modulating apoptosis. The medicament can be placed in a vial and be incorporated into a kit to be used for treating a subject. In certain embodiments, other medicaments which modulate the same responses or which favorably affect the ASP-1, ASP-2 or I-ASP compositions can also be included in the same kit, such as chemotherapeutic agents. The kits can include instructions or other printed material on how to administer the ASP-1, ASP-2 or I-ASP compositions and any other components of the kit.

An embodiment of the invention will now be described, by example only, and with reference to the following examples and figures and table;
Figure 1a represents the DNA sequence of ASP-1; Figure 1b represents the DNA sequence of ASP-2; Figure 1c represents the protein sequence of ASP-1; and Figure 1d represents the protein sequence of ASP-2; Figure 1e is a genomic map of ASP-1;
Figure 2a represents a northern blot of ASP-1 mRNA; Figure 2b represents a northern blot of ASP-2 mRNA; Figure 2c represents a northern blot of ASP-1 and ASP-2 mRNA including an actin mRNA loading control;
Figure 3a represents a Coomassie stained SDS polyacrylamide gel of recombinant GST-53BP; Figure 3b represents a western blot showing the specificity of monoclonal antibody DX54.10 for ASP-2; Figure 3c represents a western blot showing the detection of endogenous ASP-2;
Figure 4a represents a western blot showing the interaction of ASP-2 with p53; Figure 4b represents part of the DNA sequence of the ASP-2/bBP2 plasmid; Figure 4c shows the difference in molecular weight between ASP-2 and ASP-2/bBP2;
Figure 5a represents the stimulation of various p53 specific promoters in the presence of combinations of p53, ASP-1 and ASP-2; Figure 5b represents the stimulation of p53 transactivation by ASP-1 and ASP-2;
Figure 6 represents the stimulation of the PIG-3 promoter by ASP-1 and ASP-2 including a western blot showing expression of various truncated ASP polypeptides;
Figure 7a represents the synergistic effect of ASP-1 and ASP-2 on the apoptotic function of p53; Figure 7b shows the synergistic effect of ASP-2 on the apoptotic function of p53; Figure 7c represents the dominant negative effect of the C-terminal half of ASP-2 on the apoptotic function of p53; Figure 7d represents the synergistic effect of ASP-2 on the apoptotic function of p53, p73 and p63;
Figure 8A is a homology comparison of ASP-1, ASP-2 and I-ASP. Saos2 cells were transfected with either vector, p53 (5µg), I-ASP (10µg) or p53+ I-ASP and then incubated for 16hrs. The cells were lysed in NP40 lysis buffer and 1000 µg of lysate subject to an immunoprecipitation performed with polyclonal antibodies to I-ASP bound to Protein G beads. The presence of p53 was detected by western blotting of the immunocomplexes using rabbit polyclonal p53 antibody CM1, Figure 8B. Saos2 cells were transfected with either ASP-1 (8µg) or ASP-2 (4µg), I-ASP (5µg) and p53 (50ng). 40 µl of the corresponding lysates were run on a 10% gel, ASP-1 was detected with V5 antibody, ASP-2 with DX.5410, I-ASP with mouse anti I-ASP antibody, p53 with DO1 and PCNA with anti-PCNA antibody, Figure 8E.; Figure 8C shows the induction of p53 induced apoptosis by ASP-1 and ASP-2 and the inhibition of p53-induced apoptosis by I-ASP; Figure 8D shows the activation of p53 responsive promoter, Bax by ASP-1 and ASP-2 and inhibition of transactivation by I-ASP.

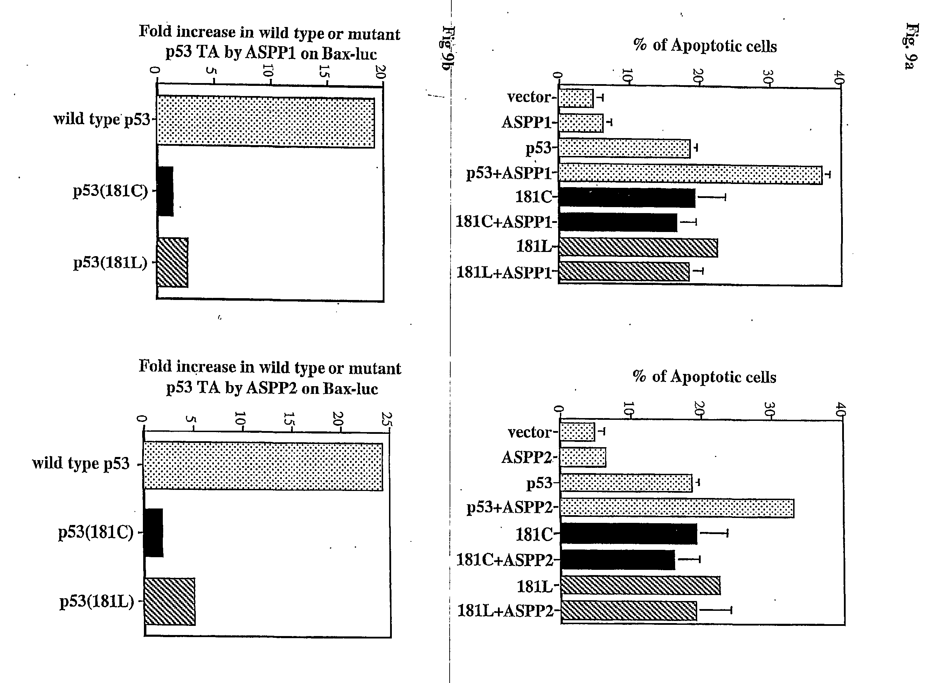

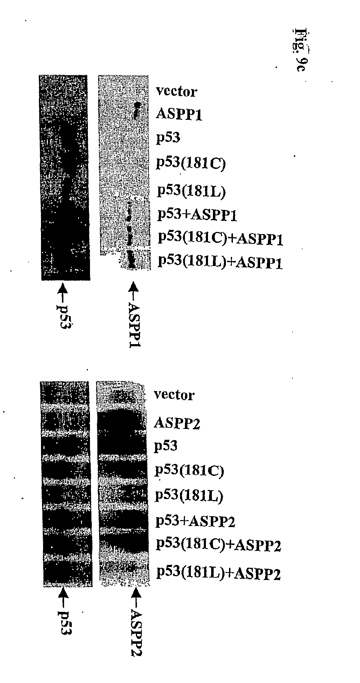

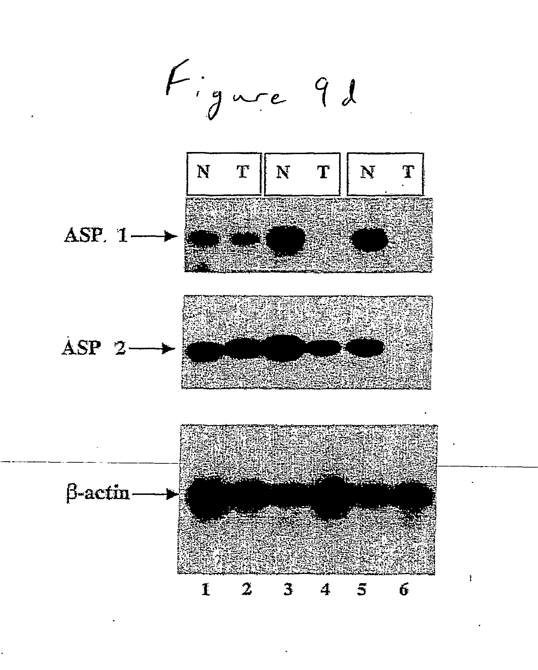

Figure 9A represents the percentage of cells with sub-G1 DNA content (apoptotic cells) in transfected Saos-2 cells expressing p53 (1µg/10 cm dish) or p53181C(1.6µg/10 cm dish) or p53181L (2µg/10 cm dish) in the presence or absence of either ASP-1(10µg/10 cm dish) or ASP-2(10µg/10 cm dish). Figure 9B shows histograms representing the transcriptional activity of either p53 or two of its mutants and the influence of either ASP-1 or ASP-2 (8 and 4 µg, respectively) as indicated, by the ability of p53(50-75 ng)) or p53181C (50ng) or p53181L (50ng) to transactivate the Bax-luc reporter in Saos-2 cells. The fold activation is obtained by the activity of the various p53 constructs in the presence of ASP-1 or ASP-2 over the activity of the promoter in the presence of the various p53 constructs alone. Figure 9C shows western blots using 40µl of the respective transactivation lysates and the proteins detected with anti p53 (DO1), anti ASP-2 (DX.5410), and anti V5 ASP-1.
Figure 10 represents the DNA sequence of I-ASP;
Figure 11 represents the protein sequence of I-ASP;

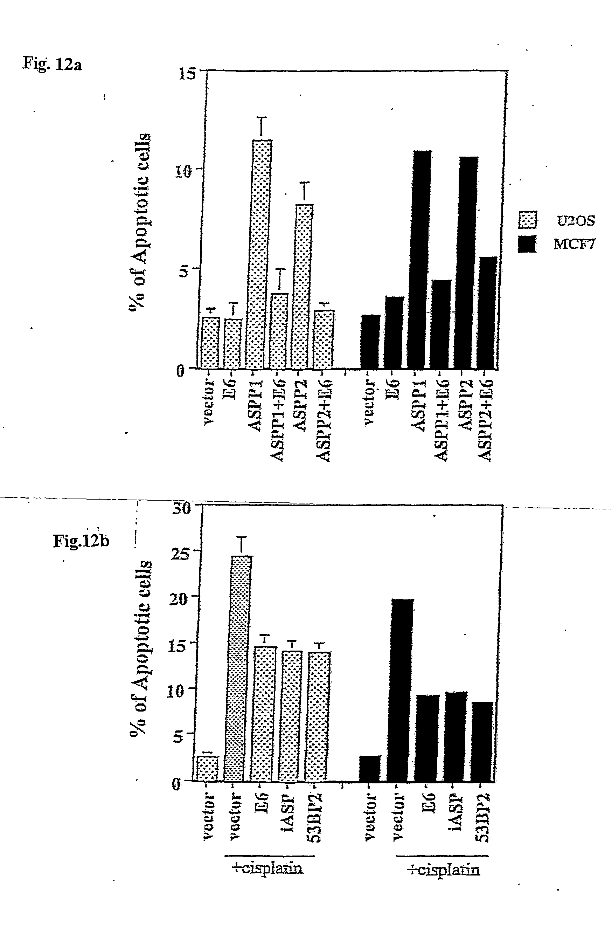

Figure 12 shows that the apoptotic function of p53 is highly regulated by ASP family members *in vivo.* Wild type p53 expressing cell lines U2OS and MCF-7 were transfected with plasmids expressing proteins as indicated together with a cell surface marker CD20 (A-E). The transfected cells were gated and analysed by FACS. The bar graphs represent the percentage of transfected cells with sub-G1 DNA content, characteristic of apoptosis. The plasmids expressing antisense RNA of ASP-1, ASP-2 and I-ASP are labelled as α-ASP-1, α-ASP-2 and α-I-ASP respectively. The viral oncoprotein E6 of human HPV16 is indicated as E6. In figures 12B and 12D, the cells were transfected with the plasmids as indicated. Subsequently, the transfected U20S and MCF-7 cells were incubated with medium containing cisplatin at concentrations of 5 and 3µg/ml respectively. 30 hours later, cells were harvested and analysed as above. For F and G, both U20S and MCF-7 cells were transfected with plasmids expressing proteins as indicated. The co-expression of ASP or p53 and endogenous Bax or mdm2 were visualised after cell fixation, by double immunofluoresence labelling as indicated in figure 12G. For figure 12F, 200 U2OS or MCF-7 cells transfected either with vector alone or ASPP plasmids were examined for the overexpression of endogenous Bax protein. The bar graphs represent the mean value of at least three independent experiments;
Figure 13A illustrates a model describing the interaction of ASP family members with p65 , IkB and p53; figure 13B is a graphical representation of the induction of apoptosis in Saos cells by the expression and co-expression of vector encoded ASP-2, IkB, and p53; figure 13C and 13D is a graphical representation of the ability of IkB affectr the transactivation function of p53 on Bax and mdm2 promoters in the presence and absence of ASP-2;
Figure 14A is a graphical representation of the ability of wild-type p65 and deletion mutant Δp65 to transactivate a NFkB reporter plasmid; Figure 14B is a graphical representation of the induction of apoptosis in cells expressing and co-expressing p53, ASP-2, p65 and Δp65
Figure 15A is a graphical illustration of the ability of Bcl-2 to inhibit the stimulating effect of ASP-1 and ASP-2 on p53H175-L-induced apoptosis; Figure 15B is a graphical illustration of the inability of Bcl-XL to inhibit the stimulating effect of ASP-1 and ASP-2 on p53 H175-L -induced apoptosis; and Figure 15C illustrates the ability of Bcl-2 to inhibit p53-induced apoptosis by ASP-1 and ASP-2;
Figure 16 A illustrates the enhancing effect of I-ASP on the transfoming function of E7; Figure 16B illustrates the enhancing effect of I-ASP on cell resistance to cisplatin; and

Table 1 represents a summary of mRNA expression of ASP-1, ASP-2 and I-ASP in 40 pairs of normal and tumour matched RNA samples derived from Grade I and II breast tumours expressing wild-type p53.

### Example 1

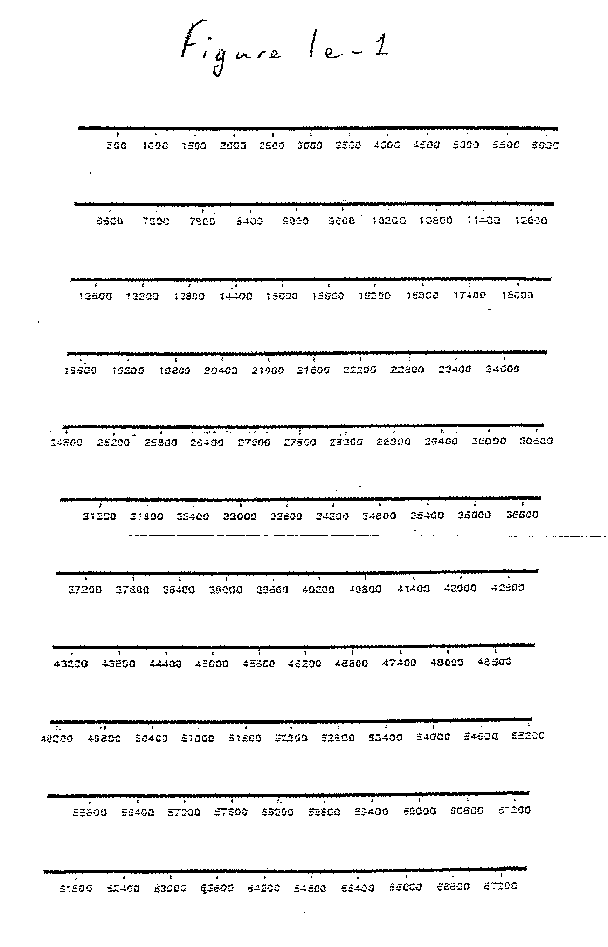

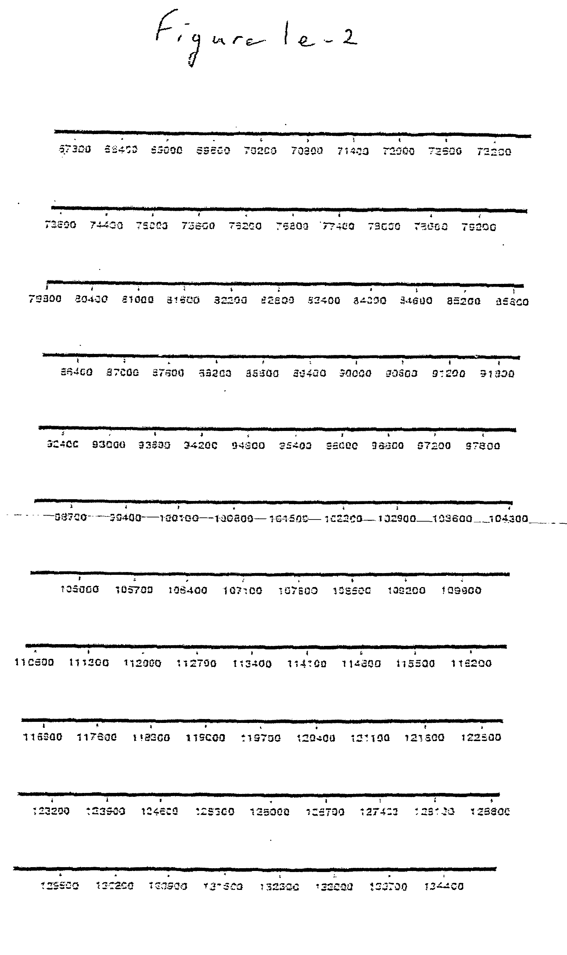

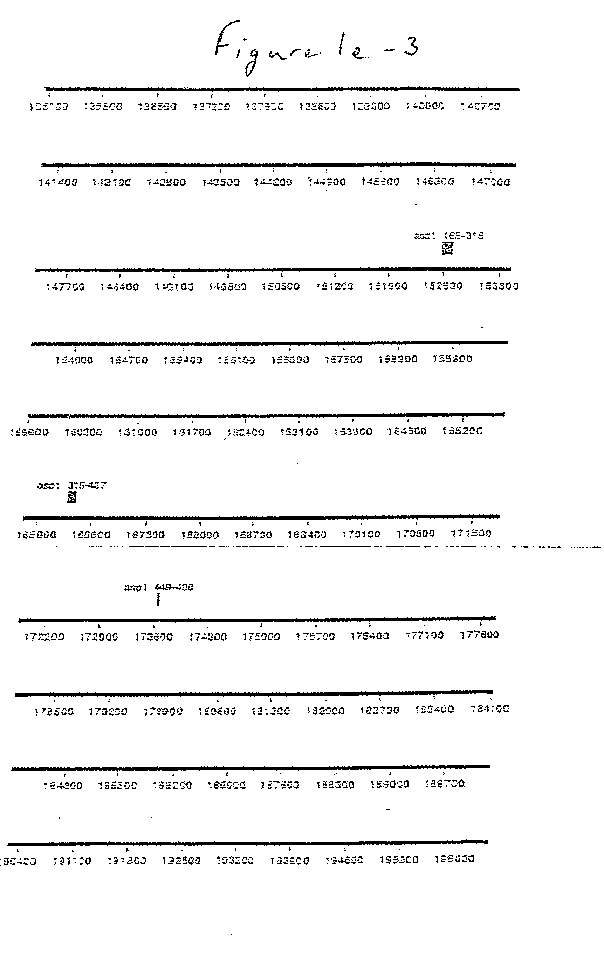

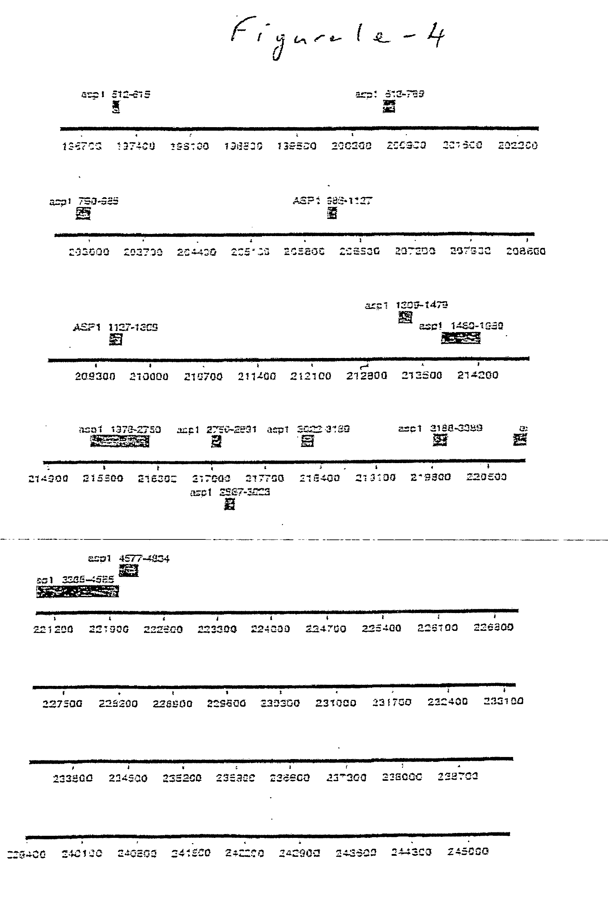

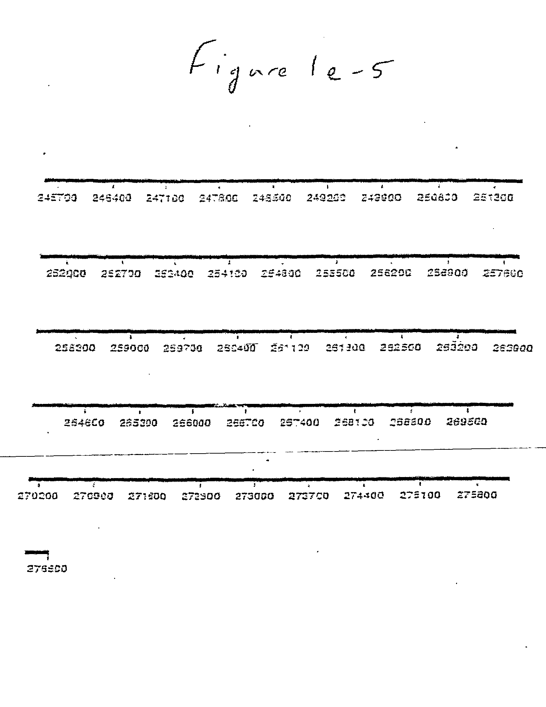

To understand how the ASP family functions *in vivo,* we investigated the tissue distribution of ASP-1 and ASP-2 using northern blot hybridization. As shown in figure 2a and 2b, both ASP-1 and ASP-2 mRNA were expressed in all the human tissues tested with a single transcript at the size of 5.5 to 5kb respectively. However the expression level of ASP-1 and ASP-2 varies. The highest expression levels of ASP-1 and ASP2 were detected in heart, skeletal muscle and kidney. Interestingly, there is a small difference between the expression pattern between ASP-1 and ASP-2. For ASP-1, the highest expression level is in heart, significantly higher than that seen in the kidney and the skeletal muscles. In contrast the expression level of ASP-2 in heart, skeletal muscle and kidney is similar. In addition a relatively high level expression of ASP-1 was also seen in human liver tissues.

### Example 2

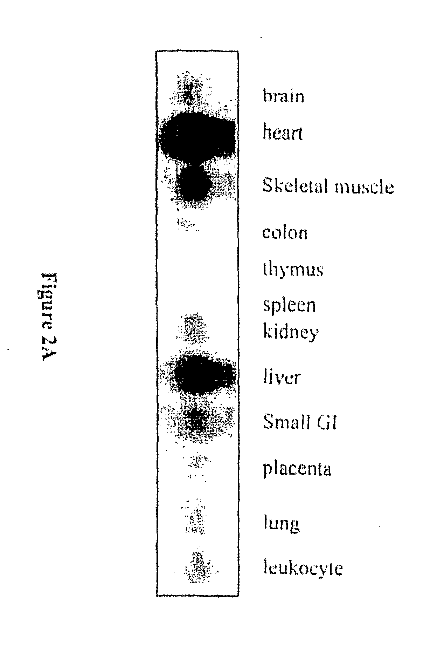

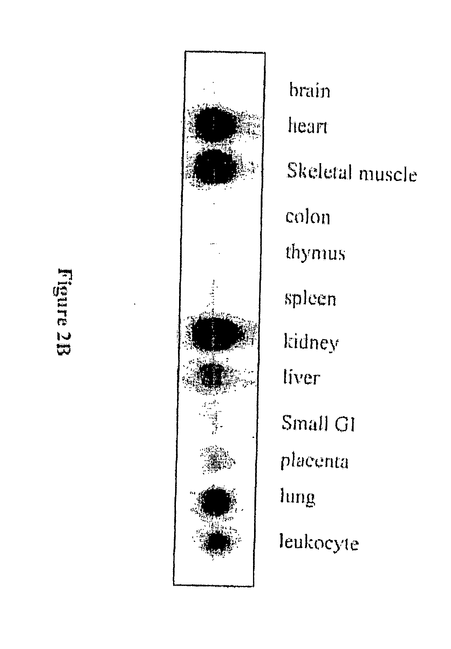

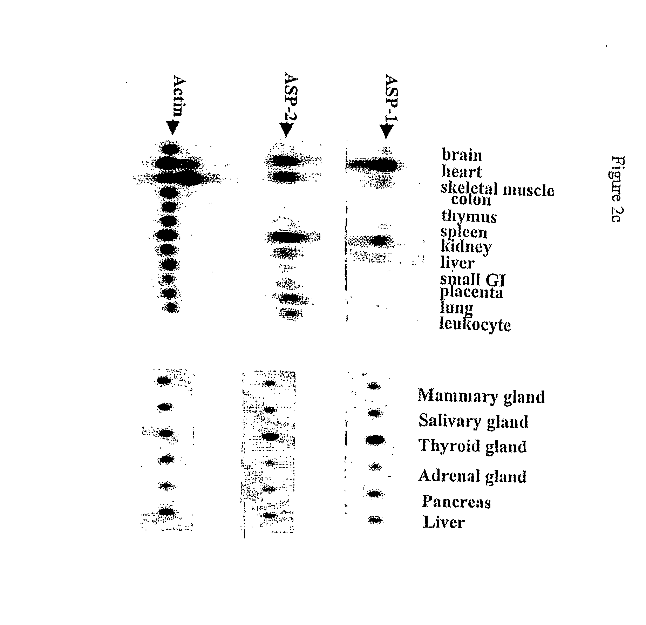

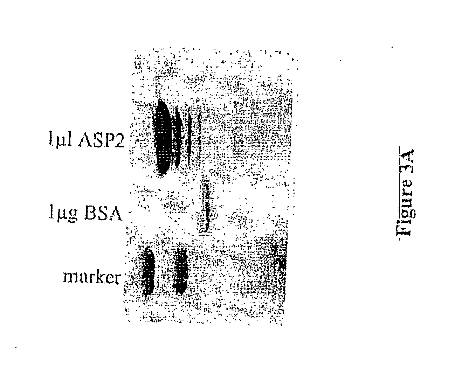

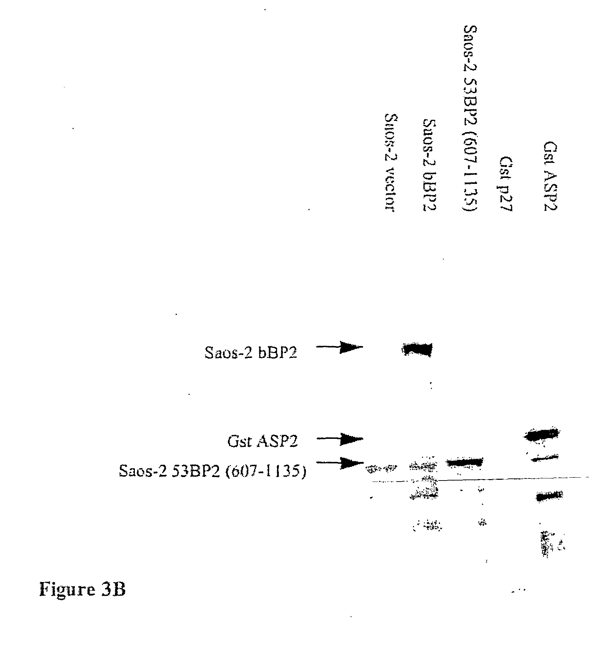

Knowing that there is a specific tissue distribution pattern of ASP family members at their mRNA levels, it was important to investigate how their expression is controlled at the protein level. We used a GST-fusion protein to generate antibodies to ASP-2. The coding region spanning amino acids 698-1135 of ASP-2 was subcloned into the EcoR1 site of the bacterial expression plasmid pGEX 2TK. The 74 KDa GST-ASP-2 (698-1135) protein was produced as shown in figure 3A. The GST-53BP2 protein was used to immunise rabbits (Eurogentec, Belgium) and mice. The immunised serum derived from the rabbits and the mice were tested using the cell lysates of Saos-2 cells transfected with a expression plasmid of ASP-2 fragment, pCMV Bam neo ASP-2/53BP2 (607-1135). The plasmid was constructed by inserting a PCR fragment of ASP-2 containing the epitope tag of 9E1 0 at the BamH1 restriction site. Using the Saos-2 lysate transfected with ASP-2 expression plasmid pCMV Bam neo ASP-2/53BP2 (607-1135) or the control vector, the specificity of the rabbit polyclonal antibody pAbASP-2/77 and the mouse monoclonal antibodies DX54-10 and DX54-7 was confirmed. As shown in figure 3B, the mouse monoclonal antibody DX54.10 did not cross react with GST protein and could recognise transfected ASP-2 expression proteins in Saos-2 cells. DX54.10 only recognised transfected ASP-2 proteins and GST-ASP-2 protein and not GST-p27 fusion protein and is therefore specific to ASP-2.

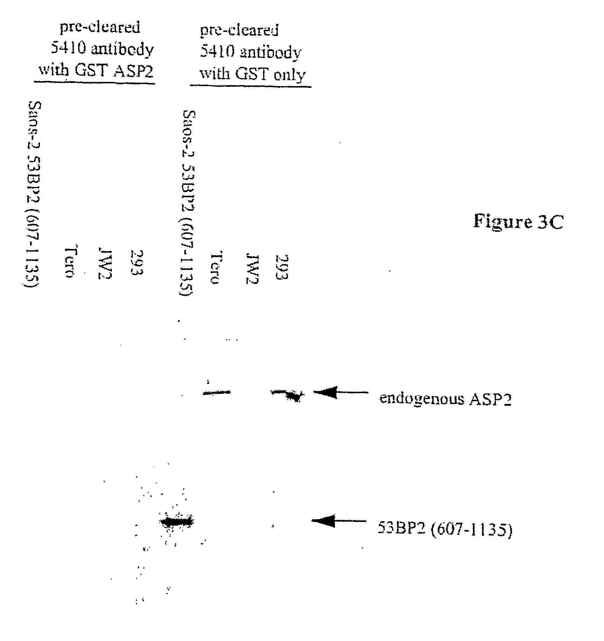

Since the monoclonal antibody is very specific to ASP-2 in the transfected Saos-2 cells, it allowed us to investigate the expression of the endogenous ASP-2 for the first time. To be sure that the reactive band to the antibody is indeed the endogenous ASP-2, the anti-ASP-2 monoclonal antibody DX54.10 supernatant was treated with either GST protein attached to glutathione beads or GST-53BP2 (698-1135) protein attached to glutathione beads. The beads were incubated with the supernatant for one hour on a rotating wheel. After such time the beads were recovered and discarded. Beads were replaced with fresh beads a total of three times. Figure 3C shows that transfected ASP-2/53BP2 fragment (607-1135) and a specific protein band were recognised by the antibody derived from the supernatant incubated with the GST beads but not the ones incubated with GST-ASP-2 beads. These results demonstrated that the recognised protein in the total cell lysates derived from 293 cells and Tero cells were indeed the endogenous ASP-2 and the monoclonal antibody DX54-10 was very specific to this protein.

### Example 3

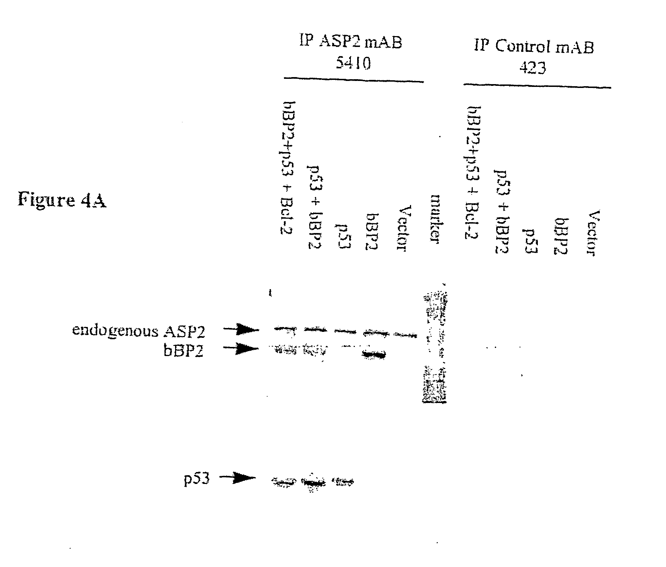

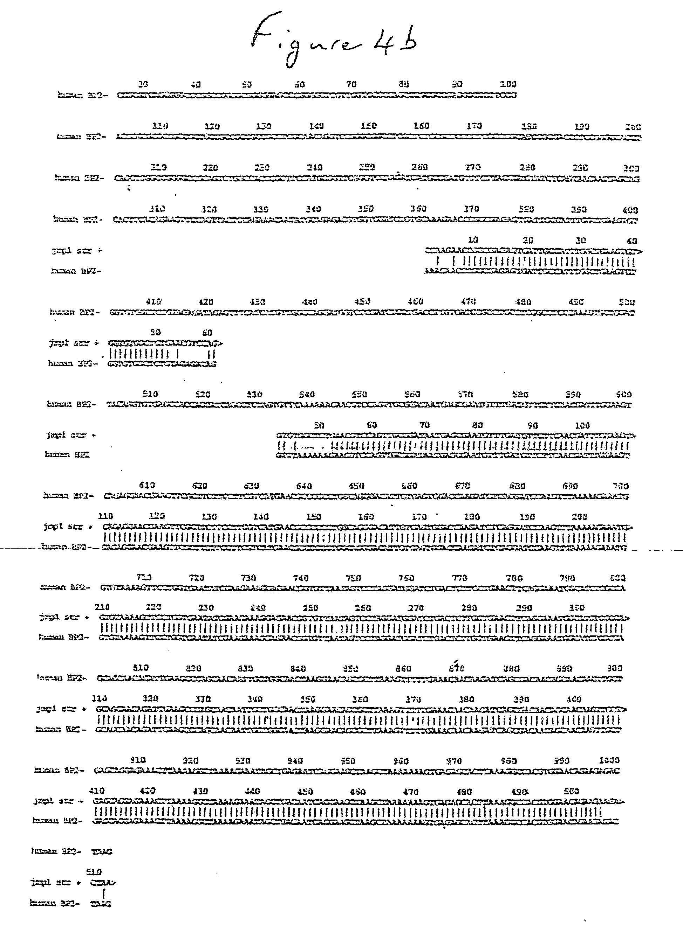

Using the anti-ASP-2 antibodies described above, we initially tested whether the interaction between p53 and ASP-2 occurred *in vivo* using exogenously expressed proteins. Expression plasmids were transfected into Saos-2 cells and an immunoprecipitation was performed using the anti-ASP-2 antibody DX54.10 or a control antibody pAb423 (an antibody to SV40 large T-antigen). Western blot analysis of the immunocomplexes of p53 and ASP-2 showed for the first time that these proteins interact *in vivo* (Figure 4A). This interaction was specific because the control antibody did not pull down either p53 or ASP-2. As shown in figure 4A, there was a discrepancy between the migration of endogenous ASP-2 and the transfected ASP-2 (also known as bBP2(130-1135)) proteins on SDS PAGE. One explanation for this could be found from the original sequence of bBP2 (Naumovski and Cleary, 1996). The nucleotide sequence shows that there are two potential ATG codons in 53BP2/bBP2 cDNA at nucleotide position 571 and 757. The 757 codon was shown to be the preferred start site by *in vitro* coupled transcription-translation. This predicts a protein of 1005 amino acid residues in size. Therefore an expression plasmid of 53BP2/bBP2 was constructed using the nucleotide 757 as start site (Naumovski and Cleary, 1996). However based on the result shown in figure 4a, it is clear that the actual protein translation start site is not 757 codon *in vivo.* Using the 5'end of bBP2 sequence to carry out the BLAST search, we discovered that the sequence of bBP2 at the 412 to 514bp has very high homology to vector sequence (EMBO entry of bBP2/53BP2). We re-sequenced this region of ASP-2/bBP2 plasmid and we have demonstrated that this region of sequence does not exist in the sequence which is shown in figure 4B. Since there was a stop codon within the region of 412-514bp of bBP2 sequence in the data base but this part of the sequence does not exist, it allowed us to predict that the start site of ASP-2 is definitely upstream of 757. By comparing with the part of the mouse ASP-2 we obtained (which we carried out by screening the cDNA library with the human ASP-2 cDNA), we believe that the start site for ASP-2 is at the 246bp of the new ASP-2 cDNA sequence. This would make the ASP-2 protein 1135 amino acids long which would account for the unexpectedly large endogenous protein.

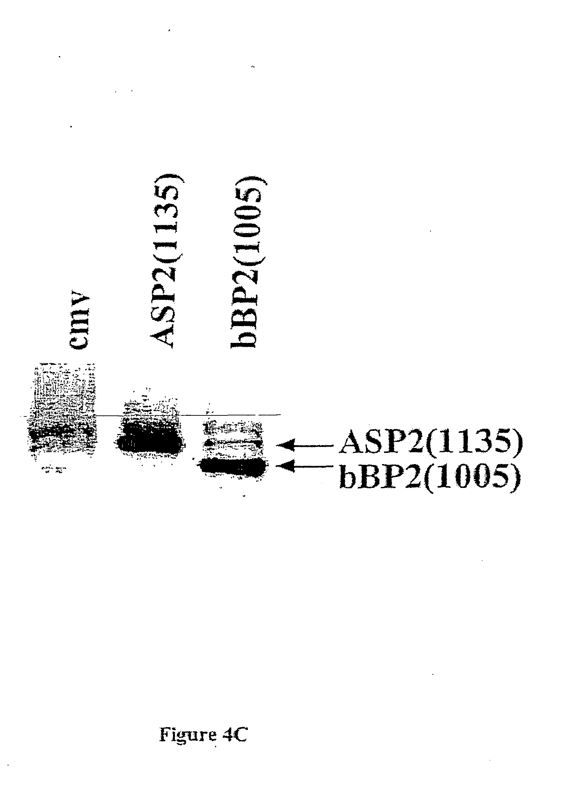

To investigate this further, 53BP2/bBP2 cDNA which contains both ATG start sites (241 and 757) was subcloned into a mammalian expression plasmid pcDNA3. The resulting plasmid, pcDNA3-ASP-2/53BP2(1-1135) was transfected into Saos-2 cells and the expression of both endogenous and exogenous ASP-2 was detected by anti-ASP-2 antibody DX54-10. As shown in figure 4C, the ASP-2 expressed from the pcDNA3-ASP-2(1-1135) migrated to the same molecular weight as that of endogenous ASP-2. From this result, we conclude that the endogenous ASP-2 uses the first ATG and the full length ASP-2 should consist of 1135 amino acids. From the results shown here and below, it became necessary to clarify the clone names which correspond to the actual sequences themselves. We thus used the name of ASP-2 to represent the full length protein which contains 1135 amino acids. We used ASP-2/bBP2 and ASP-2/53BP2 to represent the proteins which contains 130-1135 and 607-1135 amino acids respectively.

In addition to the endogenous ASP-2, the ASP-2/bBP2 could also interact with p53 in vivo (figure 4A).

### Example 4

p53 is a transcription factor which transactivates a growing number of target genes including mdm-2, Bax and cyclin G. ASP-2/53BP2 on the other hand was originally isolated as an inhibitor of p53 because it can inhibit the DNA binding activity of p53 *in vitro* by binding to the central DNA binding region of p53 (Iwabuchi et al., 1993). So, with respect to p53, ASP-2/53BP2 could be the cellular equivalent of the large T-antigen of SV40 DNA tumour virus. One would then predict that ASP-2 should have oncogenic activity. However, the behaviour of ASP-2/bBP2 demonstrated that it confers growth suppression rather than promoting activity (Naumovski and Cleary, 1996). This discrepancy could be due to the fact that the original clone of ASP-2/53BP2 only contains the C-terminal portion of the protein. It is possible that full length ASP-2 protein would have a different effect on p53 from its C-terminal fragment ASP-2/53BP2. We also do not know what effect ASP-1 would have on the activities of p53.

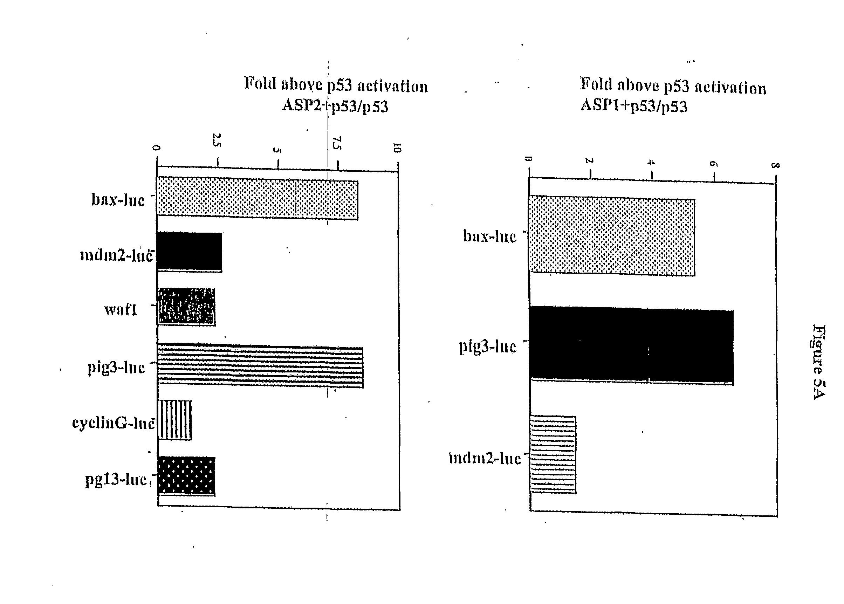

To determine the effect of ASP family members would have on the activities of p53, we first studied p53-dependent transcriptional activity in transient reporter assays. Cells null for p53 were transfected with five p53 reporter plasmids: mdm-2, Bax, cyclin G and p21Waf-1 all derived from the promoters of p53 target genes and PG, a synthetic promoter construct linked to the expression of the luciferase gene. *In vitro* DNA binding assays and the study of mutant p53 transactivation functions have divided some of the known p53 binding sites into two groups (Ludwig et al., 1996). Bax-like sites are usually weak for p53 transcription stimulation while the mdm2-like sites can be stimulated by p53 very effectively. Interestingly, co-expression of ASP-1 or ASP-2 together with p53 resulted in a 10-50 fold stimulation of the Bax promoter. In contrast to the Bax promoter, co-expression of either ASP-1 or ASP-2 with p53 only showed a very modest stimulation of the promoter activity of mdm2 and cyclin G (figure 5A). ASP-2/53BP2 failed to stimulate mdm2 and cyclin G promoters while a slight stimulation on p21wafl and PG synthetic promoters was seen.

The ability of ASP-2/53BP2 to specifically stimulate the promoter activity of Bax but not mdm2 showed for the first time that the promoter specificity of p53 can be regulated in cells. Since Bax is one of the p53 target genes which is pro-apoptotic, we therefore asked whether the ASP family members can specifically stimulate the transactivation of other p53 target genes also known to be involved in promoting apoptosis. One such gene is PIG-3. Using the transient transfection reporter assays shown in figure 5a, we were able to show that both ASP-1 and ASP-2 can specifically stimulate the promoter activity of PIG-3.

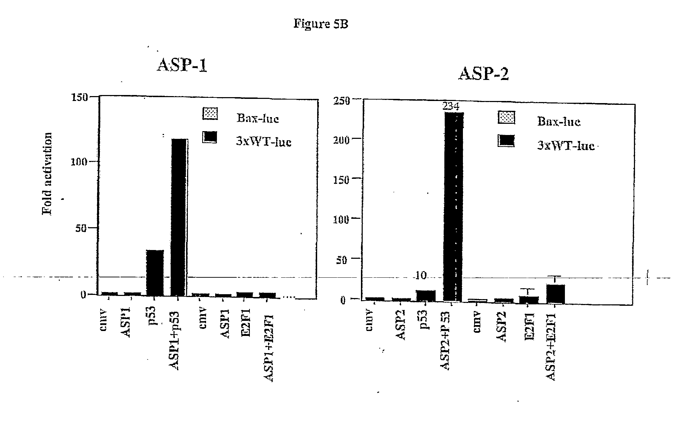

It was shown recently that the transactivation function of p53 can be co-activated by a general transcription co-activator p300/CBP. Thus it was of interest to us to determine whether the ASP family members act like the p300/CBP-like protein which is not specific to p53 and can stimulate a large number of transcription factors. One of the transcription factors we tested was E2F1. Like p53, the transactivation function of E2F1 can be stimulated by the co-expression of p300/CBP. However, the co-expression of ASP-1 or ASP-2 with E2F1 failed to stimulate its transactivation function on a few known reporter promoters, including cyclin A, b-myb and the synthetic promoter 3xwt (figure 5B). This result strongly suggests that ASP-1 and ASP-2 stimulate the transactivation function of p53 specifically. Since the general transcription co-activators p300/CBP can bind to and stimulate the transcriptional activity of both p53 and E2F1, this result also implies that both ASP-1 and ASP-2 can stimulate the transactivation function of p53 independently of p300/CBP.

### Example 5

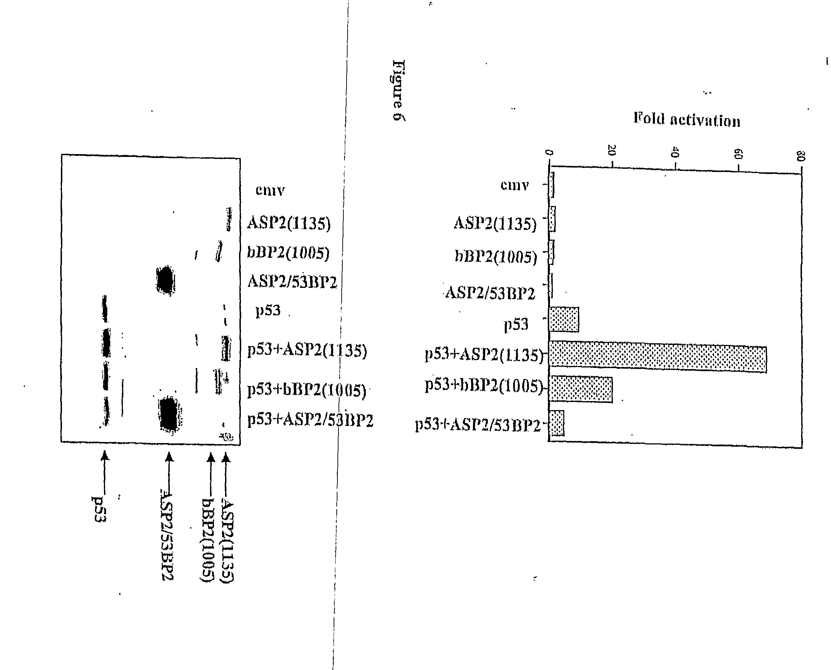

Knowing that the co-expression of the ASP can specifically stimulate the transactivation function of p53, it was important for us to identify the minimal region of ASP required for such activity. We therefore tested the three different versions of ASP-2 for their effects on the transactivation function of p53. As expected, the co-expression of full length ASP-2 (1135aa) was able to further stimulate the transactivation function of p53 about 7 fold. Interestingly under the same conditions, the co-expression of ASP-2/bBP2 (1005aa) only stimulated the transactivation function of p53 about 2-fold. In addition, the co-expression of ASP-2/53BP2(607-1135) reduced the transactivation function by about 50%, (Figure 6). Failure to stimulate the transactivation function of p53 by ASP-2/bBP2 was not due to the lack of expression (figure 6A, lower panel). Thus, ASP-2/bBP2 which lacks only the first 130 amino acids of ASP-2 failed to stimulate the transactivation function of p53 significantly. These data suggested that full-length protein (1-1135aa) was required for ASP-2 to enhance the transactivation function of p53. The reduced transactivation function of p53 by ASP-2/53BP2 suggest that ASP-2/53BP2 can act as a dominant negative mutant to inhibit the action of endogenous ASP-2 on p53.

### Example 6

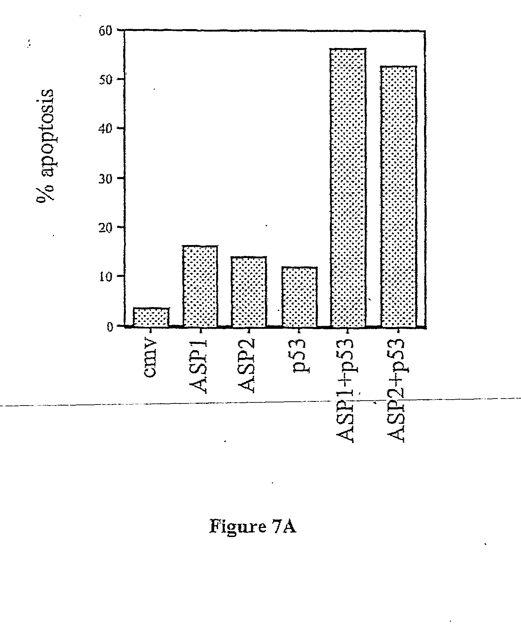

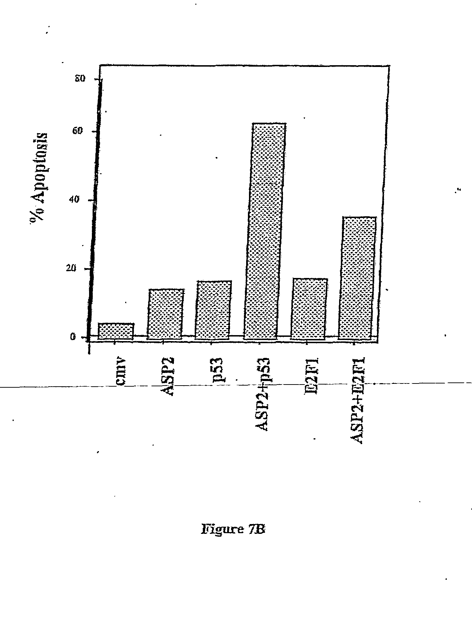

Knowing that ASP-1 and ASP-2 can specifically stimulate the transactivation function of p53 on the promoters of Bax and PIG-3, it was anticipated that when co-expressed ASP-1 or ASP-2 would be able to synergize with p53 to induce apoptosis. It was also hypothesised that if the pro-apoptotic function of ASP-1 and ASP-2 was via their abilities to stimulate the transactivation function of p53, it would have very little effect on the apoptotic function of Bax itself. These hypotheses were tested in Saos-2 cells which are null for p53 and also express a relatively low level of ASP-2. The amount of p53 used in the experiments was determined by titration so that it caused about 17% of transfected cells to undego apoptosis. Apoptosis was identified by the expression of the co-transfected cell surface marker CD20. Interestingly, the expression of ASP-1 or ASP-2 alone resulted in a lower level of apoptosis, consistent with the observation that either ASP-1 or ASP-2 alone could enhance Bax promoter activity slightly, possibly due to the effect of ASP-1 and ASP-2 on p73 and p63. Co-expression of p53 with ASP-1 or ASP-2 however resulted in a significant increase in the number of cells that die of apoptosis. Approximately 50% of the transfected cells now die of apoptosis (Figure 7A). This synergistic effect in enhancing apoptosis was specific to p53 since co-expression of either ASP-1 or ASP-2 with E2F1 resulted in only an additive increase in the percentage of cells that die of apoptosis (figure 7B).

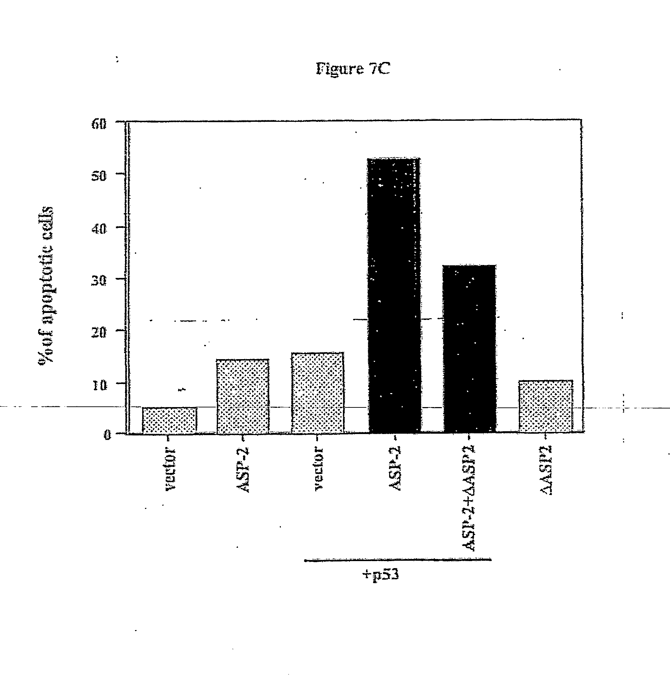

We also used the ASP-2 mutant, ASP-2/53BP2 to test the hypothesis that ASP can stimulate the apoptotic function of p53 by enhancing the transactivation function of p53. ASP-2/53BP2 was shown to inhibit ASP-2 stimulation of p53 transactivation function of p53 of the Bax promoter. Therefore it was anticipated that the apoptotic function of p53 would not be enhanced if both ASP-2 and ASP-2/53BP2 were co-expressed with p53. This indeed proved to be the case. When ASP-2 and p53 were co-expressed 50% of the cells were apoptotic. However when p53, ASP-2 and ASP-2/53BP2 were all co-expresssed, only 30% of cells were apoptotic. Thus, ASP-2 can only enhance the apoptotic function of p53 by increasing its transactivation function (figure 7C).

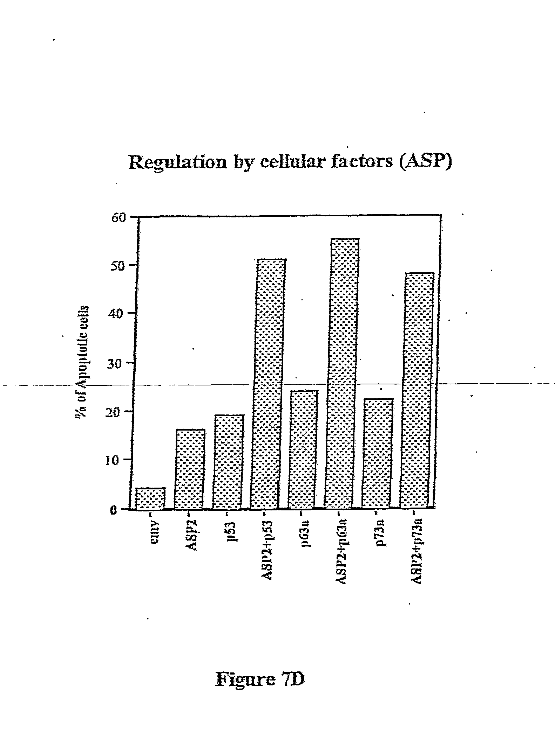

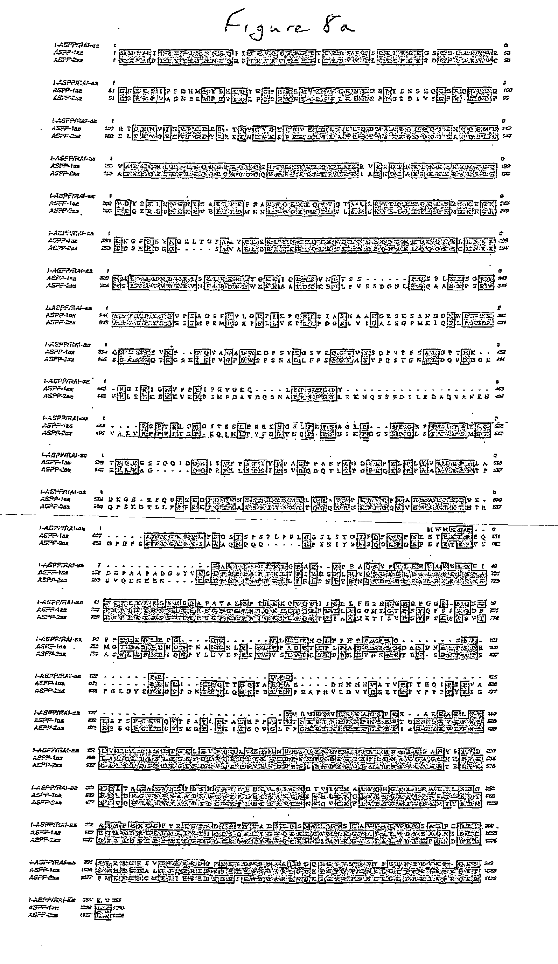

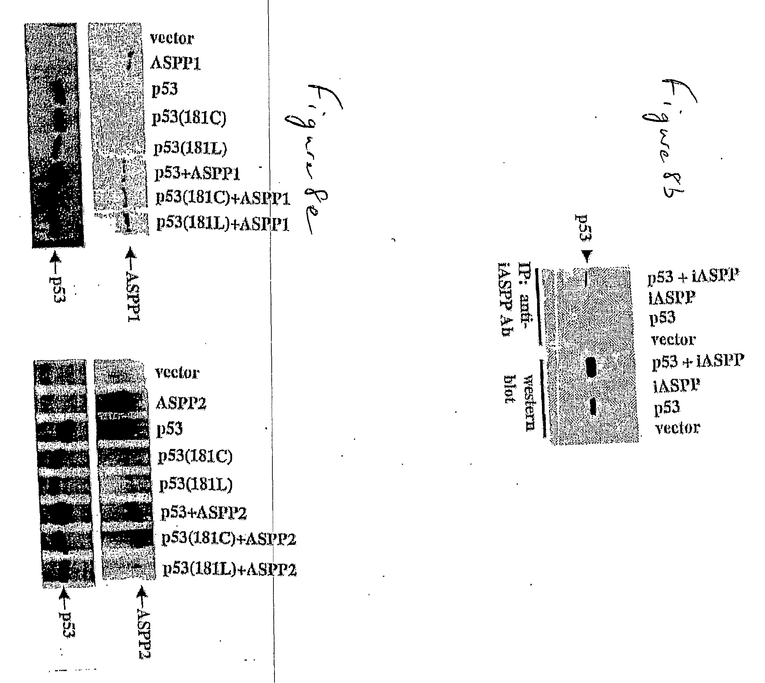

We also studied the effect of ASP on the apoptotic function of p53 family members, p73 and p63. The results are shown in figure 7D. The co-expression of either ASP-1 or ASP-2 enhances the apoptotic function of all the members of p53 family. These results indicate that the ASP family could be a novel tumour supppressor family.

### Example 7

A recently isolated sequence, reL Associated Inhibitor (RAT) was identified as a p65 rel A binding protein which contains 315 amino acids. It has a sequence homology to the C-terminal half of ASP-1 and ASP-2 (figure 8A). It is like the ASP-2 mutant, 53BP2/ASP-2(600-1128) in the following characteristics. RAI does not have the α-helical domain of ASP-1 or ASP-2 but it does contain the proline rich region, the ankryin repeats and the SH3 domain. The p53 contact residues of ASP-2 are also conserved in RAI. To investigate the activity of RAI which we have named I-ASP, we cloned the coding sequence into a mammalian expression vector pcDNA3. We also synthesised a peptide (RLQPALPPEAQSVPELEE) found in I-ASP which does not have sequence similarity to ASP-1 and ASP-2. A mouse antibody specific to this unique I-ASP peptide did not cross react with either ASP-1 or ASP-2 (data not shown). Using this mouse anti-I-ASP specific antibody to immunoprecipitate the transfected I-ASP in Saos-2 cells, we were able to demonstrate that I-ASP can also interact with p53 (figure 8B). Like the ASP-2 mutant, 53BP2/ASP-2 (600-1128), the expression of I-ASP did not induce apoptosis on its own. When I-ASP was co-expressed with p53, it had a small inhibitory effect on the apoptotic function of p53. The most significant effect of I-ASP on the apoptotic function of p53 was observed when ASP-1 or ASP-2 were co-expressed. In agreement with our prediction, the co-expression of I-ASP inhibited the enhanced apoptotic function of p53 effected by ASP-1 and ASP-2 (figure 8C). Similarly, co-expression of I-ASP together with ASP-1 or ASP-2 was able to completely abolish the ability of both ASP-1 and ASP-2 to stimulate the transactivation function of p53 on the Bax promoter (figure 8D). The co-expression of I-ASP did not significantly alter the expression levels of either p53 or ASP (figure 8E). These results indicated that *in vivo* the pro-apoptotic function of ASP-1 and ASP-2 may be regulated by the natural inhibitor I-ASP. Thus the balance between the expression levels of ASP-1, ASP-2 and I-ASP may determine cell fate, ie whether a cell lives or dies.

### Example 8

Recent studies have demonstrated that some apoptotic-defective mutants of p53 can transactivate the promoters of many p53 target genes including mdm2 and p21wafl but not the pro-apoptotic genes such as Bax, PIG-3 and IGF-BP3. Among all the p53 mutants studied, two of them, 181L and 181C were of particular interest. The mutation of p53 at residue 181 has been reported in many human tumour types including breast carcinoma and cervical cancer. From the crystal structures of p53 and 53BP2, the residue 181 of p53 was identified as one of the contact sites within p53 for 53BP2 but this residue was not a contact site for DNA. In agreement with the prediction from the crystal structure results, previous studies have shown that both 181L and 181C can bind to DNA and transactivate many promoters of p53 target genes such as mdm2 and p21wafl. Interestingly, however, both mutants have reduced ability to induce apoptosis or suppress transformation. Since the interaction of ASP and p53 can specifically enhance the apoptotic function of p53, we postulated that the reduced apoptotic function of 181L and 181C may be due to some kind of defect in these two p53 mutants to co-operate with ASP to induce apoptosis. Therefore we examined the effect of ASP on the apoptotic function of the two p53 tumour-derived mutants, 181L and 181C. As shown in figure 9A, the co-expression of ASP-1 or ASP-2 failed to enhance the apoptotic function of either of the p53 mutants, even though within the same experiments, the co-expression of ASP enhanced the apoptotic function of wild type p53 significantly. Subsequently, we also examined the effects of ASP on the transactivation function of the p53 mutants. Consistent with the observation that mutation of residue 181 can impair the ability of ASP to activate the apoptotic function of p53, the co-expression of ASP-1 or ASP-2 was unable to stimulate the transactivation function of the mutant p53, 181C on the Bax gene promoter. The effect of ASP on the p53 mutant 181L was also similar (figure 9B). The inability of ASP to stimulate the activities of p53 mutants, was not due to the lack of protein expression (figure 9C). All these results indicate that the failure of ASP-1 and ASP-2 to stimulate the transactivation function of the two p53 mutants on pro-apoptotic genes may provide the molecular mechanism by which these two mutant p53 molecules were defective in inducing apoptosis. This may also explain why the mutation of this site of p53 was selected for in some of the human tumours. The results shown here also demonstrates the importance of the co-activation function of ASP on the tumour suppression function of p53.

### Example 9

All four of the identified 53BP2 contact residues on p53 were found to be mutated in human tumours. Knowing that the expression of ASP-1 and ASP-2 can increase the apoptotic function of p53 significantly, it was predicted that the expression levels of ASP-1 and ASP-2 would be down-regulated in human tumours expressing wild type p53. Consistent with this, the down-regulation of ASP-2 expression has been found in one case of highly malignant human breast carcinoma in a gene array analysis. To provide further *in vivo* evidence, we used a semi-quantitative RT-PCR technique to study the expression levels of both ASP-1 and ASP-2 in a panel of paired normal and tumour RNA samples derived from 40 breast cancer patients. It is important to note that all 40 of the breast carcinomas express wild type p53. The expression levels of ASP-1 and ASP-2 were frequently down regulated in human breast carcinomas (Table 1 and representative data in figure 9D). Among the 40 carcinoma samples, 24 expressed ASP-1 and 9 expressed ASP-2. Interestingly, it was also noted that 8/9 tumours with reduced expression of ASP-2 also had a reduced expression of ASP-1. This expression pattern suggested that the selective pressure of down regulating the expression of ASP-1 is higher than that of ASP-2. This is consistent with the fact that in the 40 breast carcinomas tested, the frequency of significantly reduced (greater than 75% reduction in the signal) or lack of expression of ASP-1 was higher than that detected for ASP-2, 60% and 22.5% respectively. Since the results were obtained by comparing the expression levels of ASP-1 and ASP-2 between normal tissue and carcinomas derived from the same individuals, it demonstrated that there is a selective advantage for the tumour cells to lose the expression of ASP-1 and ASP-2. These results agree with the above observation that the ASP-binding-impaired-p53 mutants, 181L and 181C, can not induce apoptosis efficiently even in the presence of ASP. This is consistent with the notion that the ASP family of proteins would have a tumour suppressing role in human breast carcinomas.

In contrast to ASP-1 and ASP-2, the expression level of I-ASP was generally low in the normal and human breast tumour tissue samples tested. However, overexpression of I-ASP was detected in 8 of the tumour tissues compared to their normal paired controls (Table 1 and figure 9D). The most striking phenomenon was the correlation of the normal expression of ASP-1 and ASP-2 with the overexpression of I-ASP. 7 of the I-ASP overexpressing tumours did not have any down regulation of ASP-1 and ASP-2 expression (Table 1). This result supports the arguement for the biological importance of I-ASP as the natural inhibitor of ASP-1 and ASP-2 *in vivo.*

### Example 10

To study the roles that endogenous ASP family members play in regulating apoptosis induced by endogenous p53, we introduced ASP-1 or ASP-2 into the cell lines U2OS and MCF7 which express wild-type p53. When expressed in these cells ASP-1 and ASP-2 induced apoptosis (figure 12A). The viral oncoprotein E6, which is derived from human papilloma virus and which can bind and specifically target p53 for degradation, inhibited the apoptosis induced by ASP-1 or ASP-2, demonstrating that ASP-1 and ASP-2 can induce p53-dependent apoptosis.

We also studied the dominant negative function of 53BP2 and I-ASP in inhibiting apoptosis induced by endogenous p53 in response to DNA damage. Before exposure to cisplatin, U2OS and MCF7 cells were transfected with plasmids encoding HPV16 E6, I-ASP, or 53BP2. The percentage of transfected cells dying of apoptosis in response to the treatment with cisplatin was then determined by FACS analysis (figure 12B). Treatment with cisplatin induced over 20% of the transfected cells to die of apoptosis. The expression of E6 reduced the percentage of apoptotic cells to below 15% suggesting that cisplatin induces p53-dependent apoptosis in U2OS cells. In agreement with this, expression of I-ASP or 53BP2 was able to inhibit cisplatin-induced apoptosis to a similar extent as E6. These results suggest that the apoptotic function of endogenous p53 can be regulated by the expression of ASP family members.

To demonstrate further that endogenous ASP family members do play significant roles in regulating the apoptotic function of p53, we used an antisense approach. We cloned fragments from the 5'ends of the ASP-1, ASP-2 and I-ASP cDNAs into a mammalian expression vector in an antisense orientation and tested the ability of the antisense RNA to specifically inhibit the protein synthesis of ASP family members *in vitro* (data not shown). Expression of antisense ASP-1 only inhibited apoptosis induced by ASP-1 but not by ASP-2. Similarly, expression of antisense ASP-2 only inhibited apoptosis induced by ASP-2 but not ASP-1. The specific effect of antisense ASP-1 and ASP-2 was further supported by the observation that co-expression of antisense ASP-1 or ASP-2 did not influence apoptosis mediated by Bax under the same conditions (figure 12C). Hence it allowed us to investigate the role of endogenous ASP-1 and ASP-2 in regulating the apoptotic function of endogenous p53 in response to DNA damage. U2OS and MCF-7 cells were transfected with the various expression plasmids prior to the treatment with cisplatin. FACS analysis showed that around 20-30% of control transfected cells undergo apoptosis. Expression of E6 reduced the percentage of apoptotic cells to half, indicating that cisplatin can induce apoptosis through both p53 dependent and independent pathways in these cells. Expression of antisense RNA of ASP-1 or ASP-2 inhibited cisplatin-induced apoptosis to the same extent as E6 (figure 12D), similar to the effects seen with 53BP2 and I-ASP. This suggests that endogenous ASP-1 and ASP-2 play important roles in regulating the apoptotic function of p53 in response to DNA damage.

We suspect that the stimulatory effect of the endogenous ASP-1 and ASP-2 on p53 induced apoptosis in response to cisplatin may be under-estimated due to high levels of I-ASP detected in these cells (data not shown) which could prevent ASP-1 and ASP-2 from enhancing the apoptotic function of p53. To study the anti-apoptotic role of I-ASP, both U2OS and MCF-7 cells were transfected with antisense I-ASP. Antisense I-ASP induced p53-dependent apoptosis that was abrogated by the co-expression of E6. Removal of the anti-apoptotic function of I-ASP by antisense I-ASP also enhanced the apoptotic function of ASP-1 and ASP-2 (figure 12E). Unlike antisense ASP-1 and ASP-2, the expression of antisense I-ASP did not inhibit cisplatin-induced apoptosis. A small increase in apoptotic cells was consistently detected (figure 12D). Those results demonstrated that ASP-1 and ASP-2 specifically stimulate the apoptotic function of p53 *in vivo.* Endogenous I-ASP functions as an inhibitor of ASP and can block apoptosis induced by endogenous p53.

The antisense nucleic acid molecules were derived from the cDNAs of ASP-1, ASP-2 and I-ASP and were amplified by PCR on the respective plasmid clones using primers spanning the following nucleotide regions (relative to the initial ATG): -74 to 923; -253 to 839 and -37 to 536 for ASP-1, ASP-2 and I-ASP respectively. The amplified segments were purified with the QIAquick PCR purification kit (QIAGEN) and ligated in the pcDNA3.1/V5-His TOPO vector (Invitrogen) according to the manufacturer's instructions. The plasmids containing antisense DNA of ASP-1, ASP-2 and I-ASP were produced by means of the QIAGEN plasmid purification kit according to manufacturers instructions.

### Example 11

It has been well documented that p53 and p65RelA of NF kappaB play very important roles in regulating apoptosis in response to stress. However, very little is known about how these two apoptotic pathways can work together *in vivo.* Attempts had been made towards the understanding of whether there is a cross-talk between p53 and NF-kappaB. The first evidence came from the recent observation that p53 can induce the DNA binding activity of p65 Rel A. Most importantly Ikb, the inhibitor of p65 Rel A, can inhibit the apoptotic function of p53. However it was not clear how p53 can induce the DNA binding activity of p65 and how Ikb can inhibit the apoptotic function of p53.

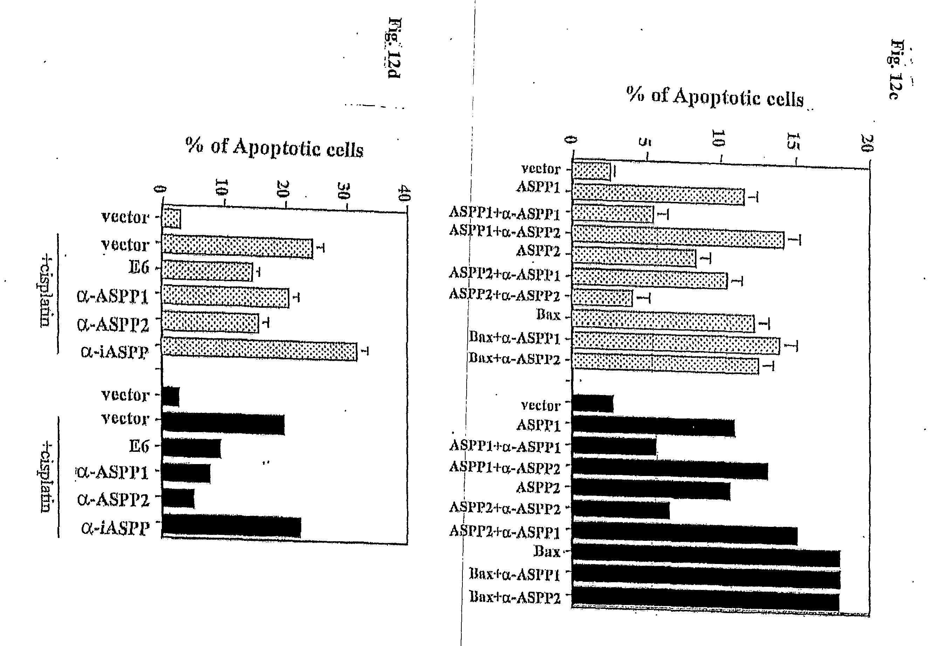

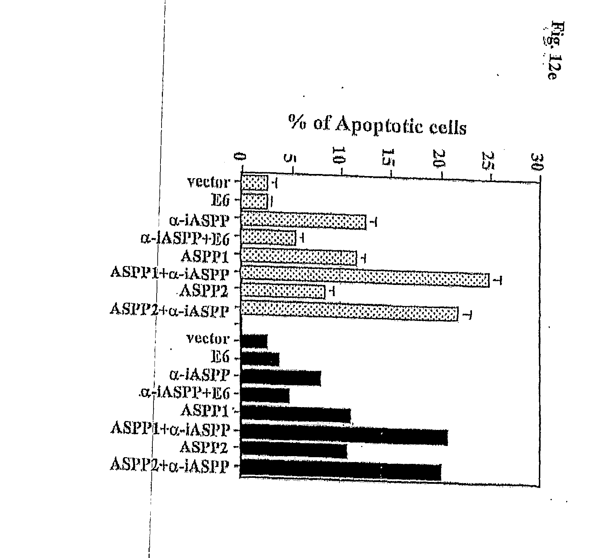

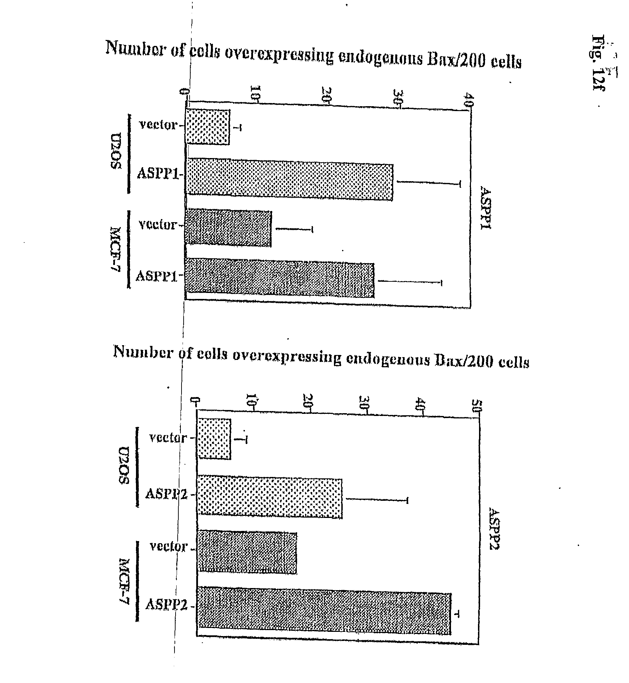

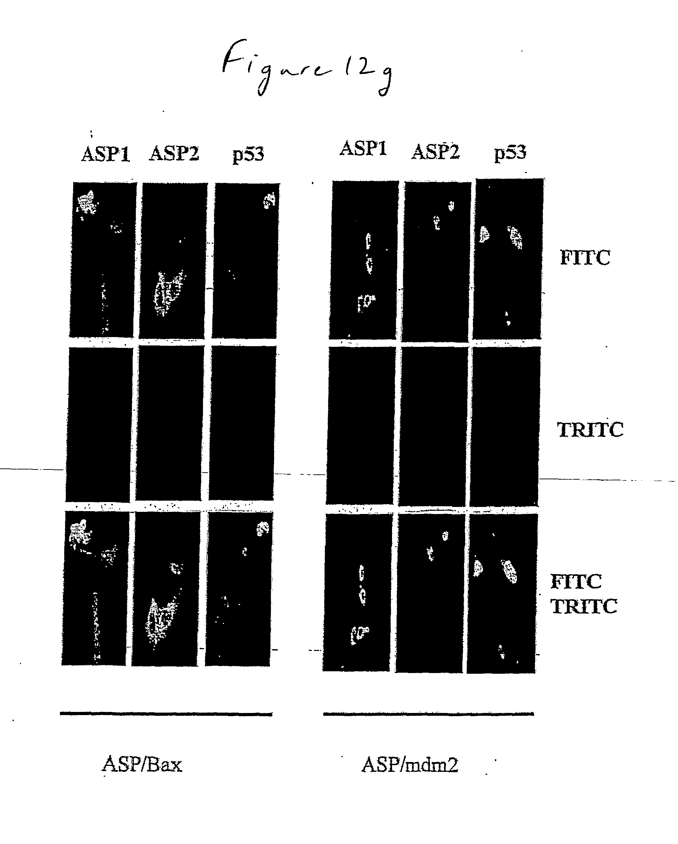

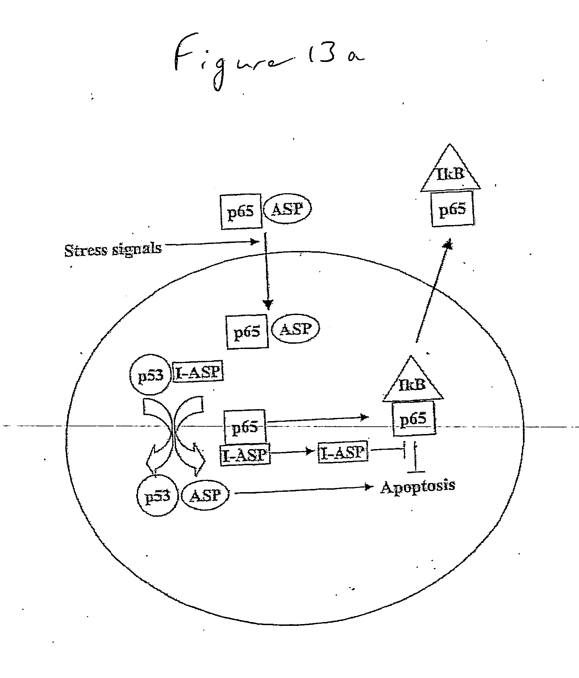

Interestingly, it was discovered very recently that both ASP-2 and I-ASP can interact with p65 rel A, a component of NF-kappaB, in a yeast hybrid assay. I-ASP can also inhibit the transactivation function of p65, although less effectively than Ikb. The region required for ASP-2 and I-ASP to interact with rel A p65 is very similar as that required for p53. Therefore it is possible that there might be some competition between p53 and p65 rel A to interact with ASP-2 and I-ASP. Since ASP family members happen to be the common partner between p53 and p65, we speculated that ASP family members may connect the apoptotic function of p53 and NF-kappaB. In this working model (figure 13A), p53 may induce the DNA binding activity of p65 by interacting with the nuclear I-ASP and allow p65 to bind DNA. In addition, Ikb could inhibit p53-induced apoptosis by binding to p65 and releasing I-ASP. The increased nuclear concentration of I-ASP can then interact with p53 and prevent ASP-2 or ASP-1 to stimulate the transactivation function of p53. If this hypothesis is correct, one would expect that the expression of Ikb should have a profound effect on p53 - induced apoptosis in the presence of ASP-1 or ASP-2.

The results shown in figure 13B are consistent with this notion. In the Ikb-expressing cells, 7.2% of the cells die of apoptosis compared to 4.6% of cells transfected with in vector alone transfected cells. The effect of Ikb on p53-induced apoptosis was also minimal since the percentage of apoptotic cells detected in p53 versus p53+Ikb expressing cells were 12% and 11 % respectively. This could be due to the very low level of ASP-1 and ASP--2 expression in Saos-2 cells. In agreement with the results shown before, the co-expression of ASP-2 produced a significant enhancement of p53 induced apoptosis. The percentage of apoptotic cells in p53+ASP2 transfected cells was 30%. Interestingly, it is under this setting, the co-expression of Ikb showed the most profound effect. The co-expression of Ikb was able to reduce the amount of apoptotic cells induced by p53 and ASP-2 from 30% to 16%. This result suggested that Ikb could inhibit p53-induced apoptosis by preventing ASP-2 to stimulate p53 function. Similar results were also obtained when Ikb was co-expressed with p53 and ASP-1.

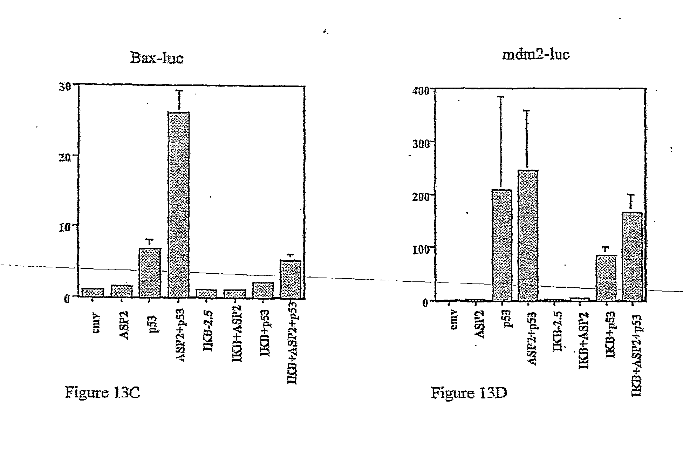

We have shown that ASP-2 can enhance the apoptotic function of p53 by specifically stimulating the transactivation function of p53 on the promoters of pro-apoptotic genes such as Bax. Thus we also investigated the effect of Ikb on the transactivation function of p53 on the Bax and mdm2 promoters in the presence or absence of ASP2, see Figures 13C and D. As shown previously, co-expression of ASP-2 and p53 stimulated the transactivation function of p53 by about 8-fold. Under the same conditions, the expression of Ikb did not show any detectable inhibition on the Bax promoter reporter activity suggesting that Ikb does not inhibit the transcriptional activity of Bax promoter non-specifically in Saos-2 cells. The co-expression of 50ng of Ikb with p53 only showed a very little inhibition on the transactivation function of p53. However, when Ikb, ASP-2 and p53 were co-expressed, Ikb was able to inhibit the ASP-2 mediated stimulation of p53 transactivation function dramatically (figure 13D).

We have shown that ASP-1 and ASP-2 can specifically stimulate the transactivation function of p53 on the Bax promoter but not the mdm2 promoter. Hence, if Ikb was indeed inhibiting the transactivation function of p53 via ASP-2 specifically, it would not be able to show a significant inhibition on the promoter activity of mdm2. Under the same conditions, we also tested the ability of Ikb to inhibit the transactivation function of p53 on the mdm2 promoter activity. As shown in figure 13D, the co-expression of ASP-2 had very little effect on the transactivation function of p53 on the mdm2 promoter. Most importantly, Ikb hardly inhibited the transactivation function of p53 on the mdm2 promoter even in the presence of ASP-2. The results shown here suggest for the first time that Ikb may inhibit the apoptotic function of p53 by preventing ASP-1 or ASP-2 from stimulating the transactivation function of p53.

To further investigate the role of the ASP family in connecting with the p53 and the NFkb pathway, we also studied the effect of the ASP-2 and p65 relA interaction on the apoptotic function of p53. Based on the working model in figure 13A, the p65/ASP interaction may facilitate the nuclear entry of ASP protein, thus allowing the p53/ASP interaction and the release of nuclear I-ASP to bind to the nuclear p65. The residues 176-406 of p65 binds to ASP-2 and I-ASP.

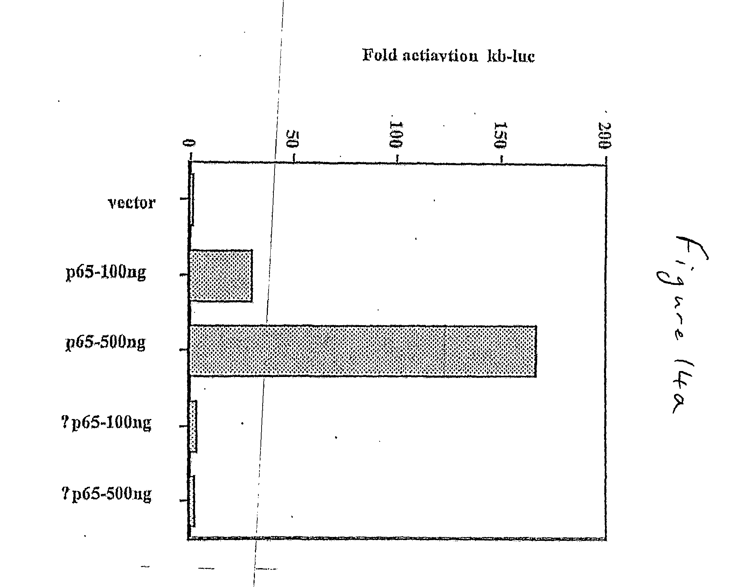

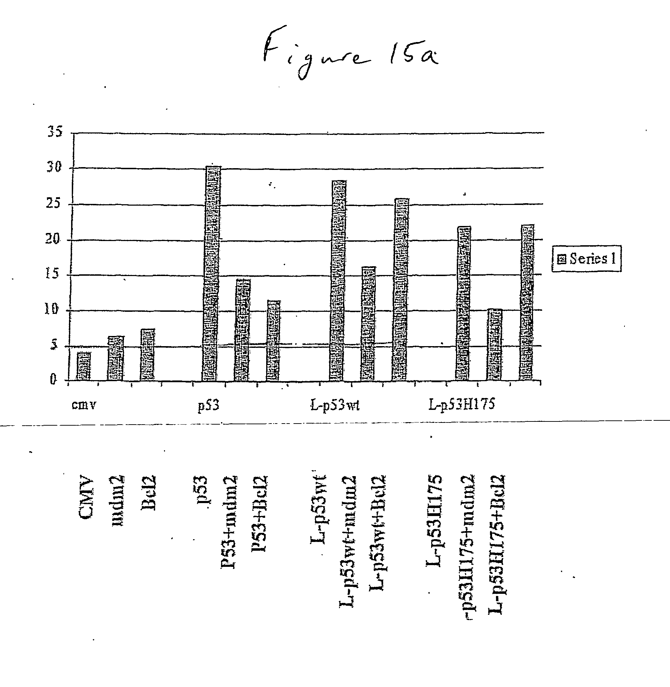

As a transcription factor, p65 can transactivate many target genes. Since p53-induced apoptosis requires p65 and is also correlated with the increased DNA-binding activity of NFkB, it suggested that the DNA-binding activity of p65 may be essential to co-operate with p53 to induce apoptosis. Being able to bind both p53 and p65 places the ASP family of proteins in a central role. One possibility is that ASP binding to p65 may be the mediator for the p53 induced DNA binding activity of p65. However, the co-expression of p53 failed to induce the transcriptional activity of p65 on its reporter. The co-expression of p53 and ASP-2 also failed to show any significant effect on the transactivation function of p65. This result suggested that ASP was not the messenger which delivers the signals from p53 to p65. Nevertheless, ASP could enable p65 to co-operate with p53 to induce apoptosis. We tested whether the action of ASP needed the DNA-binding activity of p65 NFkB. One hundred amino acids of p65 were removed from the N-terminus which contains the DNA binding region of p65. The Δp65 construct thus generated was transcriptionally inactive when tested on the NFkb reporter plasmid. If the ASP-p65 interaction is the mediator of p53 and NFkB pathways, Δp65 would have similar effect on the apoptotic function of p53 as the full-length p65. Otherwise, Δp65 would be inactive since it is defective to transactivate any of its target genes (figure 14A). The result shown in figure 14B showed that not only both p65 and Δp65 stimulated the apoptotic function of p53 in the presence of ASP2, but that Δp65 was even more active than p65 in enhancing the apoptotic function of p53. This may due to the fact that Δp65 is more nuclear than p65. The data obtained from Δp65 argue strongly that p65 can influence the apoptotic function of p53 independent of the DNA-binding activity of p65. Hence, the interaction of ASP-2-p65 could be the mechanism of action.

### Example 12

The anti-apoptotic function of the Bcl-2 oncoprotein has been well established. Interestingly, p53-induced apoptosis can be inhibited by Bcl-2. Furthermore, Bcl-2 can also interact with ASP-2. However, nothing is known about the biological consequences of this interaction. How Bcl-2 inhibits p53-induced apoptosis is also not known. Using the experimental systems mentioned above, we investigated whether Bcl-2 inhibits p53-induced apoptosis by preventing ASP-1 and ASP-2 from stimulating p53.

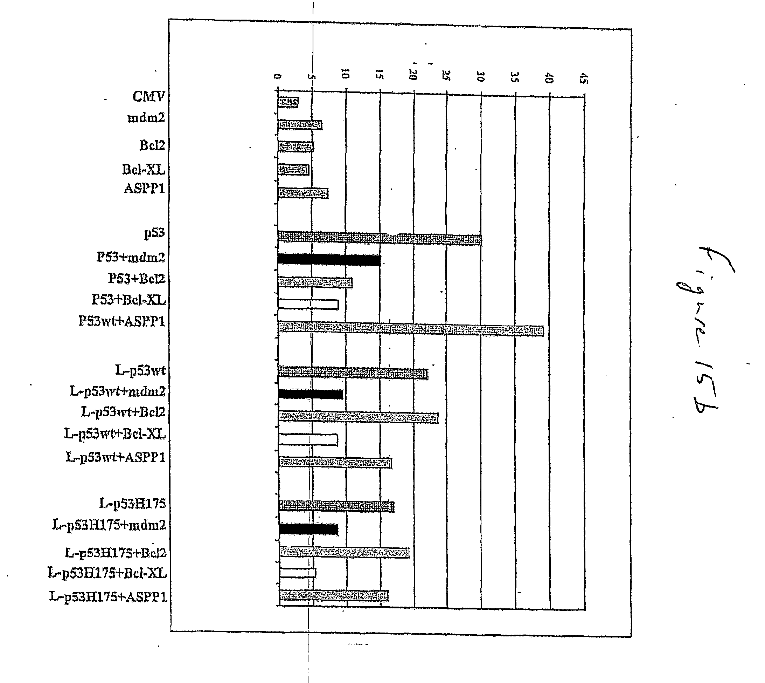

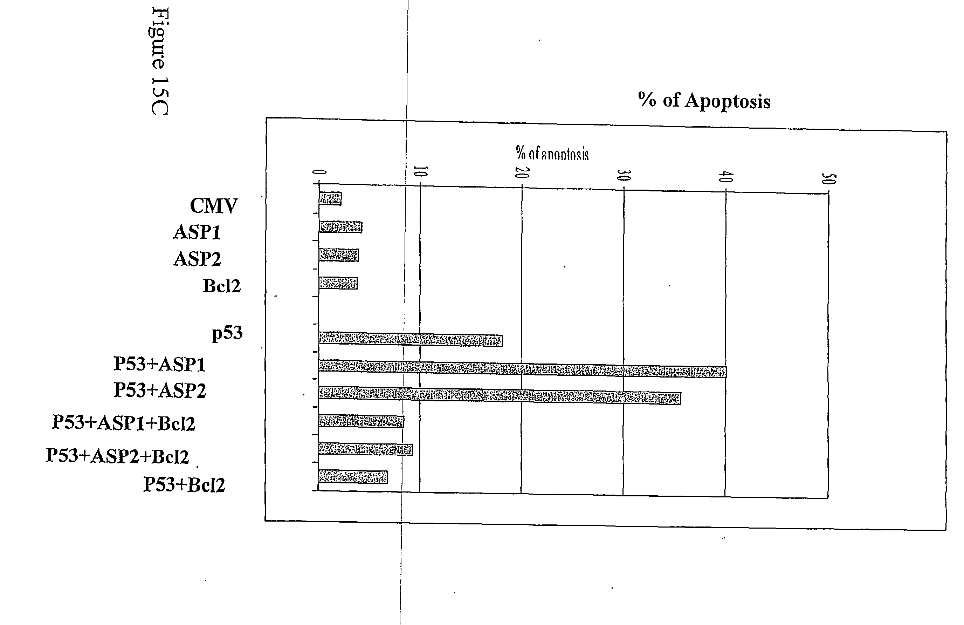

There is increasing evidence to suggest that p53 induces apoptosis through both transcriptional dependent and independent pathways. We have shown that ASP-1 and ASP-2 stimulate the apoptotic function of p53 by specifically enhancing the DNA binding and transactivation function of p53 on promoters of apoptotic genes such as Bax and PIG3. We were also interested in investigating whether ASP can enhance the apoptotic function of p53 independently of its transactivation function. Apoptosis was induced in Saos-2 cells by the expression of wild type p53 or a transcriptioanlly inactive p53, p53H175-L, a mutant p53 which is targeted to mitochondria by a leader sequence and which is known to induce apoptosis independent of the transactivation function of p53. In agreement with previous reports, the apoptotic function of wild type p53 was stimulated by the expression of ASP-1 and ASP-2. However, the co-expression of ASP-1 and ASP-2 failed to enhance the apoptotic function of p53H175-L. Most importantly, only wild type p53-induced apoptosis was inhibited by the co-expression of Bcl-2. Under the same conditions, co-expression of Bcl-2 failed to inhibit apoptosis induced by p53H175-L (figure 15A). Such selective inhibition of p53-induced apoptosis was not seen with Bcl-XL, another inhibitor of apoptosis in the Bcl-2 family (figure 15B). The close association between the ability of ASP to stimulate and the ability of Bcl-2 to inhibit the apoptotic function of p53 suggests that Bcl-2 inhibits p53-induced apoptosis by preventing ASP from stimulating p53. This was confirmed by the data shown in figure 3C that Bcl-2 very effectively prevented ASP-1 and ASP-2 from enhancing the apoptotic function of p53.

### Example 13

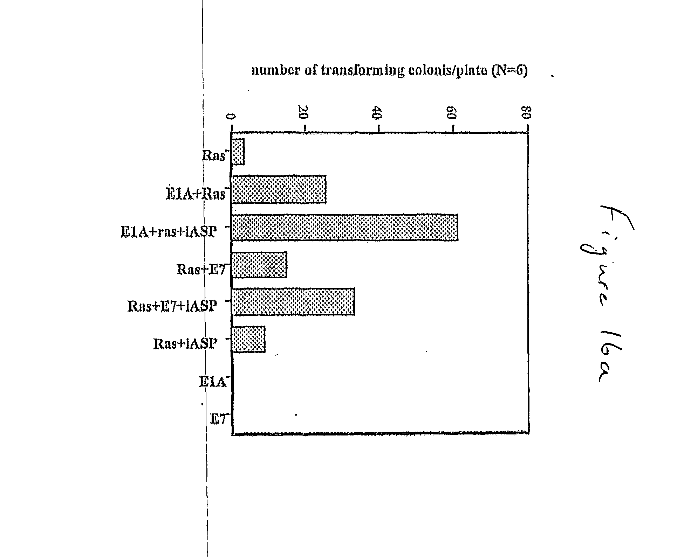

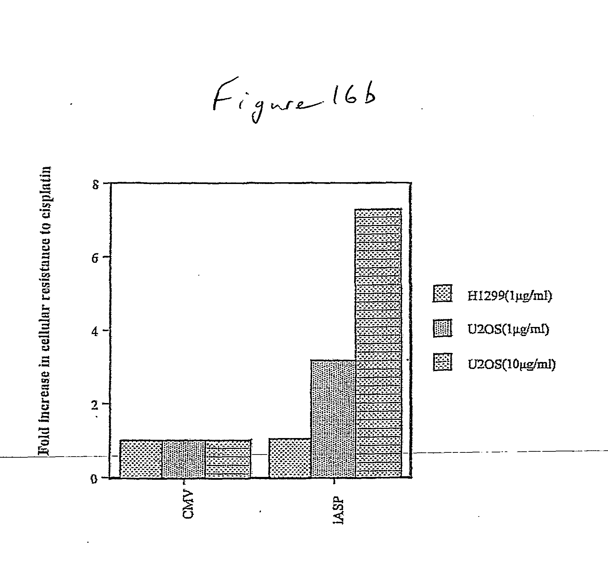

So far we have shown that I-ASP can inhibit p53-induced apoptosis in various cell lines and that its expression level is up-regulated in breast carcinoma cells *in* vivo. All these data suggest that I-ASP could be an oncogene. Since the tumour suppression function of p53 is best linked to its ability to induce apoptosis, it is likely that inhibition of p53-induced apoptosis can remove the tumour suppression function of p53. To test the oncogenic function of I-ASP, rat embryo fibroblasts (REFs) were transfected with plasmids expressing I-ASP and the oncoprotein, E7. The expression of I-ASP enhanced the transforming function of E7 significantly (figure16A). This demonstrated that I-ASP is indeed an oncogene.

As most of the chemotherapy drugs are DNA-damage agents and induce apoptosis via p53-dependent pathways, we reasoned that the ability of I-ASP to inhibit p53-induced apoptosis may make cells more resistant to the cytotoxic effect of chemotherapy drugs such as cisplatin. MCF-7 cells (a human breast cancer cell line) were transfected with an I-ASP-expressing plasmid. The cellular resistance to the cytotoxic effect of cisplatin were compared between I-ASP-expressing and non-expressing I-ASP MCF-7 cells. The results in figure 16B shows that the expression of I-ASP can enhance the cellular resistance by about 2.5 fold. Such an increase in cellular resistance to cisplatin is biologically very significant with respect to cancer treatment. Hence, the high level of expression of I-ASP not only explains why wild type p53 is not functional in some of the human tumour cells. It may also predict the tumour response to treatments. Finally, using I-ASP overexpressing cells may allow the future identification of a more effective chemotherapy drugs.

### References

Iwabuchi, K., Li, B., Bartel, P., and Fields, S. (1993). Use of the two-hybrid system to identify the domain of p53 involved in oligomerization. Oncogene 8, 1693-1696.
Lane, D. P., and Crawford, L. V. (1979). T-antigen is bound to host protein in SV40-transformed cells. Nature 278, 261-263.
Ludwig, R. T., Bates, S., and Vousden, K. H. (1996). Differential activation of target cellular promoters by p53 mutants with impaired apoptotic function. Mol. Cell. Biol. 16, 4952-4960.
Miyashita, T., and Reed, J. C. (1995). Tumour suppressor p53 is a direct transcriptional activator of the human bax gene. Cell 80, p293-299.
Momand, J., Zambetti, G. P., Oslon, D. C., George, D., and Levine, A. J. (1992). The mdm-2 oncogene product forms a complex with p53 protein and inhibits p53-mediated transactivation. Cell 69, 1237-1245.
Naumovski, L., and Cleary, M. L. (1996). The p53-binding protein 53BP2 also interacts with bcl2 and impedes cell cycle progression at G2/M. Mol. Cell. Biol. 16, 3884-3892.
Polyak, K., Xia, Y., Zweier, J. L., Kinzler, K. W., and Vogelstein, B. (1997). A model for p53-induced apoptosis. Nature 389, 300-305.

**Table 1. mRNA expression of ASPP in wild type p53 expressing human breast tumour samples (grade I and II)**

| Tumour | ASP1 | ASP2 | I-ASP |
|---|---|---|---|
| 1 | ↓ | + | - |
| 2 | ↓ | + | - |
| 3 | ↓ | + | - |
| 4 | ↓ | + | - |
| 5 | ↓ | + | - |
| 6 | ↓ | + | - |
| 7 | ↓ | ↓ | - |
| 8 | + | + | ↑ |
| 9 | ↓ | ↓ | - |
| 10 | ↓ | ↓ | - |
| 11 | ↓ | + | - |
| 12 | ↓ | ↓ | - |
| 13 | ↓ | ↓ | - |
| 14 | ↓ | + | - |
| 15 | ↓ | ↓ | - |
| 16 | ↓ | ↓ | - |
| 17 | + | + | ↑ |
| 18 | ↓ | + | - |
| 19 | ↓ | + | - |
| 20 | + | + | ↑ |
| 21 | + | + | - |
| 22 | + | + | - |
| 23 | ↓ | + | - |
| 24 | + | + | - |
| 25 | + | + | ↑ |
| 26 | + | ↓ | - |
| 27 | ↓ | ↓ | - |
| 28 | + | + | ↑ |
| 29 | + | + | - |
| 30 | ↓ | + | - |
| 31 | + | + | - |
| 32 | + | + | ↑ |
| 33 | ↓ | + | - |
| 34 | ↓ | + | - |
| 35 | + | + | - |
| 36 | + | + | - |
| 37 | + | + | - |
| 38 | + | + | ↑ |
| 39 | ↓ | + | - |
| 40 | ↓ | + | ↑ |

## Claims

**1.** An assay to diagnose a disorder **characterized by** down regulation of a polypeptide in an isolated tissue sample, which polypeptide is a stimulator of p53 apoptotic activity, wherein said polypeptide is encoded by a nucleic acid molecule selected from the group consisting of:
i) a DNA molecule consisting of a sequence as represented by SEQ ID NO:1 or 2;
ii) DNA molecules which hybridize under stringent hybridization conditions to SEQ ID NO:1 or 2 and which encode a stimulator of p53 apoptotic activity; and
iii) DNA molecules which are degenerate as a result of the genetic code to DNA sequences defined in (i) and (ii);
wherein said assay comprises the steps of:
a) providing an isolated tissue sample to be tested;
b) providing conditions suitable for the detection of the polypeptide by an agent and detecting the presence of the polypeptide by the agent; and
c) comparing the up-regulation of the polypeptide to matched normal controls.

**2.** An assay according to claim 1 wherein the tissue sample is breast tissue.

**3.** An assay according to claim 1 or 2 wherein the agent is an antibody.

**4.** An assay according to claim 3 wherein the assay is an immunoassay.

**5.** An assay to diagnose a disorder **characterized by** down regulation of a nucleic acid molecule in an isolated breast tissue sample, which nucleic acid encodes a stimulator of p53 apoptotic activity, wherein said molecule is selected from the group consisting of:
i) a nucleic acid molecule consisting of a sequence as represented by SEQ ID NO: 1 or 2;
ii) nucleic acid molecules which hybridize to SEQ ID NO:1 or 2 and which encode a stimulator of p53 apoptotic activity; and
iii) nucleic acid molecules which are degenerate as a result of the genetic code to the sequences defined in (i) and (ii);
wherein said assay comprises the steps of:
a) providing sample to be tested;
b) providing conditions suitable for the detection of said nucleic acid molecule by said agent and detecting the presence of the nucleic acid molecule by the agent; and
c) comparing the down -regulation of the nucleic acid to matched normal controls.

**6.** An assay according to claim 5 wherein the agent is a nucleic acid molecule capable of hybridizing to said nucleic acid sequence.

**7.** An assay according to claim 6 wherein the nucleic acid is at least one oligonucleotide.

**8.** An assay according to claim 6 or 7 wherein the assay is a polymerase chain reaction assay.

**9.** An isolated nucleic acid molecule that encodes a polypeptide which stimulates the transactivation activity of p53 wherein said nucleic acid molecule is selected from the group consisting of:
i) a nucleic acid molecule comprising a DNA sequence as represented in SED NO: 1 or 2; or
ii) a nucleic acid molecule comprising DNA sequences which hybridize under stringent hybridization conditions to the DNA sequence presented in (i) and encode a polypeptide with p53 transactivation activity that is greater than the transactivation activity of bBP2/53BP;
iii) a nucleic acid molecule that encodes an ASP 1 or ASP 2 polypeptide as represented by the amino acid sequences as shown in Figure 8A.

**10.** A nucleic acid molecule according to Claim 9 wherein said p53 transactivation activity of the polypeptide is about 2-fold.

**11.** A nucleic acid molecule according to Claim 9 wherein said p53 transactivation activity of the polypeptide is about 7-fold.

**13.** A nucleic acid molecule according to any of Claims 9-11 wherein said transactivation activity is with respect to p53 responsive pro-apoptotic promoters.

**14.** A nucleic acid molecule according to Claim 13 wherein said pro-apoptotic promoter is derived from the Bax or PIG-3 pro-apoptotic promoter.

**15.** An isolated polypeptide encoded by a nucleic acid molecule according to any of Claims 9-14.

**16.** A vector comprising a nucleic acid molecule according to any of claims 9-14.

**17.** A cell transformed or transfected with a nucleic acid molecule according to any of Claims 9-14, or a vector according to Claim 16.

**18.** An antibody or binding part thereof, wherein said antibody binds a part of a polypeptide according to claim 15 and does not bind the polypeptide bBP2/53BP.

**19.** A polypeptide according to claim 15 for use as a pharmaceutical.

**20.** A vector according to Claim 16 for use as a pharmaceutical.

**21.** A method to screen for agents capable of modulating the pro-apoptotic activity of a polypeptide comprising:
i) providing a cell according to claim 17;
ii) exposing said cell to at least one agent to be tested; and
iii) monitoring the effect of the agent(s) on the activity of the polypeptide.

**22.** A method to screen for agents capable of modulating the proapoptotic activity of a polypeptide comprising:
i) providing a polypeptide according to claim 15;
ii) exposing the polypeptide to at least one agent to be tested; and
iii) monitoring the binding of said agent(s) by said polypeptide.


SEQUENCE LISTING

<110>  Ludwig Institute for Cancer Research

<120>  Suppressor Genes

<130>  P38080WO

<140>  PCT/GB01/03524
<141>  2001-08-06

<150>  0019018.1
<151>  2000-08-04

<160>  7

<170>  PatentIn version 3.1

<210>  1
<211>  4834
<212>  DNA
<213>  Homo sapiens

<400>  1
gagccccgca tcccgccgca gctgccgcct cgccgcggcc gggccggaga gcacggcggc      60

gggagcgcgg ccttaggagg cggccggagc ggtgggcaca gctcggcgcg gagcgtcctg     120

tcaggcggcg gccgagggcg tcgcggactc tccccgcgat gatgccgatg atattaactg     180

ttttcttgag caacaatgaa cagattttaa cagaagttcc tataacaccg gaaacaacct     240

gtcgagatgt tgtagaattt tgcaaggaac ctggagaagg cagctgccat ttagctgaag     300

tgtggagggg aaatgaacgt cccataccct ttgatcatat gatgtacgaa catcttcaga     360

tatggggtcc acggagggaa gaagtgaaat ttttccttcg acacgaggac tccccaactg     420

agaacagtga acaaggtggc cgtcagaccc aagagcaacg aactcagaga aatgtaataa     480

atgtacctgg agataaacgt actgaatatg gggttgggta tccacgtgtt gaacttaccc     540

tctcagagct ccaagatatg gcagctaggc aacagcagca gattgaaaat cagcagcaga     600

tgttggttgc caaggaacag cgtttacatt ttctaaagca acaggagcgc cgtcagcagc     660

agtctatttc tgaaaatgaa aagcttcaga aattgaaaga acgagttgaa gcccaggaga     720

acaagctgaa gaaaattcgt gcaatgagag gacaagtcga ctacagcaaa atcatgaacg     780

gcaatctgtc tgctgaaata gaaaggttca gtgccatgtt ccaggaaaag aagcaggaag     840

tacagactgc aattttaagg gttgatcagc ttagtcagca attggaagat ttaaagaaag     900

gaaaactgaa tgggttccag tcttacaatg gcaaattgac gggaccagcg gcggtggagt     960

taaaaagact gtaccaagaa ctacagattc gtaaccaact taaccaggaa caaaattcaa    1020

aacttcagca gcagaaggaa ctcttaaata agcgcaacat ggaggtggcc atgatggaca    1080

agcgaatcag tgaactgcgt gaacgtctct atgggaaaaa aattcagctg aaccgtgtga    1140

atggcacgtc atcaccacag tcccctctga gcacatcggg cagggtcgct gctgtggggc 1200

cttatatcca ggttcccagt gccggaagct ttcctgtgct gggggaccct ataaagcccc 1260

agtctctcag tattgcctca aatgctgctc atggaagatc caaatccgct aatgatggaa 1320

actggccaac attaaaacag aattctagct cttccgtgaa accagtgcag gtggccggtg 1380

cagactggaa ggatccgagc gtggaggggt ctgtcaagca gggcactgtc tccagccagc 1440

ctgtgccctt ctcagcactg ggacccacgg agaagccggg catcgagatt ggtaaagtgc 1500

cacctcccat cccgggtgta ggcaagcagc tgcctccaag ctatgggaca tacccaagtc 1560

ctacacctct gggtcctggg tcgacaagct ccctggaaag gaggaaggaa ggcagcttgc 1620

ccaggcccag tgcaggcctg ccaagtcgac agaggcccac cctgctgccc gccacaggca 1680

gcacccccca gccaggctcc tcacaacaga ttcagcagag gatttccgta ccgccaagtc 1740

ccacgtaccc gccagcggga ccacctgcat ttccagctgg ggacagcaag cctgaactcc 1800

cactgacagt ggccattagg cctttcctgg ctgataaagg gtcaaggcca cagtctccca 1860

ggaaaggacc ccagacagtg aattcaagtt ccatatactc catgtacctc cagcaagcca 1920

caccacctaa gaattaccag ccggcagcac acagcgcctt aaataagtca gttaaagcag 1980

tgtatggtaa gcccgtttta ccttcgggtt caacctctcc atcgccgctg ccgtttcttc 2040

acgggtcact gtccacgggc acaccacagc ctcagccacc ttcagaaagt actgagaaag 2100

agcctgagca ggatggcccc gccgcccccg cagatggcag caccgtggag agcctgccac 2160

ggccactcag ccccaccaag ctcacgccca tcgtgcattc gccactgcgc taccagagtg 2220

atgcagacct ggaggccctc cgcaggaagc tggccaacgc gccccggccc ctgaaaaagc 2280

gcagctccat cacagagccc gagggccccg gcgggcccaa catccagaag ctgctgtacc 2340

agcgcttcaa caccctggcc ggtggcatgg agggcacccc tttctaccag cccagcccct 2400

cccaggactt catgggcacc ttggccgatg tggacaatgg aaacaccaat gccaatggaa 2460

acctggaaga gctcccccct gcccagccca cagccccact ccccgctgag cctgccccgt 2520

catcagatgc caatgataat gagttacctt cccccgaacc agaggagctc atctgtcccc 2580

aaaccaccca ccaaactgcc gagccggcag aggacaataa caacaacgtg gccacggtcc 2640

ccaccacgga gcagatcccg agtcctgtgg ctgaggcccc atctccaggg gaagagcagg 2700

tccctccagc acctcttccc cctgccagcc accctcctgc cacctccacg aacaagcgga 2760

ccaacttgaa gaagcccaac tcggagcgga cggggcacgg gctgagagtc cggtttaacc 2820

ccctggcact gctcctagac gcgtctctgg aaggagagtt cgatctggtg cagaggatca 2880

tctatgaggt ggaagatccc agcaagccca acgatgaagg gatcacccca ctgcacaacg 2940

ccgtctgcgc cggccaccat cacatcgtga agttcctgct ggattttggt gtcaacgtga 3000

atgctgctga tagtgatgga tggacgccgc tgcactgcgc tgcctcttgt aacagcgttc 3060

acctctgcaa acagctggtg gagagtggtg ccgccatttt tgcctcaacc ataagcgaca 3120

ttgaaactgc tgcagacaag tgtgaggaga tggaggaagg ctacatccag tgctcccagt 3180

ttctatatgg ggtgcaggaa aagctgggtg tgatgaacaa aggtgtggcg tatgctctgt 3240

gggactacga ggcccagaac agtgacgagc tgtccttcca cgaaggggac gccctcacca 3300

tcctgaggcg caaggacgaa agcgagactg agtggtggtg ggctcgcctt ggagaccggg 3360

agggctatgt gcccaaaaac ctgctggggc tgtatccacg gatcaaaccc cgacagcgaa 3420

cactcgcctg aacttccttt tggagcaccg catggtcttg ccagctacca ggagccactt 3480

aagagattat tgtgctgttt tccaggaaag ctgcagctag aaaatggtct taatggtgct 3540

cactttagca gacagcgtcc acaatgtgaa tcctacagtt tccaggtgag gccctttctc 3600

cagtttgccc attaactggg agaggtactt tcgcctccaa ggactgaatt ttgccaatta 3660

ctataaatcc aaataaatac ccactttcaa aacacccacc cctcttgcca ttaagaagtc 3720

ccataacccc cggttggttg ccagtgaaga cagaagctct tactgacttg gccccgaggc 3780

catcaccccc tccagcagtg aacactgtcc gccgctgtga ggcctgctcc cctgcgaccg 3840

ccctgccccc cgtcaccgaa tcggacactc atcctttctc acacttccca cacatgatcc 3900

ttcttccctt catcaccaaa ggagcctctg tatggaaaca tgtccagtgt tgctgcccag 3960

tgtgtatgcc tcccagtacc cactctgctc ggccgccttg ggggttccgc ttcctgttcc 4020

agttcaccta aaggctgatt gtgcaggccc agcactgtgg ctggactgcc gcgccacggg 4080

caccaggacc cctaagacca agtgacaact gggagagcct cagcatatac tcttctcctc 4140

cgatctcaca gcctgtcatg ctgctcagtg tggttctcac ccctgcaagc tcaaattcag 4200

ttccctgaat ggagtcaggt gctggaggcc gtggcagcgg agggtggttg gggttggggc 4260

tgggggtgga ctggtgtgag ggcagaccag ggccaggtag acggggctgt ttggtgcctg 4320

aaggatggca gacgcctggt gtcaggaggg gccgccacca aggagcagca gctggggcag 4380

aggagctggg gtcaggggcc acccctctct gccgatctcc ctgcctgggc tggctgtgag 4440

gccacctttg tcccaggccc agcctcaagg caaggagggc gcttcactga ggtgtgaatt 4500

gtacgtacag gctttttata taccaaaagt attttttgac tagaccattc aaagctaccc 4560

gaactatgtt ggaaattttt ttttttctca ttaaaataca ggcccttagg ctctattttt 4620

catgtatgag tcgtgtgtaa tttatgtaaa aatgtgtgta cagactcact gatgcagcac 4680

tgtagcccat caccttggag cactgactgt acatagtgtg gtgaagaaaa gtgaacgccc 4740

ttgtagagca gcccgaccac aggagcatgg ccgctgccag cccagacgct gctgacgctg 4800

tgtaaatgtg cacaataaac ccgtctcacc ccgg 4834

<210> 2
<211> 4402
<212> DNA
<213> Homo sapiens

<400> 2
gtcacgagcg tcgaagagac aaagccgcgt cagggggccc ggccggggcg ggggagcccg 60

gggcttgttg gtgccccagc ccgcgcggag ggcccttcgg acccgcgcgc cgccgctgcc 120

gccgccgccg cctcgcaaca ggtccgggcg gcctcgctct ccgctcccct cccccgcatc 180

cgcgaccctc cggggcacct cagctcggcc ggggccgcag tctggccacc cgcttccatg 240

cggttcgggt ccaagatgat gccgatgttt cttaccgtgt atctcagtaa caatgagcag 300

cacttcacag aagttccagt tactccagaa acaatatgca gagacgtggt ggatctgtgc 360

aaagaacccg gcgagagtga ttgccatttg gctgaagtgt ggtgtggctc tgaacgtcca 420

gttgcggata atgagcgaat gtttgatgtt cttcaacgat ttggaagtca gaggaacgaa 480

gttcgcttct tccttcgtca tgaacgcccc cctggcaggg acattgtgag tggaccaaga 540

tctcaggatc caagtttaaa aagaaatggt gtaaaagttc ctggtgaata tcgaagaaag 600

gagaacggtg ttaatagtcc taggatggat ctgactcttg ctgaacttca ggaaatggca 660

tctcgccagc agcaacagat tgaagcccag caacaattgc tggcaactaa ggaacagcgc 720

ttaaagtttt tgaaacaaca agatcagcga caacagcaac aagttgctga gcaggagaaa 780

cttaaaaggc taaaagaaat agctgagaat caggaagcta agctaaaaaa agtgagagca 840

cttaaaggcc acgtggaaca gaagagacta agcaatggga aacttgtgga ggaaattgaa 900

cagatgaata atttgttcca gcaaaaacag agggagctcg tcctggctgt gtcaaaagta 960

gaagaactga ccaggcagct agagatgctc aagaacggca ggatcgacag ccaccatgac 1020

aatcagtctg cagtggctga gcttgatcgc ctctataagg agctgcagct aagaaacaaa 1080

ttgaatcaag agcagaatgc caagctacaa caacagaggg agtgtttgaa taagcgtaat 1140

tcagaagtgg cagtcatgga taagcgtgtt aatgagctga gggaccggct gtggaagaag 1200

aaggcagctc tacagcaaaa agaaaatcta ccagtttcat ctgatggaaa tcttccccag 1260

caagccgcgt cagccccaag ccgtgtggct gcagtaggtc cctatatcca gtcatctact 1320

atgcctcgga tgccctcaag gcctgaattg ctggtgaagc cagccctgcc ggatggttcc 1380

ttggtcattc aggcttcaga ggggccgatg aaaatacaga cactgcccaa catgagatct 1440

ggggctgctt cacaaactaa aggctctaaa atccatccag ttggccctga ttggagtcct 1500

tcaaatgcag atcttttccc aagccaaggc tctgcttctg tacctcaaag cactgggaat 1560

gctctggatc aagttgatga tggagaggtt ccgctgaggg agaaagagaa gaaagtgcgt 1620

ccgttctcaa tgtttgatgc agtagaccag tccaatgccc caccttcctt tggtactctg 1680

aggaagaacc agagcagtga agatatcttg cgggatgctc aggttgcaaa taaaaatgtg 1740

gctaaagtac cacctcctgt tcctacaaaa ccaaaacaga ttaatttgcc ttattttgga 1800

caaactaatc agccaccttc agacattaag ccagacggaa gttctcagca gttgtcaaca 1860

gttgttccgt ccatgggaac taaaccaaaa ccagcagggc agcagccgag agtgctgcta 1920

tctcccagca taccttcggt tggccaagac cagacccttt ctccaggttc taagcaagaa 1980

agtccacctg ctgctgccgt ccggcccttt actccccagc cttccaaaga caccttactt 2040

ccacccttca gaaaacccca gaccgtggca gcaagttcaa tatattccat gtatacgcaa 2100

cagcaggcgc caggaaaaaa cttccagcag gctgtgcaga gcgcgttgac caagactcat 2160

accagagggc cacacttttc aagtgtatat ggtaagcctg taattgctgc tgcccagaat 2220

caacagcagc acccagagaa catttattcc aatagccagg gcaagcctgg cagtccagaa 2280

cctgaaacag agcctgtttc ttcagttcag gagaaccatg aaaacgaaag aattcctcgg 2340

ccactcagcc caactaaatt actgcctttc ttatctaatc cttaccgaaa ccagagtgat 2400

gctgacctag aagccttacg aaagaaactg tctaacgcac caaggcctct aaagaaacgt 2460

agttctatta cagagccaga gggtcctaat gggccaaata ttcagaagct tttatatcag 2520

aggaccacca tagcggccat ggagaccatc tctgtcccat catacccatc caagtcagct 2580

tctgtgactg ccagctcaga aagcccagta gaaatccaga atccatattt acatgtggag 2640

cccgaaaagg aggtggtctc tctggttcct gaatcattgt ccccagagga tgtggggaat 2700

gccagtacag agaacagtga catgccagct ccttctccag gccttgatta tgagcctgag 2760

ggagtcccag acaacagccc aaatctccag aataacccag aagaaccaaa tccagaggct 2820

ccacatgtgc ttgatgtgta cctggaggag taccctccat acccaccccc accataccca 2880

tctggggagc ctgaagggcc cggagaagac tcggtgagca tgcgcccgcc tgaaatcacc 2940

gggcaggtct ctctgcctcc tggtaaaagg acaaacttgc gtaaaactgg ctcagagcgt 3000

atcgctcatg gaatgagggt gaaattcaac ccccttgctt tactgctaga ttcgtctttg 3060

gagggagaat ttgaccttgt acagagaatt atttatgagg ttgatgaccc aagcctgccc 3120

aatgatgaag gcatcacggc tcttcacaat gctgtgtgtg caggccacac agaaatcgtt 3180

aagttcctgg tacagtttgg tgtaaatgta aatgctgctg atagtgatgg atggactcca 3240

ttacattgtg ctgcctcatg taacaacgtc caagtgtgta agtttttggt ggagtcagga 3300

gccgctgtgt ttgccatgac ctacagtgac atgcagactg ctgcagataa gtgcgaggaa 3360

atggaggaag gctacactca gtgctcccaa tttctttatg gagttcagga gaagatgggc 3420

ataatgaata aaggagtcat ttatgcgctt tgggattatg aacctcagaa tgatgatgag 3480

ctgcccatga aagaaggaga ctgcatgaca atcatccaca gggaagacga agatgaaatc 3540

gaatggtggt gggcgcgcct taatgataag gagggatatg ttccacgtaa cttgctggga 3600

ctgtacccaa gaattaaacc aagacaaagg agcttggcct gaaacttcca cacagaattt 3660

tagtcaatga agaattaatc tctgttaaga agaagtaata cgattatttt tggcaaaaat 3720

ttcacaagac ttattttaat gacaatgtag cttgaaagcg atgaagaatg tctctagaag 3780

agaatgaagg attgaagaat tcaccattag aggacattta gcgtgatgaa ataaagcatc 3840

tacgtcagca ggccatactg tgttggggca aaggtgtccc gtgtagcact cagataagta 3900

tacagcgaca atcctgtttt ctacaagaat cctgtctagt aaataggatc atttattggg 3960

cagttgggaa atcagctctc tgtcctgttg agtgttttca gcagctgctc ctaaaccagt 4020

cctcctgcca gaaaggacca gtgccgtcac atcgctgtct ctgattgtcc ccggcaccag 4080

caggccttgg ggctcactga aggctcgaag gcactgcaca ccttgtatat tgtcagtgaa 4140

gaacgttagt tggttgtcag tgaacaataa ctttattata tgagtttttg tagcatctta 4200

agaattatac atatgtttga aatattgaaa ctaagctaca gtaccagtaa ttagatgtag 4260

aatcttgttt gtaggctgaa ttttaatctg tatttattgt cttttgtatc tcagaaatta 4320

gaaacttgct acagacttac ccgtaatatt tgtcaagatc atagctgact ttaaaaacag 4380

ttgtaataaa ctttttgatg ct 4402

<210> 3
<211> 2156
<212> DNA
<213> Homo sapiens

<400> 3
gcggccgcgt cgacccggcg ttcagacgcg ggcagctacc ggcgctcgct gggctccgcg 60

gggccgtcgg gcactttgcc tcgcagctgg cagcccgtca gccgcatccc catgcccccc 120

tccagccccc agccccgcgg ggccccgcgc cagcgtccca tccccctcag catgatcttc 180

aagctgcaga acgccttctg ggagcacggg gccagccgcg ccatgctccc tgggtccccc 240

ctcttcaccc gagcaccccc gcctaagctg cagccccaac cacaaccaca gccccagcca 300

caatcacaac cacagcccca gctgccccaa cagccccaga cccaacccca aacccctacc 360

ccagcctccc acatccgcat ccccaacaga catggccccc tgtgaacgaa ggacccccca 420

aaccccccac cgagctggag cctgagccgg agatagaggg gctgctgaca ccagtgctgg 480

aggctggcga tgtggatgaa ggaccctgta gcaaggcctc tcagccccac gaggctgcag 540

ccagcactgc caccggaggc acagtcggtg cccgagctgg aggaggtggc acgggtgttg 600

gcggaaattc cccggcccct caaacgcagg ggctccatgg agcaggcccc tgctgtggcc 660

ctgcccccta cccacaagaa acagtaccag cagatcatca gccgcctctt ccatcgtcat 720

ggggggccag ggcccggggg gcggagccag agctgtcccc catcactgag ggatctgagg 780

ccagggcagg gccccctgct cctgccccac cagctcccat tccaccgccc ggccccgtcc 840

cagagcagcc caccagagca gccgcagagc atggagatgc gctctgtgct gcggaaggcg 900

ggctccccgc gcaaggcccg ccgcgcgcgc ctcaaccctc tggtgctcct cctggacgcg 960

gcgctgaccg gggagctgga ggtggtgcag caggcggtga aggagatgaa cgacccgagc 1020

cagcccaacg aggagggcat cactgccttg cacaacgcca tctgcggcgc caactactct 1080

atcgtggatt tcctcatcac cgcgggtgcc aatgtcaact cccccgacag ccacggctgg 1140

acacccttgc actgcgcggc gtcgtgcaac gacacagtca tctgcatggc gctggtgcag 1200

cacggcgctg caatcttcgc caccacgctc agcgacggcg ccaccgcctt cgagaagtgc 1260

gacccttacc gcgagggtta tgctgactgc gccacctacc tggcagacgt cgagcagagt 1320

atggggctga tgaacagcgg ggcagtgtac gctctctggg actacagcgc cgagttcggg 1380

gacgagctgt ccttccgcga gggcgagtcg gtcaccgtgc tgcggaggga cgggccggag 1440

gagaccgact ggtggtgggc cgcgctgcac ggccaggagg gctacgtgcc gcggaactac 1500

ttcgggctgt tccccagggt gaagcctcaa aggagtaaag tctagcagga tagaaggagg 1560

tttctgaggc tgacagaaac aagcattcct gccttccctc cagacctctc cctctgtttt 1620

ttgctgcctt tatctgcacc cctcaccctg ctggtggtgg tccttgccac cggttctctg 1680

ttctcctgga agtccaggga agaaggaggg ccccagcctt aaatttagta atctgcctta 1740

gccttgggag gtctgggaag ggctggaaat cactggggac aggaaaccac ttccttttgc 1800

caaatcagat cccgtccaaa gtgcctccca tgcctaccac catcatcaca tcccccagca 1860

agccagccac ctgcccagcc gggcctggga tgggccacca caccactgga tattcctggg 1920

agtcactgct gacaccatct ctcccagcag tcttggggtc tgggtgggaa acattggtct 1980

ctaccaggat ccctgcccca cctctcccca attaagtgcc ttcacacagc actggtttaa 2040

tgtttataaa caaaatagag aaactggttt aatgtttata aacaaaatag agaaactttc 2100

gcttataaat aaaagtagtt tgcacagaaa tgaaaaaaaa aaaaaaaaaa aaaaaa 2156

<210> 4
<211> 350
<212> PRT
<213> Homo sapiens

<400> 4

Met Trp Met Lys Asp Pro Val Ala Arg Pro Leu Ser Pro Thr Arg Leu
1               5                   10                  15

Gln Pro Ala Leu Pro Pro Glu Ala Gln Ser Val Pro Glu Leu Glu Glu
            20                  25                  30

Val Ala Arg Val Leu Ala Glu Ile Pro Arg Pro Leu Lys Arg Arg Gly
        35                  40                  45

Ser Met Glu Gln Ala Pro Ala Val Ala Leu Pro Pro Thr His Lys Lys
    50                  55                  60

Gln Tyr Gln Gln Ile Ile Ser Arg Leu Phe His Arg His Gly Gly Pro
65                  70                  75                  80

Gly Pro Gly Gly Arg Ser Gln Ser Cys Pro Pro Ser Leu Arg Asp Leu
                85                  90                  95

Arg Pro Gly Gln Gly Pro Leu Leu Leu Pro His Gln Leu Pro Phe His
            100                 105                 110

Arg Pro Ala Pro Ser Gln Ser Ser Pro Pro Glu Gln Pro Gln Ser Met
        115                 120                 125

Glu Met Arg Ser Val Leu Arg Lys Ala Gly Ser Pro Arg Lys Ala Arg
    130                 135                 140

Arg Ala Arg Leu Asn Pro Leu Val Leu Leu Leu Asp Ala Ala Leu Thr
145                 150                 155                 160

Gly Glu Leu Glu Val Val Gln Gln Ala Val Lys Glu Met Asn Asp Pro
                165                 170                 175

Ser Gln Pro Asn Glu Glu Gly Ile Thr Ala Leu His Asn Ala Ile Cys
            180                 185                 190

Gly Ala Asn Tyr Ser Ile Val Asp Phe Leu Ile Thr Ala Gly Ala Asn
        195                 200                 205

Val Asn Ser Pro Asp Ser His Gly Trp Thr Pro Leu His Cys Ala Ala
    210                 215                 220

Ser Cys Asn Asp Thr Val Ile Cys Met Ala Leu Val Gln His Gly Ala
225                 230                 235                 240

Ala Ile Phe Ala Thr Thr Leu Ser Asp Gly Ala Thr Ala Phe Glu Lys
                245                 250                 255

Cys Asp Pro Tyr Arg Glu Gly Tyr Ala Asp Cys Ala Thr Tyr Leu Ala
            260                 265                 270

Asp Val Glu Gln Ser Met Gly Leu Met Asn Ser Gly Ala Val Tyr Ala
        275                 280                 285

Leu Trp Asp Tyr Ser Ala Glu Phe Gly Asp Glu Leu Ser Phe Arg Glu
    290                 295                 300

Gly Glu Ser Val Thr Val Leu Arg Arg Asp Gly Pro Glu Glu Thr Asp
305                 310                 315                 320

Trp Trp Trp Ala Ala Leu His Gly Gln Glu Gly Tyr Val Pro Arg Asn
                325                 330                 335

Tyr Phe Gly Leu Phe Pro Arg Val Lys Pro Gln Arg Ser Lys
            340                 345                 350

<210>  5
<211>  18
<212>  PRT
<213>  Homo sapiens

<400>  5

Arg Leu Gln Pro Ala Leu Pro Pro Glu Ala Gln Ser Val Pro Glu Leu
1               5                   10                  15

Glu Glu

<210>  6
<211>  1593
<212>  PRT
<213>  human

<400>  6

<210>  7
<211>  1446
<212>  PRT
<213>  human

<400>  7